(19) Europäisches Patentamt / European Patent Office / Office européen des brevets

(11) **EP 4 389 150 A1**

(12) **EUROPEAN PATENT APPLICATION**

(43) Date of publication:
**26.06.2024 Bulletin 2024/26**

(21) Application number: **22216113.5**

(22) Date of filing: **22.12.2022**

(51) International Patent Classification (IPC):
***A61K 47/54*** (2017.01) ***A61K 47/65*** (2017.01)
***A61K 51/04*** (2006.01) ***A61P 35/00*** (2006.01)

(52) Cooperative Patent Classification (CPC):
**A61K 47/542; A61K 47/64; A61K 47/65; A61K 51/0402; A61K 51/0497; A61K 51/083; A61K 51/088; A61P 35/00**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(71) Applicants:
- **Technische Universität München**
  **80333 München (DE)**
- **The Board of Regents of The University of Texas System**
  **Austin, TX 78701 (US)**

(72) Inventor: **The designation of the inventor has not yet been filed**

(74) Representative: **Engelhard, Markus**
**Boehmert & Boehmert**
**Anwaltspartnerschaft mbB**
**Pettenkoferstrasse 22**
**80336 München (DE)**

Remarks:
The complete document including Reference Table(s) and the Sequence Listing(s) can be downloaded from the EPO website

# (54) CONJUGATE COMPOUNDS AND THEIR USE IN CANCER TREATMENT AND DIAGNOSIS

(57) The present invention relates to conjugate compounds comprising a disease-targeting moiety and at least one PARP inhibitor moiety and pharmaceutical compositions comprising the conjugate compounds. The present invention relates to the use of the conjugate compounds for use in the treatment of cancer. The present invention further relates to methods of treatment of cancer and for patient selection.

EP 4 389 150 A1

## Description

The present invention relates to conjugate compounds comprising a disease-targeting moiety and at least one PARP inhibitor moiety and pharmaceutical compositions comprising the conjugate compounds. The present invention relates to the use of the conjugate compounds for use in the treatment of cancer. The present invention further relates to methods of treatment of cancer and for patient selection.

BACKGROUND OF THE INVENTION

Prostate cancer (PCa) is the fifth leading cause of cancer-related deaths in men worldwide with an estimated 360,000 deaths in 2018 (Bray *et al.,* 2018). Most of these deaths are associated with metastatic castration-resistant PCa (mCRPC), a lethal form of PCa that arises from progression and spread of the disease during androgen deprivation therapy. Currently approved life-prolonging treatments for mCRPC include taxanes (docetaxel and cabazitaxel), an immunotherapeutic agent (sipuleucel-T), androgen receptor pathway inhibitors (abiraterone, enzalutamide), and a bone targeting alpha-emitting radionuclide (radium-223 chloride) (Gillessen *et al.,* 2015). Despite the different mechanisms of action of these therapies, patients with mCRPC inevitably progress and develop resistance. Thus, there is a critical need for new treatment options that improve outcomes and quality of life for these patients.

There is a need in the art for improved means and methods which allow for a targeted treatment of cancer, such as PSMA-positive tumors.

SUMMARY OF THE INVENTION

According to the present invention this object is solved by a conjugate compound comprising

a **disease-targeting moiety (T)**,
at least one **PARP inhibitor moiety (P)**,
a **cleavable linker (X)** connecting the PARP inhibitor moiety (P) to the conjugate compound, and
optionally, a **chelating agent (C)**,
or a salt, solvate or tautomer thereof.

According to the present invention this object is solved by a pharmaceutical composition comprising at least one conjugate compound of the present invention and optionally pharmaceutically acceptable carrier and/or excipient(s).

According to the present invention this object is solved by providing the conjugate compound of the present invention as vehicle for the specific delivery of at least one PARP inhibitor moiety to cells expressing the target the disease-targeting moiety (T) recognizes or binds to, wherein said cells are preferably tumor cells.

According to the present invention this object is solved by providing the conjugate compound of the present invention as vehicle for the specific delivery of at least one PARP inhibitor moiety to PSMA-positive cells and tumors, or to NAALADaseL- or mGluR8-positive cells and tumors.

According to the present invention this object is solved by providing the conjugate compound of the present invention or the pharmaceutical composition of the present invention for use in medicine.

According to the present invention this object is solved by providing the conjugate compound of the present invention or the pharmaceutical composition of the present invention for use in the treatment of cancer.

According to the present invention this object is solved by providing the conjugate compound of the present invention or the pharmaceutical composition of the present invention for use in patient selection.

According to the present invention this object is solved by a method of treatment of cancer comprising the administration of a therapeutically effective amount of a conjugate compound or pharmaceutical composition of the present invention to a cancer patient.

According to the present invention this object is solved by a method of patient selection comprising the administration of a conjugate compound or pharmaceutical composition of the present invention to a cancer patient and performing molecular imaging, such as positron emission tomography (PET) or single-photon emission computed tomography (SPECT).

DESCRIPTION OF THE PREFERRED EMBODIMENTS OF THE INVENTION

Before the present invention is described in more detail below, it is to be understood that this invention is not limited to the particular methodology, protocols and reagents described herein as these may vary. It is also to be understood that the terminology used herein is for the purpose of describing particular embodiments only and is not intended to limit the scope of the present invention which will be limited only by the appended claims. Unless defined

otherwise, all technical and scientific terms used herein have the same meanings as commonly understood by one of ordinary skill in the art. For the purpose of the present invention, all references cited herein are incorporated by reference in their entireties.

Concentrations, amounts, and other numerical data may be expressed or presented herein in a range format. It is to be understood that such a range format is used merely for convenience and brevity and thus should be interpreted flexibly to include not only the numerical values explicitly recited as the limits of the range, but also to include all the individual numerical values or sub-ranges encompassed within that range as if each numerical value and sub-range is explicitly recited. As an illustration, a numerical range of "1 to 21" should be interpreted to include not only the explicitly recited values of 1 to 21, but also include individual values and sub-ranges within the indicated range. Thus, included in this numerical range are individual values such as 1, 2, 3, 4, 5 .... 17, 18, 19, 20, 21 and sub-ranges such as from 2 to 10, 8 to 15, etc. This same principle applies to ranges reciting only one numerical value, such as "at least 90%". Furthermore, such an interpretation should apply regardless of the breadth of the range or the characteristics being described.

*Conjugate compounds*

As outlined above, the present invention provides a conjugate compound.

The conjugate compound of the present invention comprises

a **disease-targeting moiety (T)**,
at least one **PARP inhibitor moiety (P)**,
a **cleavable linker (X)** connecting the PARP inhibitor moiety (P) to the conjugate compound, and
optionally, a **chelating agent (C)**,

or a salt, solvate or tautomer thereof.

*- disease-targeting moiety (T)*

The **disease-targeting moiety (T)** preferably comprises a peptide or small molecule, which internalizes upon binding to a cell surface biomarker.

The term "peptides" as used herein refer to peptides comprising natural and/or non-natural amino acids, L- and/or D-amino acids as well as peptidomimetics.

Said peptide or small molecule preferably targets the prostate-specific membrane antigen (PSMA), somatostatin receptor (SSTR subtypes 2, 3, and 5), gastrin-releasing peptide receptor (GRPR), C-X-C motif chemokine receptor 4 (CXCR4), $\alpha v\beta 6$-integrin, $\alpha v\beta 8$-integrin, $\alpha 5\beta 1$-integrin, $\alpha v\beta 1$-integrin, folate receptor (FR), neurotensin receptor 1 (NTSR1), and/or melanocortin receptor 1 (MC1R).

Examples of such peptides or small molecules are

- urea derivatives targeting prostate specific membrane antigen (PSMA),
- somatostatin analogs (SSTR),
- bombesin analogs (GRPR),
- ligands for CXCR4,
- RGD-motif containing peptides ($\alpha v\beta 6$, $\alpha v\beta 8$, $\alpha 5\beta 1$ or $\alpha v\beta 1$ integrins),
- folic acid analogs,
- neurotensin analogs and/or
- melanocortin analogs.

In one embodiment, the disease-targeting moiety (T) comprises monomers or multimers.

The disease-targeting moiety (T) can comprise further component(s). Said further component(s) can be additional structural components to optimize the pharmacokinetic properties of the disease-targeting moiety, including but not limited to PEG linker(s), albumin-binding domain(s), amino acid(s) or amino acid sequence(s) and/or peptidomimetics.

In one embodiment, the disease-targeting moiety (i) targets PSMA and preferably comprises Glu-urea-Glu (EuE), Glu-urea-Lys (EuK) and/or other urea-based peptides or peptidomimetics, such as Lys-urea-aminoadipic acid (Lys-urea-Aad).

*- PARP inhibitor moiety (P)*

Preferably, the **PARP inhibitor moiety (P)** comprises or consists of rucaparib, olaparib, talazoparib, niraparib

or analogs thereof.

An analog refers to a PARP inhibitor moiety (P) that has been modified for conjugation and/or cleavage.

For example, the PARP inhibitor moiety (P) can comprise parts of the cleavable bond, such as a thiol group after cleavage.

In some embodiments, the conjugate compounds comprise two or more PARP inhibitor moieties (P), such as 2 or 3.

In some embodiments, the PARP inhibitor moiety (P) can be connected via an anchor unit (A), such as a lysine or cysteine, to the conjugate compound. Thereby, the PARP inhibitor moiety (P) is preferably connected via the cleavable linker (X) to said anchor unit (A).

*- cleavable linker (X)*

The PARP moiety (P) is preferably connected via the cleavable linker (X) to the conjugate compound.

The **cleavable linker (X)** allows intracellular or extracellular release of the PARP moiety (P) upon enzymatic cleavage or redox reaction.

The cleavable linker (X) preferably comprises a substrate for enzyme cleavage or redox reaction.

Examples for such substrates are a substrate for substrate for prostate specific antigen (PSA), caspases or cathepsins or for the antioxidant glutathione.

Examples for cathepsin B linker are valine-citrulline-p-aminobenzyloxycarbonyl (PABC) and valine-alanine-PABC.

Preferably, the conjugate compounds of the present invention selectively deliver PARP inhibitor(s) to target-positive cells, such as PSMA-positive tumor cells.

The cleavable linker is preferably cleaved intracellularly, i.e. after uptake of the conjugate compound into the target tumor cell, thereby releasing the PARP inhibitor.

In the embodiment where the cleavable linker comprises a disulfide bridge, the cleavage is carried out intracellularly by glutathione (GSH) for release of the PARP inhibitor(s).

Thus, the conjugate compounds of the present invention serve as vehicles for the specific delivery of the PARP inhibitor(s) to target-expressing cells, such as PSMA-positive cells, in particular tumor-targeted delivery of PARP inhibitor(s).

*- Chelating agent (C)*

The conjugate compound preferably comprises a **chelating agent (C**).

In one embodiment, the chelating agent (C) is a macrocyclic chelator or a non-cyclic chelating agent.

Preferred examples of macrocyclic chelators are:

1,4,7,10-tetraazacyclododecane (cyclen),
2-(7-(4-aminobutyl)-4,10-bis(carboxymethyl)-1,4,7,10-tetraazacyclododecan-1-yl)pentanedioic acid (MMC),
2,2',2''-(10-(2,6-dioxotetrahydro-2H-pyran-3-yl)-1,4,7,10-tetraazacyclododecane-1,4,7-triyl)triacetic acid (DOTAGA),
2,2',2'',2'''-(1,4,7,10-tetraazacyclododecane-1,4,7,10-tetrayl)tetraacetic acid (DOTA),
2,2',2''-(1,4,7-triazacyclononane-1,4,7-triyl)triacetic acid (NOTA),
2-[1,4,7-triazacyclononan-1-yl-4,7-bis(tBu-ester)]-1,5-pentanedioic acid (NODAGA),
1,4,7,10-tetraazacyclo- dodecane-1,4,7,10-tetrakis[methylene(2-carboxyethyl)phosphinic acid] (DOTPI),
N,N'-Di(2-hydroxybenzyl)ethylenediamine-N,N'-diacetic acid monohydrochloride (HBED),
N,N'-bis-[2-hydroxy-5-(carboxyethyl)benzyl]ethylenediamine-N,N'-diacetic acid (HBED-CC),
2,2',2'',2'''-(1,4,7,10-tetraazacyclododecane-1,4,7,10-tetrayl)tetraacetamide (DOTAM),
3,3',3''-(((1,4,7-triazonane-1,4,7-triyl)tris(methylene))tris(hydroxyphosphoryl)) tripropanoic acid (TRAP),
3-(((4,7-bis((hydroxy(hydroxymethyl)phosphoryl)methyl)-1,4,7-triazonan-1-yl)methyl)(hydroxy)phosphoryl)propanoic acid (NOPO),
3,6,9,15-Tetraazabicyclo[9.3.1]pentadeca-1(15),11,13-triene-3,6,9-triacetic acid (PCTA), and
ethylenediaminetetraacetic acid (EDTA), and
derivatives thereof.

The chelating agent (C) is preferably selected from the group consisting of cyclen, DOTAGA, DOTA, NOTA, NODAGA, DOTPI, HBED, HBED-CC, DOTAM, TRAP, NOPO, PCTA and EDTA and derivatives thereof.

The chelating agent (C) can comprise or contain a **radionuclide**.

The radionuclide is preferably selected from $^{44}$Sc, $^{45}$Ti, $^{47}$Sc, $^{59}$Fe, $^{60}$Cu, $^{61}$Cu, $^{62}$Cu, $^{64}$Cu, $^{67}$Cu, $^{67}$Ga, $^{68}$Ga,

$^{89}Sr$, $^{90}Y$, $^{94m}Tc$, $^{99m}Tc$, $^{111}In$, $^{149}Pm$, $^{153}Gd$, $^{153}Sm$, $^{161}Tb$, $^{166}Ho$, $^{177}Lu$, $^{186}Re$, $^{188}Re$, $^{203}Pb$, $^{211}At$, $^{212}Bi$, $^{212}Pb$ or $^{225}Ac$.

In a preferred embodiment, the conjugate compound of the present invention has the general formula (I) or (II)

$$\mathbf{T\text{-}(S)_m\text{-}(C)_n\text{-}X\text{-}(P)_o} \qquad (I)$$

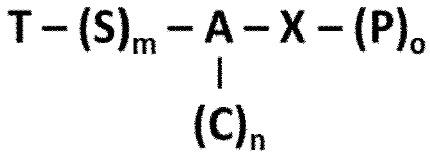

(II)

wherein

**T** is said **disease-targeting moiety**, as defined herein,
**S** is a **functional spacer**,
**m** is 0 or 1,
**C** is said **chelating agent**, as defined herein,
**n** is 0 or 1,
**A** is an **anchor unit**, such as lysine or cysteine,
**X** is said **cleavable linker**, as defined herein,
**P** is said **PARP inhibitor moiety**, as defined herein,
**o** is at least 1, preferably 1, 2 or 3,
or a salt, solvate or tautomer thereof.

The conjugate compounds with general formula I have a linear structure.

The conjugate compounds with general formula II have a branched structure.

*- Functional spacer (S)*

Preferably the **functional spacer (S)** comprises at least two amino acids or amino acid derivatives, preferably

non-natural amino acids,
such as aminohexanoic acid (Ahx), naphthylalanine (Nal), trans-4-(aminomethyl)-cyclohexanecarboxylic acid (TXA),
D-amino acids,

such as D-phenylalanine, D-tyrosine, D-aspartic acid, D-lysine, β-amino acids,
such as β-alanine (βA),

and combinations thereof,
and/or the **functional spacer (S)** comprises polyethylenglycol (PEG), preferably $(PEG)_n$ wherein n is 1 to 24.

The functional spacer (S) can comprise 2 to 10 amino acids or amino acid derivatives, such as 2, 3, 4, 5, 6, 7, 8, 9 or 10.

The functional spacer (S) preferably comprises or consists of an amino acid sequence selected from

| | |
|---|---|
| TXA-L-Nal, | |
| D-Asp-D-Tyr-D-Phe-D-Phe-Ahx | [SEQ ID NO. 1], |
| D-Lys-D-Asp-β-Ala-TXA-L-Nal | [SEQ ID NO. 2], |
| D-Lys-D-Asp-D-Tyr-D-Phe-D-Phe-Ahx | [SEQ ID NO. 3], |
| D-Lys-D-Asp-D-Tyr-D-Phe-L-Nal-Ahx | [SEQ ID NO. 4], |
| D-Lys-D-Asp-D-Tyr-D-Phe-D-Phe-Ahx-Ahx | [SEQ ID NO. 5], |
| D-Asp-D-Asp-β-Ala-TXA-L-Nal | [SEQ ID NO. 6], |
| D-Asp-D-Asp-D-Tyr-D-Phe-D-Phe-Ahx-Ahx | [SEQ ID NO. 7], |
| D-Lys-D-Asp-D-Asp-β-Ala-TXA-L-Nal-D-Lys | [SEQ ID NO. 8], |

(continued)

| | |
|---|---|
| D-Lys-D-Asp-D-Asp-D-Tyr-D-Phe-D-Phe-Ahx-D-Lys | [SEQ ID NO. 9], |
| D-Asp-D-Asp-β-Ala-TXA-L-Nal-D-Lys | [SEQ ID NO. 10], |
| D-Lys(D-Lys)-D-Asp-D-Asp-D-Tyr-D-Phe-D-Phe-Ahx | [SEQ ID NO. 11], |
| D-Lys(D-Lys)-D-Asp-D-Asp-D-Tyr-D-Phe-L-Nal-Ahx | [SEQ ID NO. 12], |
| D-Lys(D-Lys)-D-Asp-D-Asp-D-Tyr-D-Phe-D-Phe-Ahx-Ahx, | [SEQ ID NO. 13], and |
| D-Lys(D-Lys)-D-Asp-D-Asp-β-Ala-TXA-L-Nal-D-Lys | [SEQ ID NO. 14]. |

In one embodiment conjugate compound of the present invention has a linear structure and has the general formula (I)

$$\mathbf{T\text{-}(S)_m\text{-}(C)_n\text{-}X\text{-}(P)_o} \qquad (I)$$

wherein, preferably,

**T** targets PSMA and preferably comprises Glu-urea-Glu (EuE), Glu-urea-Lys (EuK) and/or other urea-based peptides or peptidomimetics, such as Lys-urea-aminoadipic acid (Lys-urea-Aad),
**P** comprises or consists of rucaparib and **o** is 1 or 2,
**m** is 1 and **S** is as defined herein above,
**n** is 1 and **C** is as defined herein above,
**X** comprises a substrate for glutathione.

Preferably, the conjugate compounds of general formula I have one of the following structures

| | |
|---|---|
| **PSMARuc-14:** | Ruc-SS-MMC-D-Asp-D-Asp-β-Ala-TXA-$_L$-Nal-KuE (Ruc-SS-MMC - SEQ ID NO. 6 - KuE), |
| **PSMARuc-15:** | Ruc-SS-MMC-D-Asp-D-Asp-D-Tyr-D-Phe-D-Phe-Ahx-Ahx-KuE (Ruc-SS-MMC - SEQ ID NO. 7 - KUE), or |
| **PSMARuc-18:** | Ruc-SS-MMC-D-Asp-D-Asp-β-Ala-TXA-$_L$-Nal-D-Lys-EuE (Ruc-S S-MMC - SEQ ID NO. 10 - KUE), |

or a salt, solvate or tautomer thereof.

**PSMARuc-14:** (((1S)-5-((2S)-2-((1R,4S)-4-((3-((2R)-2-((2R)-2-(4-(4,10-bis(carboxymethyl)-7-(4-(3-((3-((4-(8-fluoro-1-oxo-2,3,4, 6-tetrahydro-1H-azepino[5,4,3-cd]indol-5-yl)benzyl)(methyl)amino)-3-oxopropyl)disulfaneyl)propanamido)butyl)-1,4,7,10-tetraazacyclododecan-1-yl)-4-carboxybutanamido)-3-carboxypropanamido)-3-carboxypropanamido)propanamido)methyl)cyclohexane-1-carboxamido)-3 -(naphthalen-2-yl)propanamido)-1-carboxypentyl)carbamoyl)-L-glutamic acid

**PSMARuc-15:** (3 S,7S,28R,31R,34R,37R,40R)-28,31-dibenzyl-45-(4,10-bis(carboxymethyl)-7-(4-(3-((3-((4-(8-fluoro-1-oxo-2,3,4, 6-tetrahydro-1H-azepino[5,4,3-cd]indol-5-yl)benzyl)(methyl)amino)-3-oxopropyl)disulfaneyl)propanamido)butyl)-1,4,7,10-tetraazacyclododecan-1-yl)-37,40-bis(carboxymethyl)-34-(4-hydroxybenzyl)-5,13,20,27,30,33,36,39,42-nonaoxo-4,6,12,19,26,29,32,35,38,41-decaazapentatetracontane-1,3,7,45-tetracarboxylic acid,

**PSMARuc-18:**(3S,10R,15S,19S)-1-((1R,4S)-4-((8R,11R)-16-(4,10-bis(carboxymethyl)-7-(4-(3-((3-((4-(8-fluoro-1-oxo-2,3,4,6-tetrahydro-1H-azepino[5,4,3-cd]indol-5-yl)benzyl)(methyl)amino)-3-oxopropyl)disulfaneyl)propanamido)butyl)-1,4,7,10-tetraazacyclododecan-1-yl)-16-carboxy-8,11-bis(carboxymethyl)-3,7,10,13-tetraoxo-2,6,9,12-tetraazahexadecyl)cyclohexyl)-3-(naphthalen-2-ylmethyl)-1,4,12, 17-tetraoxo-2,5, 11, 16, 18-pentaazahenicosane-10,15,19,21-tetracarboxylic acid.

In one embodiment conjugate compound of the present invention has a branched structure and has the general formula (II)

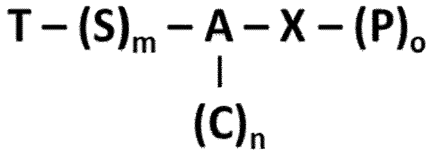

(II)

wherein, preferably,

**T** targets PSMA and preferably comprises Glu-urea-Glu (EuE), Glu-urea-Lys (EuK) and/or other urea-based peptides or peptidomimetics, such as Lys-urea-aminoadipic acid (Lys-urea-Aad),
**P** comprises or consists of rucaparib and **o** is 1 or 2,
**m** is 1 and **S** is as defined herein above,
**n** is 1 and **C** is as defined herein above,
**X** comprises a substrate for glutathione.

Preferably, the conjugate compounds of general formula II have one of the following structures

**PSMARuc-9**: DOTAGA-D-Lys(Ruc-SS)-D-Asp-β-Ala-TXA-$_L$-Nal-KuE
(DOTAGA - SEQ ID NO. 2 - KuE, wherein Lys of SEQ ID NO. 2 has Ruc-SS attached),
**PSMARuc-10**: DOTAGA-D-Lys(Ruc-SS)-D-Asp-D-Tyr-D-Phe-D-Phe-Ahx-KuE
(DOTAGA - SEQ ID NO. 3 - KuE, wherein Lys of SEQ ID NO. 3 has Ruc-SS attached),
**PSMARuc-11:** DOTAGA-D-Lys(Ruc-SS)-D-Asp-D-Tyr-D-Phe-L-Nal-Ahx-KuE
(DOTAGA - SEQ ID NO. 4 - KuE, wherein Lys of SEQ ID NO. 4 has Ruc-SS attached),
**PSMARuc-12:** DOTAGA-D-Lys(Ruc-SS)-D-Asp-D-Tyr-D-Phe-D-Phe-Ahx-Ahx-KuE
(DOTAGA - SEQ ID NO. 5 - KuE, wherein Lys of SEQ ID NO. 2 has Ruc-SS attached),
**PSMARuc-16:** DOTAGA-D-Lys(Ruc-SS)-D-Asp-D-Asp-β-Ala-TXA-$_L$-Nal-D-Lys-EuE,
(DOTAGA - SEQ ID NO. 8 - EuE, wherein the first Lys of SEQ ID NO. 8 has Ruc-SS attached),
**PSMARuc-17**: DOTAGA-D-Lys(Ruc-SS)-D-Asp-D-Asp-D-Tyr-D-Phe-D-Phe-Ahx-D-Lys-EuE
(DOTAGA - SEQ ID NO. 9 - EuE, wherein the first Lys of SEQ ID NO. 9 has Ruc-SS attached),
**PSMARuc$_2$-10 (10Lys):** DOTAGA-D-Lys(D-Lys(Ruc-SS)$_2$)-D-Asp-D-Asp-D-Tyr-D-Phe-D-Phe-Ahx-KuE
(DOTAGA - SEQ ID NO. 11 - KuE, wherein the D-Lys(D-Lys) of SEQ ID NO. 11 each have Ruc-SS attached),
**PSMARuc$_2$-11 (11Lys)**: DOTAGA-D-Lys(D-Lys(Ruc-SS)$_2$)-D-Asp-D-Asp-D-Tyr-D-Phe-L-Nal-Ahx-KuE
(DOTAGA - SEQ ID NO. 12 - KuE, wherein the D-Lys(D-Lys) of SEQ ID NO. 12 each have Ruc-SS attached),
**PSMARuc$_2$-12 (12Lys)**: DOTAGA-D-Lys(D-Lys(Ruc-SS)$_2$)-D-Asp-D-Asp-D-Tyr-D-Phe-D-Phe-Ahx-Ahx-KuE
(DOTAGA - SEQ ID NO. 13 - KuE, wherein the D-Lys(D-Lys) of SEQ ID NO. 13 each have Ruc-SS attached),
**PSMARuc$_2$-16 (16Lys)**: DOTAGA_D-Lys($_D$-Lys(Ruc-SS)$_2$)-$_D$-Asp-$_D$-Asp-β-Ala-TXA-$_L$-Nal-D-Lys-EuE
(DOTAGA - SEQ ID NO. 14 - EuE, wherein the D-Lys(D-Lys) of SEQ ID NO. 14 each have Ruc-SS attached),

or

**PSMARuc$_2$-17 (17Lys)**: DOTAGA-D-Lys(D-Lys(Ruc-SS)$_2$)-D-Asp-D-Asp-D-Tyr-D-Phe-D-Phe-Ahx-D-Lys-EuE
(DOTAGA - SEQ ID NO. 11 - EuE, wherein the D-Lys(D-Lys) of SEQ ID NO. 11 each have Ruc-SS attached),
or a salt, solvate or tautomer thereof.

**PSMARuc-9:** (((1S)-1-carboxy-5-((2S)-2-(4-((3-((2R)-3-carboxy-2-((2R)-2-(4-carboxy-4-(4,7,10-tris(carboxymethyl)-1,4,7,10-tetraazacyclododecan-1-yl)butanamido)-6-(3-((3-((4-(8-fluoro-1-oxo-2,3,4,6-tetrahydro-1H-azepino[5,4,3-cd]indol-5-yl)benzyl)(methyl)amino)-3-oxopropyl)disulfaneyl)propanamido)hexanamido)propanamido)propanamido)methyl)cyclohe xane-1-carboxamido)-3-(naphthalen-2-yl)propanamido)pentyl)carbamoyl)-L-glutamic acid,

**PSMARuc-10:** (3S,7S,21R,24R,27R,30R,33R)-21,24-dibenzyl-30-(carboxymethyl)-33-(4-(3-((3-((4-(8-fluoro-1-oxo-2,3,4,6-tetrahydro-1H-azepino[5,4,3-cd]indol-5-yl)benzyl)(methyl)amino)-3-oxopropyl)disulfaneyl)propanamido)butyl)-27-(4-hydroxybenzyl)-5,13,20,23,26,29,32,35-octaoxo-38-(4,7,10-tris(carboxymethyl)-1,4,7,10-tetraazacyclododecan-1-yl)-4,6,12,19,22,25,28,31,34-nonaazaoctatriacontane-1,3,7,38-tetracarboxylic acid,

**PSMARuc-11:** (3S,7S,21S,24R,27R,30R,33R)-24-benzyl-30-(carboxymethyl)-33-(4-(3-((3-((4-(8-fluoro-1-oxo-2,3,4,6-tetrahydro-1H-azepino[5,4,3-cd]indol-5-yl)benzyl)(methyl)amino)-3-oxopropyl)disulfaneyl)propanamido)butyl)-27-(4-hydroxybenzyl)-21-(naphthalen-2-ylmethyl)-5,13,20,23,26,29,32,35-octaoxo-38-(4,7,10-tris(carboxymethyl)-1,4,7,10-tetraazacyclododecan-1-yl)-4,6,12,19,22,25,28,31,34-nonaazaoctatriacontane-1,3,7,38-tetracarboxylic acid,

**PSMARuc-12:** (3S,7S,28R,31R,34R,37R,40R)-28,31-dibenzyl-37-(carboxymethyl)-40-(4-(3-((3-((4-(8-fluoro-1-oxo-2,3,4,6-tetrahydro-1H-azepino[5,4,3-cd]indol-5-yl)benzyl)(methyl)amino)-3-oxopropyl)disulfaneyl)propanamido)butyl)-34-(4-hydroxybenzyl)-5,13,20,27,30,33,36,39,42-nonaoxo-45-(4,7,10-tris(carboxymethyl)-1,4,7,10-tetraazacyclododecan-1-yl)-4,6,12,19,26,29,32,35,38,41-decaazapentatetracontane-1,3,7,45-tetracarboxylic acid,

**PSMARuc-16:** (3S,10R,15S,19S)-1-(4-((16R,19R,22R)-16-(4-carboxy-4-(4,7,10-tris(carboxymethyl)-1,4,7,10-tetraazacyclododecan-1-yl)butanamido)-19,22-bis(carboxymethyl)-1-(4-(8-fluoro-1-oxo-2,3,4,6-tetrahydro-1H-azepino[5,4,3-cd]indol-5-yl)phenyl)-2-methyl-3,10,17,20,23,27-hexaoxo-6,7-dithia-2,11,18,21,24,28-hexaazanonacosan-29-yl)cyclohexyl)-3-(naphthalen-2-ylmethyl)-1,4,12,17-tetraoxo-2,5,11,16,18-pentaazahenicosane-10,15,19,21-tetracarboxylic acid,

**PSMARuc-17:** (3S,7S,12R,26R,29R,32R,35R,38R,41R)-26,29-dibenzyl-35,38-bis(carboxymethyl)-41-(4-(3-((3-((4-(8-fluoro-1-oxo-2,3,4,6-tetrahydro-1H-azepino[5,4,3-cd]indol-5-yl)benzyl)(methyl)amino)-3-oxopropyl)disulfaneyl)propanamido)butyl)-32-(4-hydroxybenzyl)-5,10,18,25,28,31,34,37,40,43-decaoxo-46-(4,7,10-tris(carboxymethyl)-1,4,7,10-tetraazacyclododecan-1-yl)-4,6,11,17,24,27,30,33,36,39,42-undecaazahexatetracontane-1,3,7,12,46-pentacarboxylic acid,

**PSMARuc$_2$-10 (10Lys):** (3S,7S,21R,24R,27R,30R,33R,36R)-21,24-dibenzyl-30,33-bis(carboxymethyl)-36-((R)-16-(3-((3-((4-(8-fluoro-1-oxo-2,3,4,6-tetrahydro-1H-azepino[5,4,3-cd]indol-5-yl)benzyl)(methyl)amino)-3-oxopropyl)disulfaneyl)propanamido)-1-(4-(8-fluoro-1-oxo-2,3,4,6-tetrahydro-1H-azepino[5,4,3-cd]indol-5-yl)phenyl)-2-methyl-3,10,17-trioxo-6,7-dithia-2,11,18-triazadocosan-22-yl)-27-(4-hydroxybenzyl)-5,13,20,23,26,29,32,35,38-nonaoxo-41-(4,7,10-tris(carboxymethyl)-1,4,7,10-tetraazacyclododecan-1-yl)-4,6,12,19,22,25,28,31,34,37-decaazahentetracontane-1,3,7,41-tetracarboxylic acid,

**PSMARuc$_2$-11 (11Lys):** (3S,7S,21R,24R,27R,30R,33R,36R)-24-benzyl-30,33-bis(carboxymethyl)-3 6-((R)-16-(3 -((3 -((4-(8-fluoro-1 -oxo-2,3,4,6-tetrahydro- 1H-azepino[5,4,3-cd]indol-5-yl)benzyl)(methyl)amino)-3-oxopropyl)disulfaneyl)propanamido)-1-(4-(8-fluoro-1-oxo-2,3,4,6-tetrahydro-1H-azepino[5,4,3-cd]indol-5-yl)phenyl)-2-methyl-3,10,17-trioxo-6,7-dithia-2,11,18-triazadocosan-22-yl)-27-(4-hydroxybenzyl)-21-(naphthalen-2-ylmethyl)-5,13,20,23,26,29,32,3 5,3 8-nonaoxo-41-(4,7,10-tris(carboxymethyl)-1,4,7,10-tetraazacyclododecan-1-yl)-4,6,12,19,22,25,28,31,34,37-decaazahentetracontane-1,3,7,41-tetracarboxylic acid,

**PSMARuc$_2$-12 (12Lys)**: (3S,7S,28R,31R,34R,37R,40R,43R)-28,31-dibenzyl-37,40-bis(carboxymethyl)-43 -((R)-16-(3 -((3 -((4-(8-fluoro-1 -oxo-2,3,4,6-tetrahydro- 1H-azepino[5,4,3-cd]indol-5-yl)benzyl)(methyl)amino)-3-oxopropyl)disulfaneyl)propanamido)-1-(4-(8-fluoro-1-oxo-2,3,4,6-tetrahydro-1H-azepino[5,4,3-cd]indol-5-yl)phenyl)-2-methyl-3,10,17-trioxo-6,7-dithia-2,11,18-triazadocosan-22-yl)-34-(4-hydroxybenzyl)-5,13,20,27,30,33,36,39,42,45-decaoxo-48-(4,7,10-tris(carboxymethyl)-1,4,7,10-tetraazacyclododecan-1-yl)-4,6,12,19,26,29,32,35,38,41,44-undecaazaoctatetracontane-1,3,7,48-tetracarboxylic acid,

**PSMARuc$_2$-16 (16Lys):** (3S,10R,15S,19S)-1-((1R,4S)-4-((16R,23R,26R,29R)-23-(4-carboxy-4-(4,7,10-tris(carboxymethyl)-1,4,7,10-tetraazacyclododecan-1-yl)butanamido)-26,29-bis(carboxymethyl)-16-(3-((3-((4-(8-fluoro-1-oxo-2,3,4,6-tetrahydro-1H-azepino[5,4,3-cd]indol-5-yl)benzyl)(methyl)amino)-3-oxopropyl)disulfaneyl)propanamido)-1-(4-(8-fluoro-1-oxo-2,3,4,6-tetrahydro-1H-azepino[5,4,3-cd]indol-5-yl)phenyl)-2-methyl-3,10,17,24,27,30,34-heptaoxo-6,7-dithia-2,11,18,25,28,31,35-heptaazahexatriacontan-36-yl)cyclohexyl)-3-(naphthalen-2-ylmethyl)-1,4,12,17-tetraoxo-2,5,11,16,18-pentaazahenicosane-10,15,19,21-tetracarboxylic acid,

**PSMARuc$_2$-17 (17Lys)**: (3S,7S,12R,26R,29R,32R,35R,38R,41R)-26,29-dibenzyl-35,38-bis(carboxymethyl)-41 -((R)-16-(3 -((3 -((4-(8-fluoro-1 -oxo-2,3,4,6-tetrahydro- 1H-azepino[5,4,3-cd]indol-5-yl)benzyl)(methyl)amino)-3-oxopropyl)disulfaneyl)propanamido)-1-(4-(8-fluoro-1-oxo-2,3,4,6-tetrahydro-1H-azepino[5,4,3-cd]indol-5-yl)phenyl)-2-methyl-3,10,17-trioxo-6,7-dithia-2,11,18-triazadocosan-22-yl)-32-(4-hydroxybenzyl)-5,10,18,25,28,31,34,37,40,43-decaoxo-46-(4,7,10-tris(carboxymethyl)-1,4,7,10-tetraazacyclododecan-1-yl)-4,6,11,17,24,27,30,33,36,39,42-undecaazahexatetracontane-1,3,7,12,46-pentacarboxylic acid.

*Pharmaceutical compositions and medical uses*

As outlined above, the present invention provides a pharmaceutical composition comprising at least one conjugate compound of the present invention and, optionally, pharmaceutically acceptable carrier and/or excipient(s).

The pharmaceutical compositions according to the present invention are very well suited for all the uses and methods described herein.

A "pharmaceutically acceptable carrier or excipient" refers to any vehicle wherein or with which the pharmaceutical compositions according to the invention may be formulated. It includes a saline solution such as phosphate buffer saline. In general, a diluent or carrier is selected based upon the mode and route of administration, and standard pharmaceutical practice.

As outlined above, the present invention provides the conjugate compound of the present invention as vehicle for the specific delivery of at least one PARP inhibitor moiety to cells expressing the target the disease-targeting moiety (T) recognizes or binds to.
Said target cells are preferably tumor cells.

As outlined above, the present invention provides the conjugate compound of the present invention as vehicle for the specific delivery of at least one PARP inhibitor moiety to PSMA-positive cells and tumors,
or to NAALADaseL- or mGluR8-positive cells and tumors.

Preferably, the conjugate compound as vehicle for the specific delivery of at least one PARP inhibitor moiety to PSMA-positive cells and tumors, or to NAALADaseL- or mGluR8-positive cells and tumors:

has general formula I, wherein

**T** targets PSMA and preferably comprises Glu-urea-Glu (EuE), Glu-urea-Lys (EuK) and/or other urea-based peptides or peptidomimetics, such as Lys-urea-aminoadipic acid (Lys-urea-Aad),
**P** comprises or consists of rucaparib and **o** is 1 or 2.

preferably having one of the following structures

**PSMARuc-14:** Ruc-SS-MMC-D-Asp-D-Asp-β-Ala-TXA-L-Nal-KuE,
**PSMARuc-15:** Ruc-SS-MMC-D-Asp-D-Asp-D-Tyr-D-Phe-D-Phe-Ahx-Ahx-KuE, or
**PSMARuc-18:** Ruc-SS-MMC-D-Asp-D-Asp-β-Ala-TXA-L-Nal-D-Lys-EuE,
or a salt, solvate or tautomer thereof.

Preferably, the conjugate compound as vehicle for the specific delivery of at least one PARP inhibitor moiety to PSMA-positive cells and tumors, or to NAALADaseL- or mGluR8-positive cells and tumors:

Has general formula II, wherein

**T** targets PSMA and preferably comprises Glu-urea-Glu (EuE), Glu-urea-Lys (EuK) and/or other urea-based peptides or peptidomimetics, such as Lys-urea-aminoadipic acid (Lys-urea-Aad),
**P** comprises or consists of rucaparib and **o** is 1 or 2,

preferably having one of the following structures

**PSMARuc-9**: DOTAGA-D-Lys(Ruc-SS)-D-Asp-β-Ala-TXA-L-Nal-KuE,
**PSMARuc-10**: DOTAGA-D-Lys(Ruc-SS)-D-Asp-D-Tyr-D-Phe-D-Phe-Ahx-KuE,
**PSMARuc-11:** DOTAGA-D-Lys(Ruc-SS)-D-Asp-D-Tyr-D-Phe-L-Nal-Ahx-KuE,
**PSMARuc-12:** DOTAGA-D-Lys(Ruc-SS)-D-Asp-D-Tyr-D-Phe-D-Phe-Ahx-Ahx-KuE,
**PSMARuc-16:** DOTAGA-D-Lys(Ruc-SS)-D-Asp-D-Asp-β-Ala-TXA-L-Nal-D-Lys-EuE,
**PSMARuc-17:** DOTAGA-D-Lys(Ruc-SS)-D-Asp-D-Asp-D-Tyr-D-Phe-D-Phe-Ahx-D-Lys-EuE,
**PSMARuc$_2$-10 (10Lys):** DOTAGA-D-Lys(D-Lys(Ruc-SS)$_2$)-D-Asp-D-Asp-D-Tyr-D-Phe-D-Phe-Ahx-KuE,
**PSMARuc$_2$-11 (11Lys)**: DOTAGA-D-Lys(D-Lys(Ruc-SS)$_2$)-D-Asp-D-Asp-D-Tyr-D-Phe-L-Nal-Ahx-KuE,
**PSMARuc$_2$-12 (12Lys)**: DOTAGA-D-Lys(D-Lys(Ruc-SS)$_2$)-D-Asp-D-Asp-D-Tyr-D-Phe-D-Phe-Ahx-Ahx-KuE,
**PSMARuc$_2$-16 (16Lys)**: DOTAGA-D-Lys(D-Lys(Ruc-SS)$_2$)-D-Asp-D-Asp-β-Ala-TXA-L-Nal-D-Lys-EuE, or
**PSMARuc$_2$-17 (17Lys)**: DOTAGA-D-Lys(D-Lys(Ruc-SS)$_2$)-D-Asp-D-Asp-D-Tyr-D-Phe-D- Phe- Ahx -D- L ys-EuE,
or a salt, solvate or tautomer thereof.

As outlined above, the present invention provides the compound or the pharmaceutical composition of the present invention for use in medicine.

As outlined above, the present invention provides the compound or the pharmaceutical composition of the present invention for use in the treatment of cancer.

The cancer to be treated is preferably

- a PSMA-positive tumor,
  such as

  prostate cancer, wherein the prostate cancer can be metastatic prostate cancer and/or biochemically recurrent prostate cancer,
  glioma,
  lung cancer,

- tumors expressing the target, wherein the target is selected from the somatostatin receptor (SSTR subtypes 2, 3, and 5), gastrin-releasing peptide receptor (GRPR), C-X-C motif chemokine receptor 4 (CXCR4), $\alpha v\beta 6$-integrin, $\alpha v\beta 8$-integrin, $\alpha 5\beta 1$-integrin, $\alpha v\beta 1$-integrin, folate receptor (FR), neurotensin receptor 1 (NTSR1), and melanocortin receptor 1 (MC1R).

A "PSMA-positive tumor" or "PSMA-expressing tumor" as used herein refers to a tumor that expresses PSMA as determined by standard of care clinical tests, including immunohistochemistry and a PSMA-PET or SPECT scan.

A PSMA-positive tumor or cancer is preferably prostate cancer, such as castration sensitive metastatic prostate cancer (mCRPC), primary prostate cancer.

"Tumors expressing the target" refers to all tumors in patients that have been tested positive for the respective target, preferably via histology or imaging.

Said tumors include, but are not limited to:

*Target = SSTR2:* e.g. neuroendocrine tumors (NET), gastroenteropancreatic NET (GEP-NET), small cell lung cancer, pituitary tumors, insulinomas, breast cancers and pancreatic cancers,
*Target = GRPR:* e.g. prostate cancer, breast cancer, small cell lung *cancer*, non-small cell lung *cancer*,
*Target = CXCR4* (confirmed in 23 tumor types): e.g. hematological malignancy, breast cancer, colorectal cancer, esophageal cancer, renal cancer, gynecologic cancer, pancreatic cancer and liver cancer,
*Target = Avb6:* epithelial cancers, e.g. pancreatic cancer, head and neck cancer,
*Target = Avb8:* epithelial cancers, e.g. lung, brain, ovarian, endometrial, melanoma, breast, prostate, colon, skin, and stomach,
*Target = A5b1:* e.g. oral squamous cell carcinoma and ovarian cancer,
*Target = Avb1:* cancer-associated fibroblasts in various tumor types,
*Target = folate receptor:* e.g. breast, cervical, colorectal, renal, nasopharyngeal, ovarian, and endometrial (in general epithelial cancers),
*Target = neurotensin-1:* e.g. pancreatic, lung, breast, prostate cancers and colon cancer,
*Target = melanocortin-1:* melanoma.

For example, the cancer treatment can be several cycles of treatment, such as daily applications. Exemplary doses can be similar or lower than clinically used doses of PARPi.

Preferred routes of administration are intravenous, intraperitoneal, oral, nasal, as solution or spray, more preferably intravenous or intraperitoneal.

In a preferred embodiment, the use for cancer treatment is in combination with another therapy, preferably

- - chemotherapy,
- - hormonal therapy,
  such as hormonal therapy of mCRPC,
- - immune-checkpoint blockade,
- - DNA damage response (DDR) inhibiting agents,
  such as ATR/ATM inhibitors, AR targeting agents, DNA damaging agents, AKT inhibitors,
- antiangiogenic agents,
- tubulin inhibitors,
  such as MMAE (maytansine) and mertansine (DM1) or further conjugates of agents wherein the agents themselves are to toxic for systemic application (e.g. antibody-drug-conjugates),

and/or

- standard of care treatment.

In case of chemotherapy or the use of a chemotherapeutic agent, a "chemotherapeutic agent" as used herein refers to all chemotherapeutic and cytotoxic agents comprising the clinically approved chemotherapeutic agents.
In a preferred embodiment, the use for cancer treatment is in combination with external beam radiation therapy.
In a preferred embodiment, the cancer treatment is in combination with endoradiotherapy, e.g. targeted radioligand therapy (RLT) or peptide receptor radiotherapy (PRRT),
preferably comprising using therapeutic isotopes in RLT or PRRT agent(s), said therapeutic isotopes being selected from $^{47}$Sc, $^{67}$Cu, $^{67}$Ga, $^{89}$Sr, $^{90}$Y, $^{149}$Pm, $^{149}$Tb, $^{153}$Sm, $^{161}$Tb, $^{166}$Ho, $^{177}$Lu, $^{186}$Re, $^{188}$Re, $^{211}$At, $^{212}$Bi, $^{212}$Pb or $^{225}$Ac in RLT or PRRT agents.
For example, the conjugate compounds, not radiolabeled or cold-labelled (labelled with a stable isotope), can be given as single dose or treatment cycles, in combination with single or multiple cycles of endoradiotherapy (e.g. RLT or PRRT) with targeted alpha- or beta-emitting ligands. Preferably, both compounds have the same target, e.g. PSMA, SSTR2, or others. In the case of PSMA-targeted therapy, RLT could be treatment with PSMA-targeting ligands, such as [177Lu]-PSMA-617 or [177Lu]-rhPSMA, which are typically given in several cycles with intervals of six to eight weeks in between each application. In the case of SSTR2-targeted therapy, PRRT could be treatment with SSTR2-targetings ligands, such as [177Lu]-DOTA-TATE or [177Lu]-DOTA-LM3, which are typically given in several cycles with intervals of six to eight weeks in between each application.
As outlined above, the present invention provides the compound or the pharmaceutical composition of the present invention for use in patient selection.
Said use preferably comprises molecular imaging, such as positron emission tomography (PET) or single-photon emission computed tomography (SPECT) with said compound or said pharmaceutical composition.
"Patient selection" as used herein refers to the determination whether the patient will likely benefit from the treatment with a compound or pharmaceutical composition of the present invention. The procedure for "patient selection" will consist of administering a sub-pharmacologic dose of the radiolabeled form of the compound and confirming accumulation in tumors, preferably by PET or SPECT.

*Methods of treatment and patient selection*

As outlined above, the present invention provides a method of treatment of cancer comprising the administration of a therapeutically effective amount of a conjugate compound or pharmaceutical composition of the present invention to a cancer patient.
The cancer to be treated is preferably

- a PSMA-positive tumor,
  such as

  prostate cancer, wherein the prostate cancer can be metastatic prostate cancer and/or biochemically recurrent prostate cancer,
  glioma,
  lung cancer,

- tumors expressing the target, wherein the target is selected from somatostatin-receptor (SSTR subtypes 2, 3, and 5), gastrin-releasing peptide receptor (GRPR), C-X-C motif chemokine receptor 4 (CXCR4), $\alpha v\beta 6$-integrin, $\alpha v\beta 8$-integrin, $\alpha 5\beta 1$-integrin, $\alpha v\beta 1$-integrin, folate receptor (FR), neurotensin receptor 1 (NTSR1), and melanocortin receptor 1 (MC1R).

As discussed above, a "PSMA-positive tumor" or "PSMA-expressing tumor" as used herein refers to a tumor that expresses PSMA as determined by standard of care clinical tests, including immunohistochemistry and a PSMA-PET or SPECT scan.
A PSMA-positive tumor or cancer is preferably prostate cancer, such as castration sensitive metastatic prostate cancer (mCRPC), primary prostate cancer.
"Tumors expressing the target" refers to all tumors in patients that have been tested positive for the respective target, preferably via histology or imaging.
Please see above for exemplary tumors.
For example, the cancer treatment can be several cycles of treatment, such as daily applications. Exemplary

doses can be similar or lower than clinically used doses of PARPi.

Preferred routes of administration are intravenous, intraperitoneal, oral, nasal, as solution or spray, more preferably intravenous or intraperitoneal.

In a preferred embodiment, and as discussed above, the use for cancer treatment is in combination with another therapy, preferably

- - chemotherapy,
- - hormonal therapy,
  such as hormonal therapy of mCRPC,
- - immune-checkpoint blockade,
- - DNA damage response (DDR) inhibiting agents,
  such as ATR/ATM inhibitors, AR targeting agents, DNA damaging agents, AKT inhibitors,
- antiangiogenic agents,
- tubulin inhibitors,
  such as MMAE (maytansine) and mertansine (DM1) or further conjugates of agents wherein the agents themselves are to toxic for systemic application (e.g. antibody-drug-conjugates),

and/or

- standard of care treatment.

In a preferred embodiment, the cancer treatment is in combination with external beam radiation therapy.

In a preferred embodiment, the cancer treatment is in combination with targeted radioligand therapy (RLT) or peptide receptor radiotherapy (PRRT),
preferably comprising using therapeutic isotopes in RLT or PRRT agent(s), said therapeutic isotopes being selected from $^{47}$Sc, $^{67}$Cu, $^{67}$Ga, $^{89}$Sr, $^{90}$Y, $^{149}$Pm, $^{149}$Tb, $^{153}$Sm, $^{161}$Tb, $^{166}$Ho, $^{177}$Lu, $^{186}$Re, $^{188}$Re, $^{211}$At, $^{212}$Bi, $^{212}$Pb or $^{225}$Ac in RLT or PRRT agents.

A "therapeutically effective amount" of a conjugate compound of the present invention refers to the amount which has to be administered to a subject in need thereof in order to achieve a desired therapeutic result or outcome. Therapeutically effective amounts and suitable administration regimen will be determined from preclinical and clinical studies designed by qualified medical personnel.

For example, the conjugate compounds, not radiolabeled or cold-labelled (labelled with a stable isotope), can be given as single dose or treatment cycles, in combination with single or multiple cycles of endoradiotherapy (e.g. RLT or PRRT) with targeted alpha- or beta-emitting ligands. Preferably, both compounds have the same target, e.g. PSMA, SSTR2, or others. In the case of PSMA-targeted therapy, RLT could be treatment with PSMA-targeting ligands, such as [177Lu]-PSMA-617 or [177Lu]-rhPSMA, which are typically given in several cycles with intervals of six to eight weeks in between each application. In the case of SSTR2-targeted therapy, PRRT could be treatment with SSTR2-targetings ligands, such as [177Lu]-DOTA-TATE or [177Lu]-DOTA-LM3, which are typically given in several cycles with intervals of six to eight weeks in between each application.

As outlined above, the present invention provides a method of patient selection comprising the administration of a sub-pharmacologic amount of a conjugate compound or pharmaceutical composition of the present invention to a cancer patient and performing molecular imaging, such as positron emission tomography (PET) or single-photon emission computed tomography (SPECT).

*Further description of preferred embodiments*

*PARP inhibitors as mono- and combination therapy agents in prostate cancer.* Inhibitors of the DNA repair enzyme Poly(ADP-ribose)Polymerase 1/2 (PARP) have been approved for treatment of numerous indications in patients with breast, ovarian, pancreatic and prostate cancer, among others. Currently, 4 PARPi are FDA and/or EMA approved, namely Olaparib, Rucaparib, Talazoparib and Niraparib (Deeks 2015; Syed 2017; Hoy 2018). Olaparib and Rucaparib (Ruc), have recently been approved for the treatment of mCRPC patients (Deeks, 2015 and Syed, 2017). Patient eligibility is based on mutations in the DNA damage repair machinery, i.e. the presence of homologous recombination deficiencies (HRD). HRD in combination with pharmacological PARP inhibition leads to synthetic lethality in the absence of functional DNA repair pathways. In the TRITON2 trial (NCT02952534) that led to the accelerated FDA approval of Ruc, 44% of patients showed an objective response, including a small number of patients with complete response and 30-40% with a partial response (Abida *et al.,* 2020). Both PARP inhibitors (PARPi) are approved as monotherapy agents in PC patients with BRCA1/2 mutations. Olaparib treatment is also approved for 12 other HRD gene alterations, including ATM and PALB2. Although up to 25% of PC patients exhibit HRD gene alterations, only around 5% have BRCA1/2 mutations,

which seem to be associated with more consistent and durable responses than other HRD gene alterations.

In order to extend the promising anti-tumor activity of PARPi to PC patients without HRD, the interest in creating a synthetic lethality phenotype in non-HRD/BRCA-functional patients by combining PARPi with other drugs emerged. Hence, almost in parallel to the approval of monotherapy, clinical studies were initiated to explore the potential of PARPi in combination with other PC therapies. Currently, over a dozen clinical trials are ongoing combining PARPi with temozolomide, AR-targeting, immunotherapy, irradiation and radionuclide therapy (Antonorakis *et al.,* 2020; Nizialek *et al.,* 2020; Pezaro 2020; Virtanen *et al.,* 2019). Several trials include both patients with and without HRD alterations. Importantly, preclinical studies have already shown that combination therapies can be effective in tumors without an HRD phenotype, supporting the concept that synthetic lethality can be induced by drug combinations. Similar findings in the clinic would extend the benefits of PARPi combination treatment to an even larger number of PC patients and could improve survival rates. However, early clinical evidence indicates significant systemic toxicity associated with combinatorial dosing regimens, often requiring dose reductions and leading to more severe adverse events than use of either monotherapy alone (Rajan *et al.,* 2012; Takebe *et al.,* 2019). Strategies that improve the tolerability of combination therapy using PARPi would address an important clinical need in urologic oncology and allow continued clinical development of critically needed new therapies for PC.

*PSMA as a tumor-selective biomarker for PC therapy.* Prostate-specific membrane antigen (PSMA) is a transmembrane glycoprotein that has 100 to 1000-fold higher expression in PC cells compared to normal prostate epithelial cells. The vast majority of prostate cancers overexpress PSMA at the cell surface. Importantly, PSMA overexpression has been found to be even higher with increasing de-differentiation or castration-resistant disease, making it an attractive biomarker for targeted probe development (Ghosh *et al.,* 2004). Indeed, a multitude of high-affinity, PSMA-directed small molecules have been translated into patients over the last decade. Increasingly, radiolabeled PSMA analogs (with gallium-68 ($^{68}$Ga) or Fluorine-18) for positron emission tomography (PET) have been used for assessing tumor burden in patients with biochemical recurrence or advanced disease, staging of primary disease, and monitoring treatment response (Hoffmann *et al.,* 2019; Fendler *et al.,* 2019; Eiber *et al.,* 2015; Fendler *et al.,* 2016).

Here, we disclose an innovative strategy that introduces selective delivery of PARP inhibitors to cells expressing tumor biomarkers (e.g. cell surface receptors).

*Ruc as an ideal agent for bioconjugate development and combination therapy of mCRPC.* Among all FDA approved PARPi, namely Olaparib, Rue, Veliparib and Talazoparib (Deeks 2015; Syed 2017; Hoy 2018), Ruc is the only one with an indole-based fluorochrome embedded in its structure, rendering it a potent anti-cancer drug and a biocompatible fluorophore at the same time (Kossatz *et al.,* 2018). This allows direct imaging to track the delivery, target-engagement, distribution, and clearance of Ruc without the need of external labeling techniques (Figure 1). Thus, Ruc fluorescence can become integral in understanding and optimizing Ruc-based treatment strategies. In the present case, Ruc fluorescence allows to straightforwardly validate and monitor PSMA-dependent delivery of PARPi.

*PSMA-targeted radioligand therapy (RLT) for combination with Ruc.* PSMA is also the target structure for PSMA-targeted $^{177}$Lu-RLT, which was recently granted breakthrough therapy designation by the FDA, after publication of the VISION trial data, which showed prolonged median overall survival (15.3 vs. 11.3 months) in late stage mCRPC patients compared to standard therapy (Sartor *et al.,* 2021). The VISION trial employed PSMA-617, a PSMA analog of particular importance because of its versatile drug design that provides $^{68}$Ga-PET and therapeutic utility with the same PSMA-targeting motif (Benesova *et al.,* 2015; Fendler *et al.,* 2017). Subsequently, $^{177}$Lu-PSMA-617 ("Pluvicto") was FDA approved for treatment of metastatic castration-resistant prostate cancer on March 23$^{rd}$, 2022 (https://www.fda.gov/drugs/resources-information-approved-drugs/fda-approves-pluvicto-metastatic-castration-resistant-prostate-cancer). Other $^{177}$Lu-labelled PSMA-targeting agents are currently in phase III clinical evaluation. When used with the therapeutic, beta-emitting radionuclide, lutetium-177 ($^{177}$Lu), precise systemic delivery of cytotoxic radiation to tumors is achieved (Heinzel *et al.,* 2019; Yadav *et al.,* 2019). Despite the immense potential of $^{177}$Lu-PSMA therapy, a significant subset of patients show a limited or no response, for reasons that are not well understood. The radiation produced by the ionizing beta particles of $^{177}$Lu result in both DNA single-strand breaks (SSB) and double-strand breaks (DSB), which are lethal if unrepaired. Therefore, the combination of RLT and PARPi can be an immensely powerful treatment to induce a synthetic lethality state, even in absence of HRD, which could benefit a much larger patient population than current PARPi treatment and lead to improved therapeutic outcomes compared to RLT alone.

This invention produces a PC-targeted PARPi that can be used, e.g. to augment the antitumor effect of $^{177}$Lu-PSMA-617, which was also recently clinically approved by the FDA for treatment of mCRPC or other therapeutic agents (e.g. chemotherapy, external beam irradiation, hormonal therapy).

Furthermore, the new conjugate compounds can substantially reduce the hematotoxicity of PARP inhibitors because PSMA is not expressed by hematopoietic cells. By eliminating or reducing the side effects of systemic PARP inhibition, tumor specific delivery of PARP inhibitors can facilitate the use of this therapy in earlier stages of prostate cancer, for example in combination with hormonal therapy of castration sensitive metastatic prostate cancer (mCRPC) or in combination with external beam radiotherapy of primary prostate cancer. This appears feasible because DNA repair defects are often present already at the time of prostate cancer diagnosis. Therefore, this invention leads not only to a

more effective palliative radionuclide therapy of advanced mCRPC, but can also serve for novel treatment approaches that result in long-term remissions in mCRPC and increase the likelihood of cure for high-risk localized prostate cancer.

Our invention centers on the development of a selective drug delivery system for PARPi to PC cells that express the prostate specific membrane antigen (PSMA). PSMA has already proven to be an effective cellular target for delivery of radioactive cargo for imaging and therapy. We disclose herein targeted drug conjugates that deliver a PARPi, e.g. Rucaparib (Ruc), to tumors via PSMA, thereby inducing PARP inhibition preferably in PSMA+ cells. Consequently, limiting PARP inhibition to PSMA+ cells should enable safe and effective combination therapies, e.g. with radioligand therapy and other PC treatment.

Our invention is also highly suitable with respect to other tumor types, wherein said tumors in particular express the following targets: somatostatin receptor (SSTR subtypes 2, 3, and 5), gastrin-releasing peptide receptor (GRPR), C-X-C motif chemokine receptor 4 (CXCR4), αvβ6-integrin, αvβ8-integrin, α5β1-integrin, αvβ1-integrin, folate receptor (FR), neurotensin receptor 1 (NTSR1), and/or melanocortin receptor 1 (MC1R). In these instances, conjugates of PARPi with molecules targeting the aforementioned targets will be employed alone or in combination with other therapeutic agents for the respective disease entities.

*Synthesis of PSMA-Ruc conjugates and their characterization*

We successfully synthesized a library of 18 PSMA-Ruc conjugates and examined their potential for PSMA-mediated Ruc delivery. As shown in Figure 2 and Table 1, the chemical composition of the conjugates include

- PSMA-targeting vectors based on EuK and EuE urea analogs (Robu *et al.,* 2018; Petrov *et al.,* 2021; Kelly *et al.,* 2017; Hensbergen *et al.,* 2020),
- different linkers for affinity/pharmacokinetic (PK) optimization, and
- different chelators (DOTAGA or multimodality chelator (MMC),

leading to branched or linear conjugates, respectively, that allow direct radiolabeling for quantitative assessment of binding and biodistribution.

We also synthesized conjugates with 2 Rucaparib moieties (Figure 3C). The structures of all synthesized conjugates are shown in Table 1.

Incorporation of a radiometal chelator into the bioconjugates allows to employ the same methodology used during preclinical development of $^{68}$Ga/$^{177}$Lu-PSMA-617 (Umbricht *et al.,* 2017; Umbricht *et al.,* 2018).

The conjugates also contain a cleavable disulfide linker that undergoes glutathione (GSH) mediated cleavage for cytoplasmic release of Ruc in target cells.

**Table 1**

| | Formula | Spacer | Structure |
|---|---|---|---|
| ***Conjugate compounds with one Ruc*** | | | |
| ***- without chelator*** | | | |
| **7** | 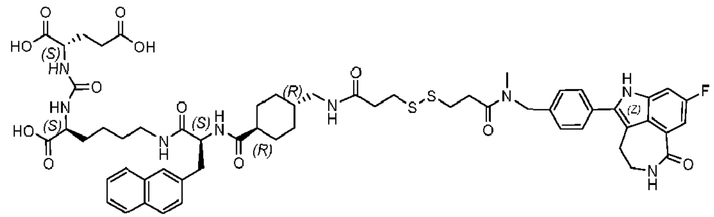 | Nal-TXA | |
| **8** | 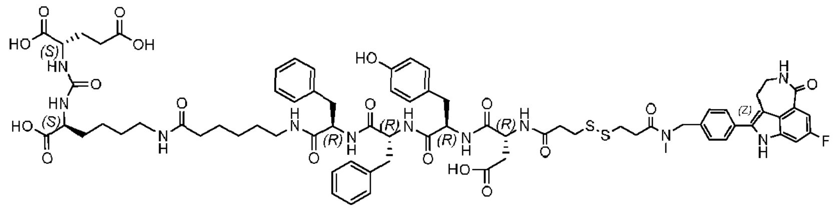 | Ahx-ffyd | |
| ***- with chelator*** | | | |
| **9** | 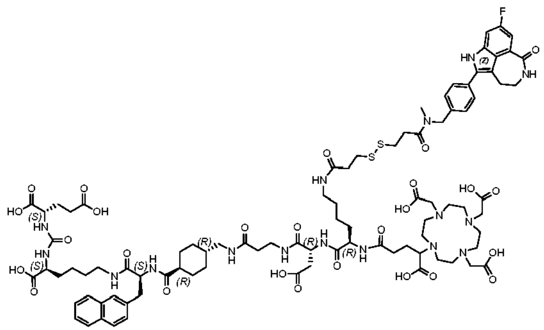 | Nal-TXA-βA-d | branched |

(continued)

| - with chelator | | | |
|---|---|---|---|
| **10** | 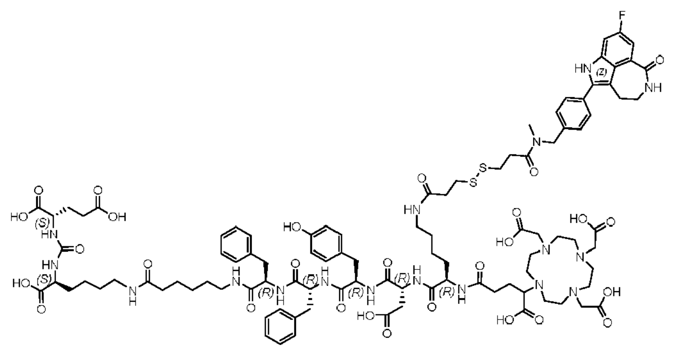 | Ahx-ffyd | branched |
| **11** | 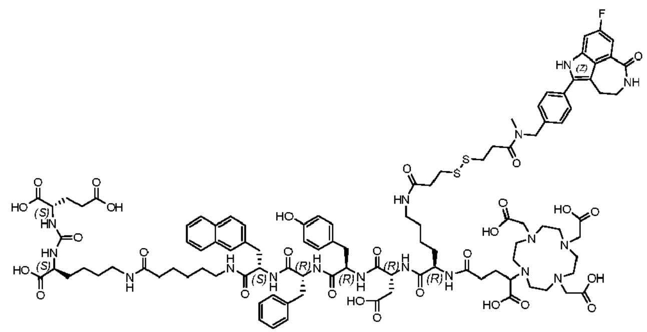 | Ahx-Nal-fyd | branched |
| **12** | 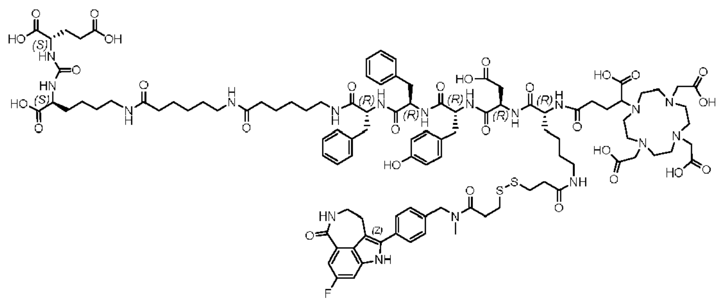 | Ahx-Ahx-ffyd | branched |

(continued)

| | | | |
|---|---|---|---|
| ***- with chelator*** | | | |
| **13*** | 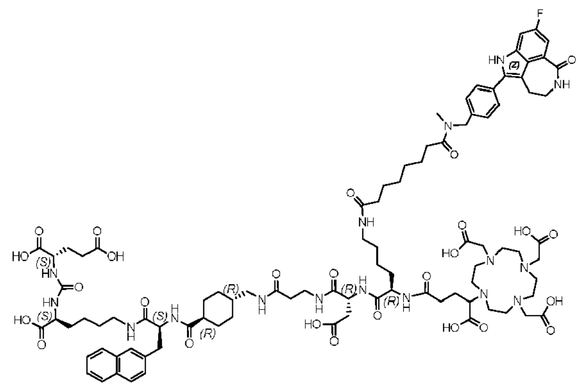 | Nal-TXA-βA-d | branched |
| **14** | 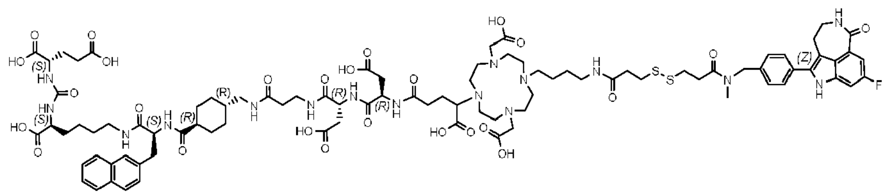 | Nal-TXA-βA-dd | linear |
| **15** | 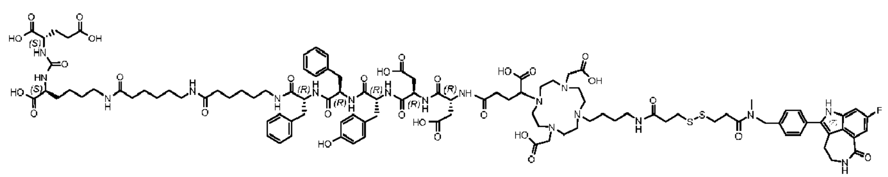 | Ahx-Ahx-ffydd | linear |

(continued)

| - *with chelator* | | | |
|---|---|---|---|
| **16** | 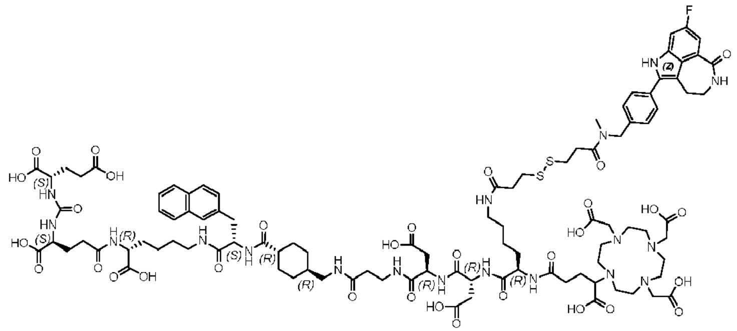 | k-Nal-TXA-βA-dd | branched |
| **17** | 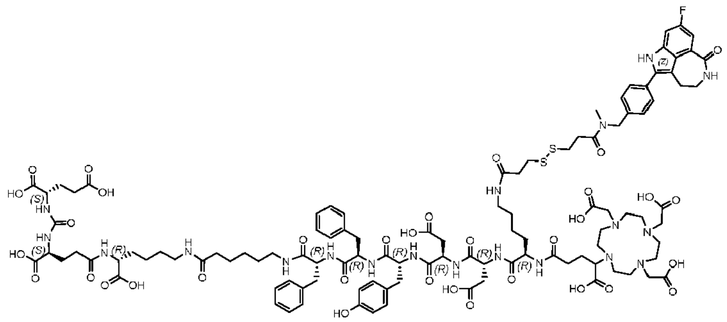 | k-Ahx-ffydd | branched |
| **18** | 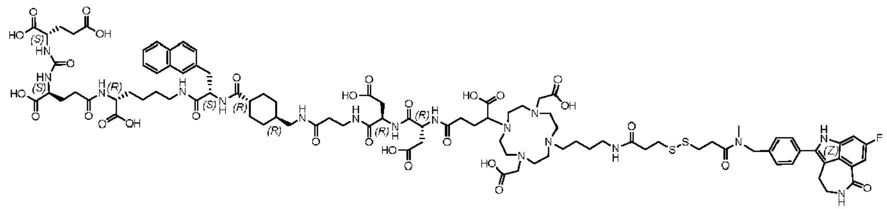 | k-Nal-TXA-βA-dd | linear |

(continued)

| Conjugate compounds with 2 Ruc and with chelator | | | |
|---|---|---|---|
| **10Lys** | 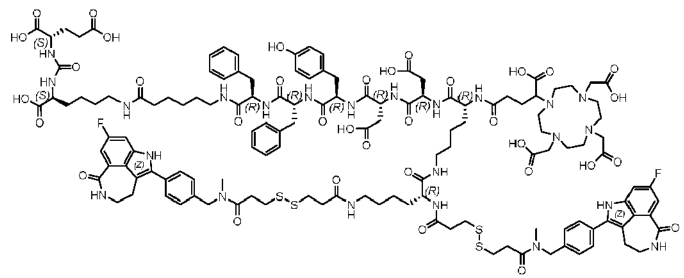 | Ahx- ffydd | branched |
| **11Lys** | 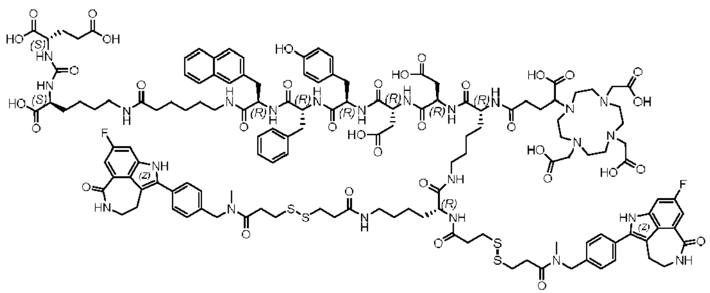 | Ahx-Nal-fydd | branched |
| **11LysSub*** | 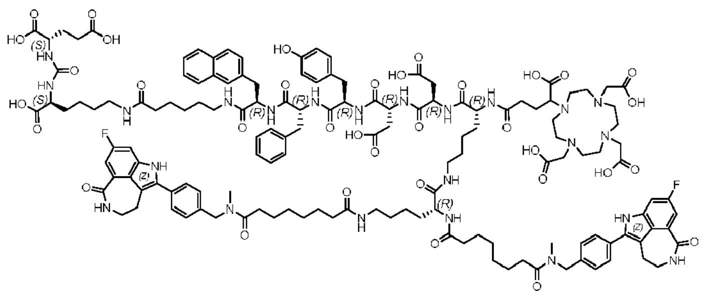 | Ahx-Nal-fydd | branched |

(continued)

| Conjugate compounds with 2 Ruc and with chelator | | | |
|---|---|---|---|
| **12Lys** | 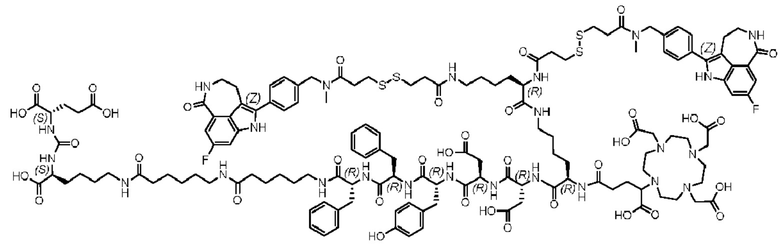 | Ahx-Ahx-ffydd | branched |
| **16Lys** | 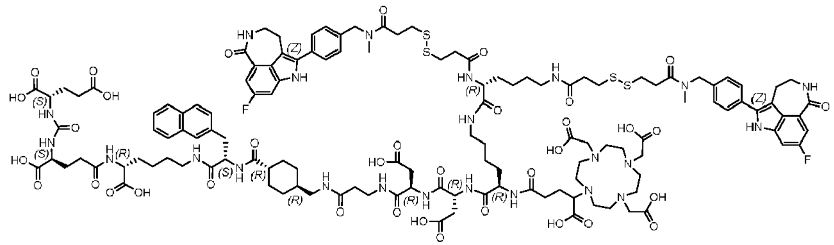 | k-Nal-TXA-βA-dd | branched |
| **17Lys** | 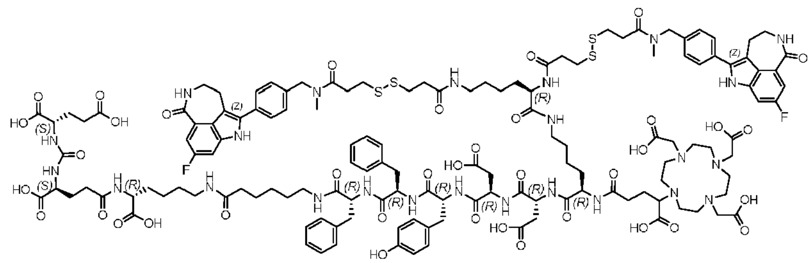 | k-Ahx-ffydd | branched |
| * Compounds 13 and 11LysSub are controls, they do not contain a cleavable bond. | | | |

Using the radiolabeled PSMA-Ruc conjugates, we confirmed that most conjugates exhibited binding and internalization properties that were comparable to $^{68}$Ga-PSMA-617 (Figure 3A) and could be blocked in the presence of the known PSMA inhibitor, 2-PMPA (Yao and Bacich, 2006) (Figure 3B). Labelling with non-radioactive Ga allowed further characterization of binding and revealed excellent binding affinities in the low nanomolar range (IC50: 1 to <10 nM), similar to $^{nat}$Ga-PSMA-617 (Table 2).

***Table 2:** Summary of ligand characterization, including cell uptake, affinity ($IC_{50}$) and logD-values.*

| Ligand | Spacer | % bound of total applied dose | $IC_{50}$ [nM] | logD (*n*-oct/PBS) |
|---|---|---|---|---|
| [$^{nat}$Ga] PSMA-617 | -Nal-TXA- | 10.23 ± 0.86 | 1.75 ± 0.16 | -3.62 ±0.49 |
| 7 | -Nal-TXA- | | 9.52 ± 2.41 | -2.98 ± 0.21 |
| 8 | -Ahx-ffyd- | | 1.58 ± 0.94 | -2.83 ± 0.07 |
| [$^{nat}$Ga]9 | -Nal-TXA-βA-d- | 8.00 ± 1.27 | 3.03 ± 0.20 | -2.10 ± 0.24 |
| [$^{nat}$Ga]10 | -Ahx-ffyd- | 6.18 ± 0.75 | 1.34 ± 0.18 | -2.33 ± 0.23 |
| [$^{nat}$Ga]11 | -Ahx-Nal-fyd- | 6.45 ± 1.16 | 5.34 ± 1.45 | -3.11 ± 0.08 |
| [$^{nat}$Ga]12 | -(Ahx)z-ffyd- | 3.48 ± 0.45 | 2.12 ± 0.52 | -2.36 ± 0.13 |
| [$^{nat}$Ga]13-NC | -Nal-TXA-βA-d- | 10.8 ± 2.07 | 0.67 ± 0.09 | -2.58 ± 0.05 |
| [$^{nat}$Ga]14 | -Nal-TXA-βA-dd- | 6.46 ± 0.98 | 2.19 ± 0.37 | -3.42 ± 0.15 |
| [$^{nat}$Ga]15 | -$(AhX)_2$-ffydd- | 6.50 ± 0.50 | 4.28 ± 0.95 | -3.40 ± 0.07 |
| [$^{nat}$Ga]16 | -k-Nal-c(Hex)-βA-dd- | 5.32 ± 0.11 | 0.82 ± 0.54 | -3.05 ± 0.14 |
| [$^{nat}$Ga]17 | -k-Ahx-ffydd- | 5.84 ± 0.59 | 1.38 ± 0.77 | -3.62 ± 0.49 |
| [$^{nat}$Ga]18 | -k-Nal-c(Hex)-βA-dd- | 6.54 ± 1.29 | 0.69 ± 0.48 | -2.98 ± 0.21 |

In a next step, we utilized the intrinsic fluorescence of Ruc to track its intracellular delivery as part of the PSMA-Ruc conjugates and translocation to the nucleus following cleavage of the disulfide bond by GSH. We observed that free Ruc localized in the cell nucleus of PSMA-expressing and non-expressing cells non-selectively, as expected. However, the Ruc-based fluorescence signal from PSMA-Ruc conjugates had a distinctly different localization pattern indicated by detection at the cell membrane (where PSMA is localized) and in the cell nucleus (where PARP1 is localized) of PC3-PIP cells (Figure 4A). No Ruc fluorescence was observed in PSMA-negative PC3-flu cells. Flow cytometry confirmed the microscopy findings and showed that incubation with PSMA-Ruc conjugates produced fluorescence only in PSMA-expressing cells, leading to an up to 40-fold higher Ruc accumulation in PC3-PIP compared to PC3-flu cells (Figure 4B). Conjugates with 2 Ruc-moieties (10Lys-17Lys) led to a higher amount of Ruc-related fluorescence than the corresponding single-Ruc variants, indicating that we can further increase the PSMA-dependent PARPi delivery of conjugates.

We determined the serum stability of PSMA-Ruc conjugates to confirm that Ruc will not be released from the conjugates *in vivo* before reaching PSMA-expressing tumor cells (Figure 5A). In addition, we showed that exposure to GSH indeed lead to effective release of Ruc within a 30 min incubation period by analyzing the occurrence of intact conjugate and free rucparib (Figure 5B).

*PSMA-Ruc conjugates induce selective cytotoxicity* in vitro

We tested the effect of PSMA-Ruc conjugates on prostate cancer cell lines to determine if i) they have a similar potency as free Rucaparib and ii) they can induce this cytotoxicity selectively (Figure 6).

We observed that free Ruc had similar cytotoxic effects on the cell viability in both PSMA+ (LNCaP) and PSMA- (PC3) cells, resulting in cell viability of 30-50% compared to untreated cells. In contrast, PSMA-Ruc conjugates only induced cytotoxic effects in PSMA+ cells, where the cell viability was 50-60% compared to untreated cells. In PSMA-negative cells, cell viability was >80% and similar to the untreated controls. Importantly, the non-cleavable control (conjugate 13), did not show cytotoxic effects in PSMA+ cells, demonstrating the need for intracellular release of Ruc to exert cytotoxic effects. Hence, PSMA-Ruc conjugates showed PSMA-selective cytotoxicity, while free Ruc did not. Differences in potency of monotherapies potentially arise from saturation of PSMA at the high concentrations of Ruc needed to exert cytotoxic effects in monotherapy.

*PSMA-Ruc in combination with $^{177}$Lu-PSMA treatment* in vitro.

We also tested the PSMA-Ruc conjugates in combination therapy with $^{177}$Lu-PSMA RLT to explore its radiosensitization potential in comparison to Ruc. Here, we used a low concentration of Ruc (1 μM; compared to 20 μM for mono-treatment) alone and in combination with $^{177}$Lu-PSMA RLT (0.1875 MBq/well). We tested the treatment combination in two PSMA-positive (LNCaP, C42) and PSMA-negative (DU145, PC3) cell lines, to explore the PSMA-specificity of the observed effects. Figure 7 shows an example of the combination therapy effects of conjugate 15, which shows strongly synergistic cytotoxicity in a PSMA-dependent manner. Combination with free Rucaparib showed similar synergistic cytotoxic effects, but was not PSMA-dependent. We subsequently analyzed the synergistic cytotoxicity in 3 PSMA-positive (LNCaP, C42, PC3-PiP) and 2 PSMA-negative (PC3, DU145) cell lines (Figure 8). Hereby, we defined synergistic cytotoxicity as a viability lower than treatment with RLT only and lower than 50% compared to vehicle treated cells at the end of the 72h treatment period. In LNCaP cells, which have high PSMA expression and the highest degree of HRD (and should therefore be sensitive to PARP inhibition), all conjugates exhibited synergistic cytoxicity comparable or stronger than Ruc + RLT. In C42 cells, 6 conjugates exhibited synergistic cytotoxicity and in PC3-PiP cells 7 exhibited synergistic cytotoxicity. Conjugates 11, 12, 14, 15 and 18 showed synergistic cytotoxicity in all PSMA-positive cell lines, as did Ruc + RLT. In the PSMA-negative cell lines, none of the conjugates showed synergistic cytoxicity, while Ruc+RLT showed synergistic cytoxicity in PC3 cells, despite the absence of PSMA. The non-cleavable control (referred to as 13 or 13-NC) did not show synergistic cytotoxicity in any of the tested cell lines.

*PSMA-selective* in vivo *imaging using a PSMA-Ruc conjugate*

Based on *in vitro* data that identified PSMA-Ruc-11 as a promising agent for *in vivo* testing, we performed an imaging study with $^{68}$Ga-PSMA-Ruc-11 (Figure 9). We prepared the PC3-PIP/PC3-flu bilateral tumor model that is routinely used in proof-of-concept studies with PSMA-targeted agents (Chen *et al.,* 2011; Banerjee *et al.,* 2014; Harmatys *et al.,* 2018; Ray Banerjee *et al.,* 2016). PET/CT imaging revealed $^{68}$Ga-PSMA-Ruc-11 uptake in PSMA+ PC3-PIP tumors and no accumulation in PSMA- PC3-Flu tumors at 60 min p.i. (Figure 9A). These findings demonstrate retention of PSMA-targeting properties after payload conjugation. We also made the observation that the biodistribution profile of $^{68}$Ga-PSMA-Ruc-11 was similar to $^{68}$Ga-PSMA-617, with uptake limited to PSMA-expressing tumors and kidneys, and fundamentally different from the typical biodistribution of PARPi (e.g., $^{18}$F-Olaparib, Wilson *et al.* 2019), which is characterized by low tumor uptake and high gut uptake. We subsequently also conducted an imaging and biodistribution study with $^{68}$Ga-PSMA-Ruc-9, $^{68}$Ga-PSMA-Ruc-16 and $^{68}$Ga-PSMA-Ruc-18 using the PC3-PIP/PC3-flu bilateral tumor model (Figure 10). Conjugates 16 and 18 showed selective uptake in PC3-PiP tumors and low uptake in PC3-Flu tumors and other organs, resulting in PiP:Flu ratios of >8 and PiP/muscle ratios >25 60 min p.i. The selective accumulation of the PSMA-targeted Ruc conjugates in PSMA-expressing tumors could also be observed on PET/CT images (Figure 10B) and the full biodistribution data (Figure 10C).

The following examples and drawings illustrate the present invention without, however, limiting the same thereto.

BRIEF DESCRIPTION OF THE DRAWINGS

**Figure 1.** *Characteristics of the PARP inhibitor Rucaparib (Ruc).*
Ruc (A) is intrinsically fluorescent, which enables intracellular drug-tracking (B) and quantification, since fluorescence intensity is correlated with the Ruc concentration (C). MFI - mean fluorescence intensity.

**Figure 2.** *Design of the PSMA-Ruc conjugates of the invention.*
Design of linear (A) and branched (B) PSMA-Ruc conjugates and conjugates with two Ruc moieties (C).

**Figure 3.** *Characterization of PSMA-binding properties of PSMA-Ruc conjugates in LNCaP cells.*
Conjugates 9-13 are branched and 14-15 are linear. Conjugate 13 is a non-cleavable control.

(A) Binding and internalization after 60 min incubation with 0.4 nM $^{68}$Ga-labelled conjugates.
(B) Binding specificity was determined via PMPA blocking. Displayed are mean values of three biological replicates.

**Figure 4.** *PSMA-dependent delivery of Ruc via PSMA-Ruc conjugates.*

(A) After incubation with conjugate 11, only PSMA+ cells showed Ruc fluorescence at the membrane and in the cell nucleus, where PARP1 is located. Free Ruc showed non-selective uptake in PSMA+ and PSMA- cells.
(B) Flow cytometry confirmed the PSMA-dependency of uptake of PSMA-Ruc conjugates. PC3-PIP or PC3-flu were incubated with 0.5 μM Ruc or PSMA-Ruc conjugate for 30 min before flow cytometry. NC - non-cleavable.

**Figure 5.** *Stability of PSMA-Ruc conjugates in serum and after GSH exposure.*

(A) After incubation in human serum, PSMA-Ruc conjugate 11 remains largely stable (93%) for 2 hours and is still 74% intact after 24 hours. The non-cleavable control 13 also remained stable (2h: 94%, 24h: 89%).
(B) Exposure to GSH lead to effective release of Ruc. After 30 min of exposure 50-90% of the detected metabolites represented free Ruc.

**Figure 6.** *Cell viability after treatment of PSMA + and PSMA- cells*
with Ruc, PSMA-Ruc conjugates or controls at 20 μM for 72h.
Ruc: free Rucaparib, 7-12: branched PSMA-Ruc conjugates, 13: non-cleavable control, 14-15: linear PSMA-Ruc conjugates. All cells were pretreated with 10 mM GSH and contained 0,2% DSMO.

**Figure 7.** *Cell viability in PSMA+ and PSMA- cell lines.*
Cells (PSMA+: LNCaP, C42; PSMA-: DU145, PC3) were treated for 72 h with 1 μM free Ruc, 1 μM Conj. 15, 0.1875 MBq RLT($^{177}$Lu-PSMA-I&T) or the combination of both. Viability was assessed via Alamar Blue assay and calculated relative to untreated control. All samples (except untreated control) contained 10 mM GSH, 0,2% DSMO. PDC-peptide drug conjugate.

**Figure 8.** *Assessment of cytotoxicity of PSMA-Ruc conjugates with one Ruc moiety per ligand.*
Cells (PSMA+: LNCaP, C42, PC3-PiP; PSMA-: DU145, PC3) were treated for 72 h with 1 μM free Ruc, 1 μM Conj. 15, 0.1875 MBq RLT($^{177}$Lu-PSMA-I&T) or the combination of both. Viability was assessed via Alamar Blue assay and calculated relative to the vehicle control (containing 10 mM GSH and 0,2% DMSO). NC-non-cleavable control. Synergistic cytotoxicity was defined as viability lower than RLT only (dotted line) and lower than 50% (solid line). Conditions with synergistic cytoxicity are represended by dotted bars.

**Figure 9.** *Proof-of-concept of PSMA-dependent Ruc delivery in vivo.*
PET (A) and biodistribution (B) data show specific PSMA-targeting of $^{68}$Ga-PSMA-Ruc-11, where uptake is limited to PSMA+ tumors and kidneys (n=3 mice). $^{68}$Ga-PSMA-Ruc-11 showed no organ uptake other than kidneys, in contrast to free PARPi, e.g. $^{18}$F-Olaparib, which shows high liver and gut uptake (see Wilson *et al.* (2019)).

**Figure 10**. *In vivo imaging and biodistribution data of several PSMA-Ruc conjugates in vivo.*
Tumor-to-organ ratios (A), PET (B, *n*=2 mice/group) and biodistribution (C, *n*=5 mice/group) data show specific PSMA-targeting for $^{68}$Ga-PSMA-Ruc-9, $^{68}$Ga-PSMA-Ruc-16 and $^{68}$Ga-PSMA-Ruc-18 where uptake is limited to PSMA+ tumors and kidneys at 1 h post-injection.

EXAMPLES

EXAMPLE 1 Synthesis of PSMA-Ruc conjugates and their characterization

***General information***

*Chemical synthesis*. For the reactions performed in this work, all protected amino acid analogs and coupling reagents were purchased from Carbolution Chemicals (St. Ingbert, Germany), Iris Biotech (Marktredwitz, Germany), Alfa Aesar (Karlsruhe, Germany) or Bachem (Bubendorf, Switzerland). The 2-chlorotrityl chloride (2-CTC) resin was obtained from Carbolution Chemicals. DOTAGA-anhydride was delivered by Chematech (Dijon, France). Rucaparib was obtained from Selleckchem (Berlin, Germany), and PSMA-617 from MedChemExpress (Sollentuna, Sweden), respectively. All solvents were purchased from Sigma-Aldrich (Munich, Germany), VWR (Darmstadt, Germany), Carl Roth (Karlsruhe, Germany) or Iris Biotech, of analytical, for synthesis, peptide or HPLC grade and used without further purification. Unless otherwise noted, all other used chemicals and reagents for the performed syntheses were obtained from Sigma-Aldrich, VWR, Merck (Darmstadt, Germany), abcr (Karlsruhe, Germany) or Alfa Aesar. Solid phase syntheses were performed in 20 mL plastic syringes from B. Braun, which were equipped with a frit from Roland Vetter Laborbedarf (Ammerbuch, Germany). By drawing up the syringe, reaction or wash solutions were added to the resin. Complete mixing was accomplished on a rotating unit. The equivalents (eq.) of amino acids were referred to the theoretical loading given by the manufacturer. Column chromatography was performed using a 100-fold excess of silica gel (40-63 μm grain size, Si 60, 230-400 mesh ASTM) from Merck at 1 bar overpressure. The respective eluent ratios were described in the experimental procedures. For detection of product fractions after column chromatography, thin-layer reaction controls (TLC) were performed on aluminum foils coated with silica gel 60 $F_{254}$ obtained from Merck using different mixtures of organic solvents. The respective eluent ratios were described in the experimental procedures as well. Via

UV absorption at 254 nm the substances were hereby detected. **Semi-preparative reversed phase high-performance liquid chromatography** (RP-HPLC) was realized on the following devices:

a) Waters (Milford, USA) instrument including a Waters 2545 (Binary Gradient Module), Waters SFO (System Fluidics Organizer), Waters 2996 (Photodiode Array Detector), and Waters 2767 (Sample Manager), respectively. The applied flow rate was 40 mL/min.
b) Shimadzu (Shimadzu Deutschland GmbH, Neufahrn, Germany) RP-HPLC system including a CBM-20A communications bus module, SPD-M20A prominence diode array detector, LC-20AP prominence preparative liquid chromatograph (for both pumps). The applied flow rate was 20 mL/min.

As eluents linear gradients of water (H2O; with 0.1% (v/v) trifluoroacetic acid (TFA), buffer A) and acetonitrile (MeCN; with 0.1% (v/v) TFA, buffer B) were applied for the purification of all compounds using a C18-column (Reprosil 100 C18, 5 μm, 150 × 30 mm, Dr. Maisch, Ammerbuch-Entringen, Germany). The UV-detection was carried out at 220, and 254 nm, respectively. All final compounds were analyzed via electrospray ionization mass spectrometry (ESI-MS) coupled to an analytical RP-HPLC system (HPLC-ESI-MS) to confirm a purity of ≥95% ($\lambda$ = 220 and 254 nm). Here, the LC-MS spectra were acquired on two devices:

a) UltiMate 3000 UHPLC (Dionex, Sunnyvale, USA) equipped with a LCQ Fleet mass spectrometer (ThermoFisher Scientific, Waltham, USA) and two different C18 columns (Hypersil Gold aQ 175 Å, 3 μm, 150 mm × 2.1 mm or Accucore C18, 80 Å, 2.6 μm, 50 × 2.1 mm, constant flow 0.9 mL/min) from Thermo Scientific.
b) Shimadzu RP-HPLC system (DGU-20A$_3$ prominence degasser, LC-30AD Nexera liquid chromatograph (for both pumps), SIL-30AC Nexera autosampler, CBM-20A prominence communications bus module, RF-20A prominence fluorescence detector, CTO-20AC prominence column oven, SPD-M20A prominence diode array detector) coupled to a Shimadzu liquid chromatograph mass spectrometer LCMS-2020 under usage of a FCV-20AH$_2$ valve unit (Shimadzu). A constant flow rate of 0.75 mL/min (RP-HPLC coupled to MS) or 1 mL/min (RP-HPLC without MS) was applied. As column a C18 column from Dr. Maisch (ReproSil Gold 120, C18, 5 μm) was used.

For both systems, linear gradients of $H_2O$ (0.1% (*v*/*v*) TFA or formic acid) and acetonitrile (MeCN; 0.1% (*v*/*v*) TFA or formic acid) were used for analytical purposes (respective gradients are noted in the experimental procedures). For logD measurements, cell binding, and internalization as well as biodistribution studies, a Wizard$^{2®}$ 2480 automatic $\gamma$-counter from PerkinElmer (Waltham, USA) was used.

*Cell culture*. For cell culture, trypsin-EDTA (10 ×), Roswell Park Memorial Institute-1640 Medium (RPMI-1640), fetal calf serum (FCS), and phosphate buffered saline (1 ×, PBS; pH 7.4) were purchased from Gibco, ThermoFisher. Poly-L-lysine solution (0.01% in sterile water) was purchased from Merck, bovine serum albumin (BSA) from Carl Roth, and alamarBlue HS cell viability reagent from ThermoFisher Scientific, respectively. For cell studies, the following cell lines were used: prostate carcinoma cell lines LNCaP, PC3, PC3-PiP, C42, and DU145, respectively. The cell lines were purchased from American Type Culture Collection (ATCC, Manassas, USA) and from the German Collection of Microorganisms and Cell Cultures (DSMZ, Braunschweig, Germany). All cell lines were cultured in RPMI-1640 medium supplemented with 10% (v/v) heat inactivated FCS (v/v) (Gibco, ThermoFisher Scientific) and 1% (v/v) penicillin/streptomycin (10,000 U/mL, Gibco, ThermoFisher Scientific) in a 37 °C incubator with a 5% (g/v) $CO_2$ atmosphere.

***Synthesis section and analytical data***

**General procedures**

**GP-1: Loading of 2-CTC resin**: Solid phase peptide synthesis (SPPS) was performed according to standard Fmoc-strategy (Merrifield, 1963; Carpino and Han, 972). First, dry 2-CTC resin (1.0 eq.; 0.97 mmol/g) was mixed with a solution of the Fmoc-protected amino acid derivative (Fmoc-AA-OH; 1.2 eq.) and N,N-diisopropylethylamine (DIPEA; 2.5 eq.) in anhydrous (anhyd.) dichloromethane (DCM; 10 mL/g resin) at room temperature (RT) for 2 h under constant shaking. Next, unconjugated 2-chlorotritylchloride groups were capped by addition of methanol (MeOH; 1.0 mL/g resin) and DIPEA (0.2 mL/g resin). After shaking the resin for 15 min, the resin was filtered, and washed with DCM (3 ×), *N*,*N*-diisopropylethylamine (DMF; 3 ×), DMF/MeOH 1: 1 (*v*/*v*) (1×), and MeOH (3 ×), respectively. Subsequent drying under vacuum overnight enabled the calculation of resin loading (n):

$$n = \frac{(m_2 - m_1) \times 1000}{(M_{AA} - M_{HCl}) \times m_2}$$

**Formula 1**. Determination of resin loading n [mmol/g]. $m_1$: mass of free resin [g]; $m_2$: mass of loaded resin [g]; $M_{AA}$: molecular weight of Fmoc-protected amino acid derivative [g/mol]; $M_{HCl}$: molecular weight of HCl [36.46 g/mol].

In cases the resin loading was not determined, a resin loading of 100% was assumed. Hereby, after capping, the resin was washed with DCM (3×), and DMF (5×), respectively.

**GP-2: On-resin Fmoc-deprotection:** The resin was treated with a solution of 20% (v/v) Piperidin in DMF (1 × 15 min, 1×10 min) at RT. Afterwards, the resin was washed with DMF (8×).

**GP-3: On-resin peptide synthesis with HATU/HOBt:** A solution (0.1-0.2 M) of Fmoc-AA-OH (2.0 eq.), O-(7-azabenzotriazol-1-yl)-*N*,*N*,*N*',*N*'-tetramethyluroniumhexafluorophosphate (HATU; 2.0 eq.), 1-hydroxybenzotriazole (HOBt×$H_2O$; 2.0 eq.), and DIPEA (5.0 eq.), respectively, in DMF was stirred for 15 min at RT. The pre-activated solution was added to the resin-bound, free amine and shaken for 1 h at RT. Subsequently, the resin was filtered and washed with DMF (5×). For coupling with resin-bound secondary amines, a pre-activated solution (0.1-0.2 M) of Fmoc-AA-OH (3.5 eq.), HATU (3.5 eq.), HOBt×$H_2O$ (3.5 eq.), and DIPEA (7.0 eq.), respectively, was added to the resin. After 3 h of shaking at RT, the resin was washed with DMF (5×).

**GP-4: Cleavage of linear peptides from the resin:** For complete cleavage, the resin was first washed with DCM (3×) and then treated with a solution of 20% (v/v) hexafluoroisopropanol in DCM (1×15 min, 2×10 min). In a next step, the solvent was evaporated under reduced pressure.

**GP-5: Trityl-, Pbf-, Boc-, and *t*Bu-deprotection in solution**: The starting material was dissolved in a mixture of TFA/DCM/triisopropylsilane (TIPS)/$H_2O$/DCM (90/5/2.5/2.5, v/v/v) and stirred for 60 min at RT. The progress of the reaction was monitored by HPLC-ESI-MS. The reaction was stopped by addition of an excess of toluene. Afterwards, the volatiles were removed under reduced pressure. End products were purified via semipreparative RP-HPLC and lyophilized, resulting in TFA salts.

**GP-6: Coupling of TFP-esters in solution:** A solution of the free amine (1.0 eq.) and DIPEA (7.0 eq.) in DMF (1.3 mM) was preincubated for 5 min at RT. Afterwards, the TFP-ester (1.5 eq.) was added, and the mixture was stirred for 16 h at RT. Finally, the crude product was purified via semipreparative RP-HPLC.

**GP-7: Standard amino acid coupling in solution:** The corresponding acid (1.2 eq.), HOBt×$H_2O$ (1.2 eq.), 2-(1H-benzotriazole-1-yl)-1,1,3,3-tetramethylaminium tetrafluoroborate (TBTU; 1.2 eq) as well as DIPEA (5.0 eq.) were dissolved in DMF (0.12 M), and incubated for 15 min at RT. The pre-activated solution was added to the deprotected, free amine (1.0 eq.), dissolved in a small amount of DMF. After stirring the reaction solution for 16-24 h at RT, the solvent was evaporated. The hereby remaining residue was redissolved in EtOAc and extracted with saturated (sat.) ammonium chloride ($NH_4Cl$)-, sat. $NaHCO_3$- as well as sat. NaCl-solution (each 1×). In a next step, the organic phase was dried over $Na_2SO_4$, filtered, and the solvents were removed under reduced pressure, respectively. In case of deprotected starting materials, the extraction was not performed. Here, after the reaction step, the volatiles were removed under reduced pressure and the product was purified via semipreparative RP-HPLC.

**GP-8: Reductive deprotection in solution:** To a solution of the starting material in MeOH palladium on carbon (10% (w/w) Pd/C) was added. The resulting suspension was hydrated by flushing the flask with hydrogen ($H_2$; 1 atm.) for 2-4 h. Reaction progress was monitored by analytical HPLC-ESI-MS. After removal of the catalyst via filtration over Celite®, the solvent was removed under reduced pressure. The crude product was directly used in the next reaction step without further purification.

**GP-9: On-resin Dde-deprotection:** The orthogonal Dde-deprotection was performed by treating the resin with a solution of hydrazine hydrate (2% (v/v)) in DMF for 15 min. The step was repeated for another 15 min with fresh hydrazine solution. The reaction progress was monitored by HPLC-ESI-MS. After completion of deprotection, the resin was washed with DMF (8×).

**GP-10: Preparation of non-radioactive $^{nat}$Ga-complexed PSMA-ligands**: A solution of the PSMA-inhibitor (2.0 mM; 10 μL) in dimethyl sulfoxide (DMSO) and a solution of $Ga(NO_3)_3$ (2.0 mM; 10 μL) in $H_2O$/DMSO (16% $H_2O$ (v/v)) were mixed and heated at 45 °C for 40 min under shaking. Chelate formation was analyzed via RP-HPLC and ESI-MS. The resulting 1.0 mM solution was used without further purification in *in vitro* experiments.

**Building blocks for EuK- and EuE-based PSMA ligands**

*Rucaparib containing building blocks*

**3-((3-((4-(8-Fluoro-1-oxo-2,3,4,6-tetrahydro-1*H*-azepino[5,4,3-cd]indol-5-yl)benzyl)-(methyl)amino)-3-oxopropyl)disulfaneyl)propanoic acid (Ruc-SS-OH) (1):**

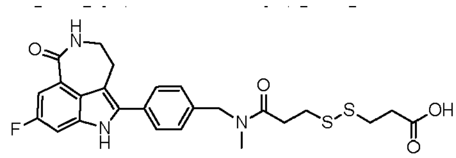

Chemical Formula: $C_{25}H_{26}FN_3O_4S_2$
Exact Mass: 515,13
Molecular Weight: 515,62

To a solution of rucaparib*HCl (40.0 mg, 112 μmol, 1.0 eq.) in DMF (15 mL), 3,3-dithiopropionic acid (47.0 mg, 222 μmol, 2.0 eq.) was added (**GP-7**). After evaporation of the solvents under reduced pressure and semipreparative RP-HPLC purification (35-60% buffer B, 15 min, buffer A and B without TFA addition), pure **1** (43.4 mg, 84.3 μmol, 76%) was obtained as yellow powder.

**RP-HPLC** (5-95%, 10 min): $t_R$ = 7.79 min. **MS** (ESI, positive): m/z = 516.4 [M+H$^+$], 1031.4 [2M+H$^+$].

**2,3,5,6-tetrafluorophenyl3-((3-((4-(8-fluoro-1-oxo-2,3,4,6-tetrahydro-1H-azepino [5,4,3-cd]indol-5-yl)benzyl)(methyl)amino)-3-oxopropyl)disulfaneyl)propanoate (Ruc-SS-OTFP) (2)**:

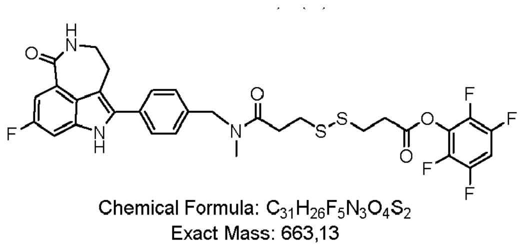

Chemical Formula: $C_{31}H_{26}F_5N_3O_4S_2$
Exact Mass: 663,13
Molecular Weight: 663,68

**1** (5.00 mg, 9.70 μmol, 1.0 eq.) in anhydr. THF (1.5 mL) was cooled to 0 °C. *N,N'*-Dicyclohexylcarbodiimide (2.00 mg, 9.7 μmol, 1.0 eq.) and tetrafluorophenol (3.00 mg, 19.4 μmol, 2.0 eq.) were added subsequently. The mixture was stirred at RT for 16 h. After removal of the solvents *in vacuo*, semipreparative RP-HPLC (20-60% buffer B, 15 min, buffer A and B without TFA addition) led to pure **2** (4.16 mg, 6.28 μmol, 64%) in form of a beige powder.

**RP-HPLC** (5-95%, 10 min): $t_R$ = 6.77 min. **MS** (ESI, positive): m/z = 664.32 [M+H$^+$], 1326.48 [2M].

**8-((4-(8-Fluoro-1-oxo-2,3,4,6-tetrahydro-1*H*-azepino[5,4,3-cd]indol-5-yl)benzyl)(methyl)-amino-8-oxooctanoic acid (Ruc-Sub-OH) (3)**:

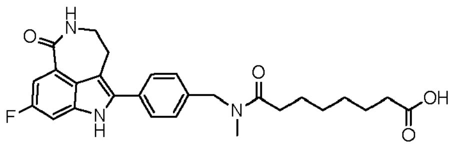

Chemical Formula: $C_{27}H_{30}FN_3O_4$
Exact Mass: 479,22
Molecular Weight: 479,55

For the preparation of **3**, rucaparib*HCl (15.0 mg, 41.7 μmol, 1.0 eq.) was coupled to suberic acid (15.0 mg, 83.4 μmol, 2.0 eq.) according to **GP-7**. After removal of the solvents under reduced pressure and semipreparative RP-HPLC purification (35-60% buffer B, 15 min, buffer A and B without TFA addition), **3** (6.12 mg, 12.7 μmol, 30%) was obtained as yellowish powder.

**RP-HPLC** (5-95%, 10 min): $t_R$ = 10.72 min. **MS** (ESI, positive): m/z = 480.3 [M+H$^+$], 959.60 [2M+2H$^+$].

**2,3,5,6-Tetrafluorophenyl-8-((4-(8-fluoro-1-oxo-2,3,4,6-tetrahydro-1*H*-azepino[5,4,3-cd]indol-5-yl)benzyl)(methyl)amino)-8-oxooctanoate (Ruc-Sub-OTFP)** (4):

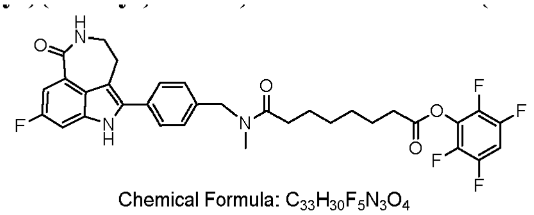

Chemical Formula: $C_{33}H_{30}F_5N_3O_4$
Exact Mass: 627,22
Molecular Weight: 627,61

As described for **2**, **3** (25.0 mg, 52.1 μmol, 1.0 eq.) gave **4** (14.6 mg, 23.2 μmol, 44%) after semipreparative RP-HPLC purification (20-60% buffer B, 15 min, buffer A and B without TFA addition).

**RP-HPLC** (5-95%, 10 min): $t_R$ = 5.13 min. **MS** (ESI, positive): m/z = 628.5 [M+H$^+$], 1255.8 [2M+H$^+$].

***N*-(4-(8-Fluoro-1-oxo-2,3,4,6-tetrahydro-1*H*-azepino[5,4,3-cd]indol-5-yl)benzyl)-3-mercapto-*N*-methylpropanamide (Ruc-SH) (5):**

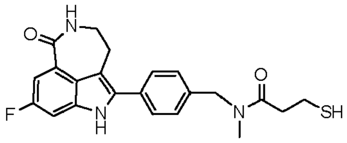

Chemical Formula: $C_{22}H_{22}FN_3O_2S$
Exact Mass: 411,14
Molecular Weight: 411,50

Rucaparib*HCl (5.00 mg, 13.9 μmol, 1.0 eq.) and 3-mercaptopropionic acid (1.60 mg, 14.5 μmol, 1.05 eq.) were coupled according to **GP-7**. After evaporation of the solvents under reduced pressure and semipreparative RP-HPLC purification (35-60% buffer B, 15 min, buffer A and B without TFA addition), **5** (1.30 mg, 3.16 μmol, 22%) was obtained as yellow powder. **RP-HPLC** (10-95%, 15 min): $t_R$ = 12,55 min. **MS** (ESI, positive): m/z = 412.2 [M+H$^+$].

*The PSMA targeting motif*

**(S)-Di-*tert*-butyl-2-(1*H*imidazole-1-carboxamido)pentanedioate (6):**

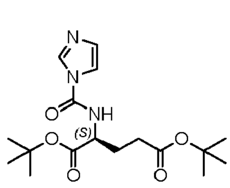

Chemical Formula: $C_{17}H_{27}N_3O_5$
Exact Mass: 353,20
Molecular Weight: 353,42

The synthesis of imidazole **6** was performed as previously described (Weineisen et al., 2014). Briefly, a solution of L-di-*tert*-butylglutamate*HCl (500 mg, 1.69 mmol, 1.0 eq.) in DCM (15.0 mL) was first treated with $NEt_3$ (600 μL, 427 mg, 4.22 mmol, 2.5 eq.) and 4-(dimethylamino)pyridine (DMAP; 8.00 mg, 67.6 μmol, 0.04 eq.) at 0 °C. After stirring the reaction mixture for 5 min, 1,1'-carbonyldiimidazole (CDI; 328 mg, 2.02 mmol, 1.2 eq.), dissolved in DCM, was slowly added over a period of 20 min. Subsequently, the mixture was stirred for 20 h under warming to RT. The reaction was

then stopped with sat. $NaHCO_3$-solution (8.00 mL) and the organic phase was washed with $H_2O$ (2×), sat. NaCl-solution (2×), dried over $Na_2SO_4$ as well as filtrated. Evaporation of the solvents under reduced pressure led to **6** (529 mg, 1.49 mmol, 88%) as colorless oil. The crude product was directly used without further purification steps.

**RP-HPLC** (5-95%, 8 min): $t_R$ = 2.07 min. **MS** (ESI, positive): m/z = 355.14 [$M+2H^+$], 708.89 [$2M+H^+$].

***N*$^2$-(((*S*)-1,5-di-*tert*-butoxy-1,5-dioxopentan-2-yl)carbamoyl)-*N*$^6$-(1-(4,4-dimethyl-2,6-dioxocyclohexylidene)ethyl)-L-lysine (K(Dde)uE(O$^t$Bu)$_2$) (7):**

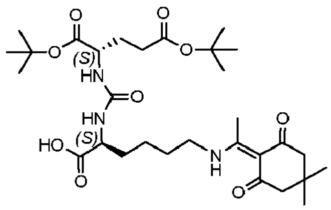

Chemical Formula: $C_{30}H_{49}N_3O_9$
Exact Mass: 595,35
Molecular Weight: 595,73

The synthesis of 7 was accomplished similarly to a literature procedure (Weineisen et al., 2014). 2-CTC resin (2.00 g, 1.93 mmol, 1.0 eq; resin loading: 0.97 mmol/g resin) was loaded with Fmoc-L-Lys(Dde)-OH (1.30 g, 2.32 mmol, 1.2 eq.) according to **GP-1**. After Fmoc-removal **(GP-2)**, a solution of **6** (1.37 g, 3.87 mmol, 2.0 eq.) and $NEt_3$ (700 μL, 450 mg, 4.45 mmol, 2.3 eq.) in dichloroethane (DCE; 20.0 mL), cooled to 0 °C, was added to the resin. The resin was shaken for 16 h at RT. Afterwards, the resin was washed with DCE (5×) and DMF (5×). Resin-bound 7 was used for the generation of the KuE-derived PSMA-ligands according to solid phase peptide synthesis/standard Fmoc-strategy.

**RP-HPLC** (5-95%, 8 min): $t_R$ = 3.30 min. **MS** (ESI, positive): m/z = 596.11 [$M+H^+$], 1212.65 [$2M+Na^+$].

**(*S*)-5-(*tert*-butoxy)-4-(3-((S)-1,5-di-*tert*-butoxy-1,5-dioxopentan-2-yl)ureido)-5-oxopentanoic acid ((O$^t$Bu)EuE(O$^t$Bu)$_2$) (8)**:

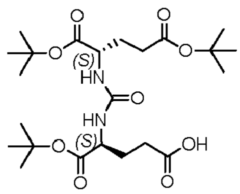

Chemical Formula: $C_{23}H_{40}N_2O_9$
Exact Mass: 488,27
Molecular Weight: 488,58

The synthesis of **8** was performed similarly to the literature (Weineisen et al., 2014; Robu et al., 2018). Here, **6** (1.80 g, 5.09 mmol, 1.0 eq.) in DCE (40.0 mL) was treated with $NEt_3$ (1.50 mL, 1.03 g, 10.1 mmol, 2.0 eq.) and H-L-Glu(Bzl)-O$^t$Bu*HCl (1.70 g, 5.09 mmol, 1.0 eq.) at 0 °C. The mixture was then stirred for 20 h at RT. After evaporation of the solvent under reduced pressure, the crude product was purified via column chromatography (EtOAc/cyclohexane/$NEt_3$ 500/500/0.8 (v/v/v)). Removal of the solvents yielded (O$^t$Bu)E(Bzl)uE(O$^t$Bu)$_2$ (2.52 g, 4.37 mmol, 86%) as colorless oil. The reductive deprotection was subsequently performed according to **GP-8**, leading to **8** (1.98 g, 4.06 mmol, 93%) as colorless oil.

**RP-HPLC** (5-95%, 10 min): $t_R$ = 9.71 min. **MS** (ESI, positive): m/z = 489.5 [$M+H^+$], 977.8 [$2M+H^+$].

***N*$^2$-((*S*)-5-(*tert*-butoxy)-4-(3-((S)-1,5-di-*tert*-butoxy-1,5-dioxopentan-2-yl)ureido)-5-oxo-pentanoyl)-*N*$^6$-(1-(4,4-dimethyl-2,6-dioxocyclohexylidene)ethyl)-L-lysine (K(Dde)-(O$^t$Bu)EuE(O$^t$Bu)$_2$) (9)**:

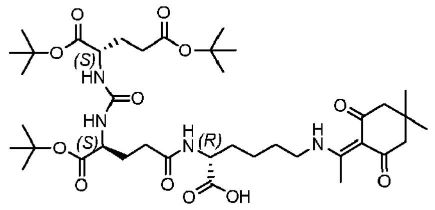

Chemical Formula: $C_{39}H_{64}N_4O_{12}$
Exact Mass: 780,45
Molecular Weight: 780,96

The synthesis **of 9** was accomplished similarly to a literature procedure (Weineisen et al., 2014; Robu et al., 2018). In short, 2-CTC resin (2.00 g, 1.93 mmol, 1.0 eq; resin loading: 1.03 mmol/g resin) was loaded with Fmoc-D-Lys(Dde)-OH (1.30 g, 2.32 mmol, 1.2 eq.) according to **GP-1.** After Fmoc-removal **(GP-2)**, the resin was treated with a solution of **8** (1.42 g, 2.90 mmol, 1.5 eq.) according to **GP-3.** Resin-bound **9** was used for the generation of EuE-based PSMA-ligands according to solid phase peptide synthesis/standard Fmoc-strategy.
**RP-HPLC** (5-95%, 10 min): $t_R$ = 9.45 min. **MS** (ESI, positive): m/z = 781.6 [M+H$^+$].

**Generation of the composite conjugates**

*Synthesis of EuK-based PSMA ligands*

**General synthesis procedure**. The peptide structures for the synthesis of the PSMA-targeting ligands were achieved in accordance with **GP-2**, **GP-3**, **GP-4**, **GP-5**, and **GP-9**, respectively, via standard solid phase peptide synthesis. As starting point, K(Dde)uE(O$^t$Bu)$_2$ (7) was Dde-deprotected according to **GP-9**. The coupling of further amino acid analogues or amino acids was achieved according to **GP-2** and **GP-3** following Fmoc-strategy. For DOTAGA coupling, the Fmoc-deprotected resin was treated with a solution of DOTAGA anhydride (4.0 eq.) and DIPEA (10 eq.) in DMSO (0.07 mM) for 48 h. Subsequently, the PSMA ligand was cleaved from the resin according to **GP-4.** After global deprotection of the acid labile protection groups, performed after **GP-5,** the ligands were purified via semipreparative RP-HPLC. Finally, the Ruc-derivative was coupled under basic conditions according to **GP-6** and the product was obtained after RP-HPLC purification.

**Ruc-SS-TXA-L-Nal-KuE (PSMARuc-7) (10):**

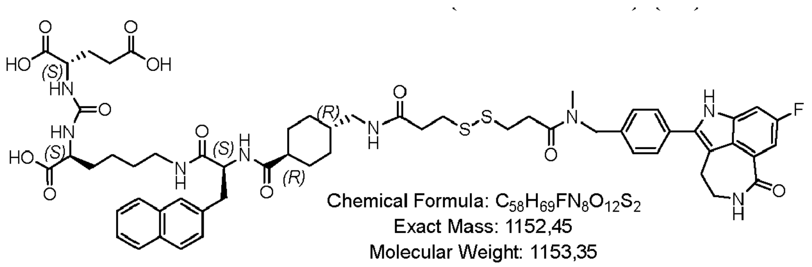

Chemical Formula: $C_{58}H_{69}FN_8O_{12}S_2$
Exact Mass: 1152,45
Molecular Weight: 1153,35

The peptide sequence was synthesized according to the general synthesis procedure. The following amino acid analogues were coupled to 7 (100 mg resin, 96.9 µmol, 1.0 eq.): *N*-Fmoc-3-(2-naphthyl)-L-alanin (Fmoc-L-Nal-OH), and trans-4-(Fmoc-aminome-thyl)-cyclohexanecarboxylic acid (Fmoc-TXA), respectively. After semipreparative RP-HPLC purification (20-60%, buffer B, 15 min), **2** (2.00 mg, 3.01 µmol, 1.5 eq.) was coupled according to **GP-6.** Via semipreparative RP-HPLC purification (20-60%, buffer B, 15 min, buffer A and B without TFA addition), **10** (1.41 mg, 1.22 µmol, 60%) was yielded as beige powder. **RP-HPLC** (10-95%, 15 min): $t_R$ = 11.64 min. **MS** (ESI, positive): m/z = 1154.5 [M+2H$^+$], 577.6 [M/2].

**RUC-SS-D-Asp-D- Tyr-D- Phe-D- Phe-Ahx-KuE (Ruc-SS-dyff-Ahx-KuE) (PSMARuc-8) (11):**

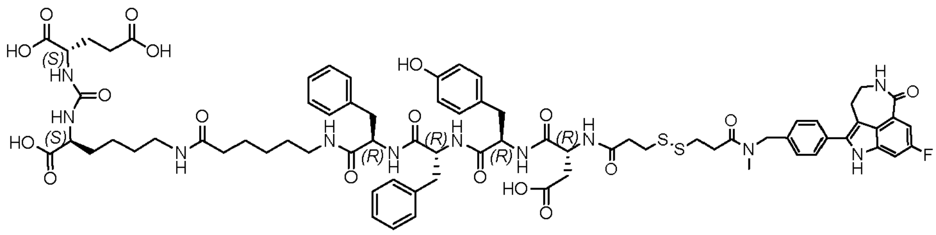

Chemical Formula: $C_{74}H_{88}FN_{11}O_{18}S_2$
Exact Mass: 1501,57
Molecular Weight: 1502,70

**11** was synthesized as **10** by coupling Fmoc-6-Ahx-OH, Fmoc-D-Phe-OH, Fmoc-D-Phe-OH, Fmoc-D-Tyr(O$^t$-Bu)-OH, and Fmoc-D-Asp(O$^t$Bu)-OH to 7 (100 mg resin, 96.9 μmol, 1.0 eq.). After semipreparative RP-HPLC purification (20-60%, buffer B, 15 min), **2** (2.00 mg, 3.01 μmol, 1.5 eq.) was coupled according to **GP-6.** Semipreparative RP-HPLC purification (20-60%, buffer B, 15 min, buffer A and B without TFA addition), yielded **11** (2.11 mg, 1.40 μmol, 69%) as beige solid.

**RP-HPLC** (10-95%, 15 min): $t_R$ = 11.32 min. **MS** (ESI, positive): m/z = 1503.6 [M+2H$^+$], 752.3 [M/2].

**DOTAGA-n-Lys(Ruc-SS)-n-Asp-β-Ala-TXA-L-Nal-KuE (DOTAGA-k(Ruc-SS)-d-βA-TXA-Nal-KuE) (PSMARuc-9) (12):**

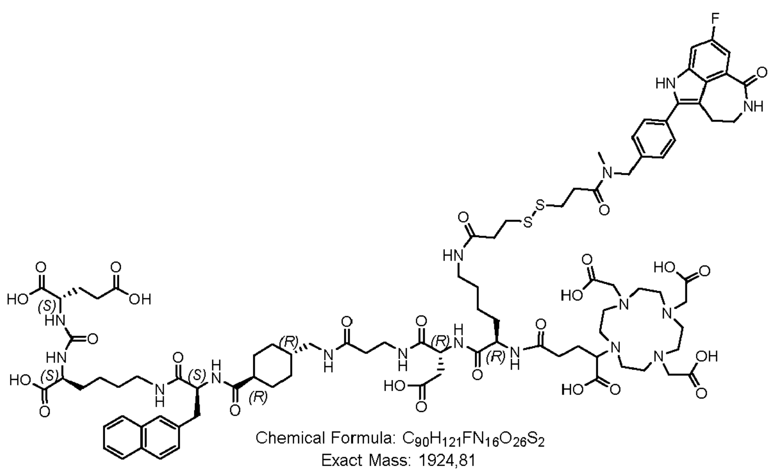

Chemical Formula: $C_{90}H_{121}FN_{16}O_{26}S_2$
Exact Mass: 1924,81
Molecular Weight: 1926,16

As already described for **10,** the following amino acid sequence was coupled to **7** (100 mg resin, 96.9 μmol, 1.0 eq.): Fmoc-L-Nal-OH, Fmoc-TXA, Fmoc-β-Ala-OH, Fmoc-D-Asp(O$^t$Bu)-OH, Fmoc-D-Lys(Boc)-OH, and DOTAGA-anhydride, respectively. After semipreparative RP-HPLC purification (20-60%, buffer B, 15 min), **2** (2.00 mg, 3.01 μmol, 1.5 eq.) was coupled according to **GP-6.** Semipreparative RP-HPLC purification (15-50%, buffer B, 15 min, buffer A and B without TFA addition) resulted in **12** (1.89 mg, 0.98 μmol, 48%) as white solid. **RP-HPLC** (10-95%, 15 min): $t_R$ = 9.81 min. **MS** (ESI, positive): m/z = 1926.7 [M+2H$^+$], 964.0 [M/2].

**DOTAGA-D-Lys(Ruc-SS)-D-Asp-D-Tyr-D-Phe-D-Phe-Ahx-KuE (DOTAGA-k(Ruc-SS)-dyff-Ahx-KuE) (PSMARuc-10) 13:**

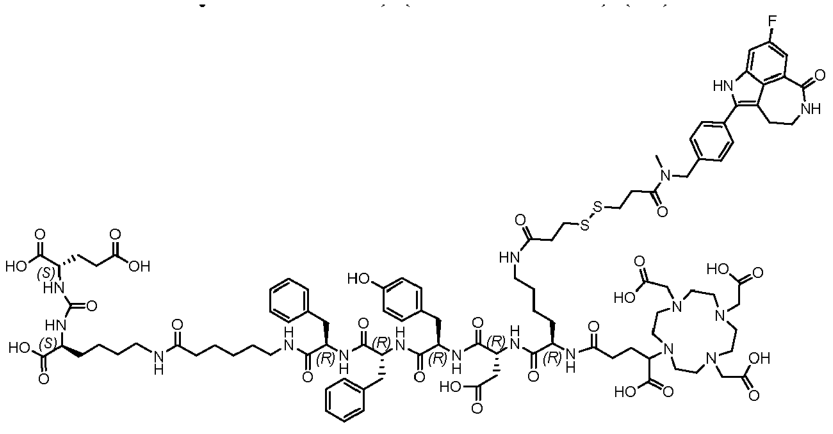

Chemical Formula: $C_{99}H_{130}FN_{17}O_{28}S_2$
Exact Mass: 2087,87
Molecular Weight: 2089,34

The synthesis of **13** was accomplished in similarity to **10**, using Fmoc-6-Ahx-OH, Fmoc-D-Phe-OH, Fmoc-D-Phe-OH, Fmoc-D-Tyr(O$^t$Bu)-OH, Fmoc-D-Asp(O$^t$Bu)-OH, Fmoc-D-Lys(Boc)-OH, and DOTAGA-anhydride, respectively. The crude product was purified via semipreparative RP-HPLC (20-60%, buffer B, 15 min), and 2 (2.00 mg, 3.01 μmol, 1.5 eq.) was coupled according to **GP-6.** Semipreparative RP-HPLC purification (15-50%, buffer B, 15 min, buffer A and B without TFA addition), yielded **13** (1.04 mg, 0.49 μmol, 24%) as white solid.

**RP-HPLC** (10-95%, 15 min): $t_R$ = 9.90 min. **MS** (ESI, positive): m/z = 1045.6 [M/2].

**DOTAGA-D-Lys(Ruc-SS)-D-Asp-D-Tyr-D-Phe-L-Nal-Ahx-KuE (DOTAGA-k(Ruc-SS)-dyf-Nal-Ahx-KuE) (PSMARuc-11) (14):**

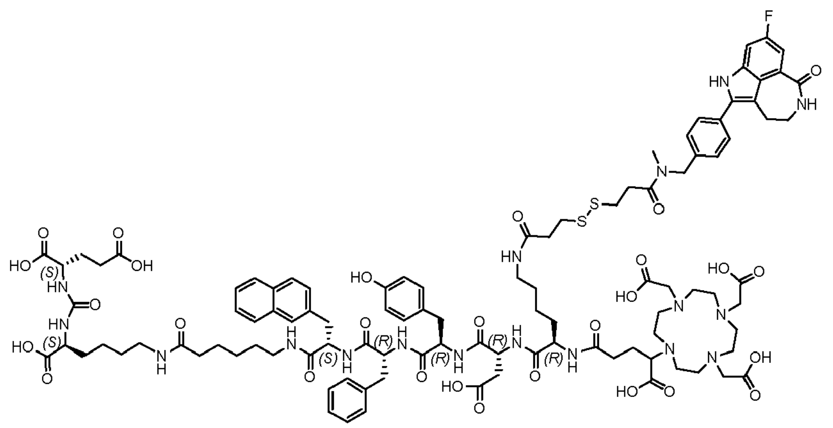

Chemical Formula: $C_{103}H_{132}FN_{17}O_{28}S_2$
Exact Mass: 2137,89
Molecular Weight: 2139,40

The synthesis of **14** was accomplished in similarity to **10,** using Fmoc-6-Ahx- OH, Fmoc-L-Nal-OH, Fmoc-D-Phe-OH, Fmoc-D-Tyr(O$^t$Bu)-OH, Fmoc-D-Asp(O$^t$Bu)-OH, Fmoc-D-Lys(Boc)-OH, and DOTAGA-anhydride, as amino acid analogues. After coupling of **2** (2.00 mg, 3.01 μmol, 1.5 eq.) according to **GP-6,** and semipreparative RP-HPLC purification (15-50%, buffer B, 15 min, buffer A and B without TFA addition), **14** (2.67 mg, 1.24 μmol, 62%) was aquired as white solid.

**RP-HPLC** (10-95%, 15 min): $t_R$ = 10.57 min. **MS** (ESI, positive): m/z = 1070.7 [M/2].

**DOTAGA-D-Lys(Ruc-SS)-D-Asp-D-Tyr-D-Phe-D-Phe-Ahx-Ahx-KuE (DOTAGA-k(Ruc-SS)-dyff-Ahx-Ahx-KuE) (PSMARuc-12) (15):**

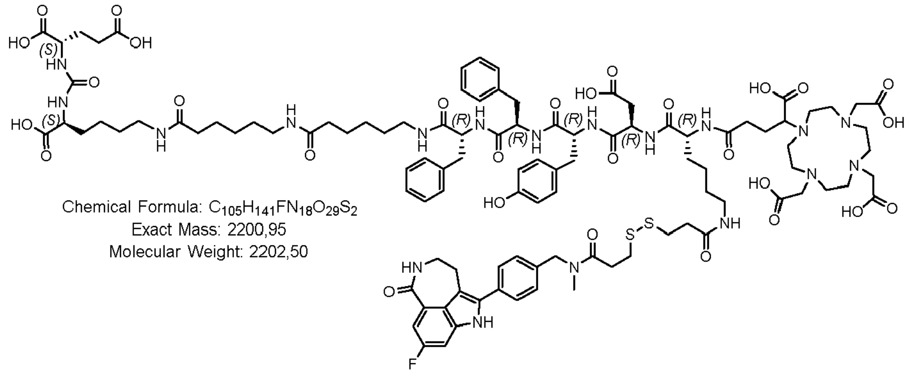

Chemical Formula: $C_{105}H_{141}FN_{18}O_{29}S_2$
Exact Mass: 2200,95
Molecular Weight: 2202,50

The synthesis of **15** was accomplished in similarity to **10,** using Fmoc-6-Ahx-OH, Fmoc-6-Ahx-OH, Fmoc-D-Phe-OH, Fmoc-D-Phe-OH, Fmoc-D-Tyr(O$^t$Bu)-OH, Fmoc-D-Asp(O$^t$Bu)-OH, Fmoc-D-Lys(Boc)-OH, and DOTAGA-anhydride. After coupling of **2** (2.00 mg, 3.01 µmol, 1.5 eq.) in accordance with **GP-6,** and semipreparative RP-HPLC purification (20-60%, buffer B, 15 min, buffer A and B without TFA addition), **15** (0.91 mg, 0.41 µmol, 20%) was obtained as beige solid.

**RP-HPLC** (10-95%, 15 min): $t_R$ = 9.88 min. **MS** (ESI, positive): m/z = 1102.2 [M/2].

**DOTAGA-n-Lys(Ruc-Sub)-n-Asp-β-Ala-TXA-L-Nal-KuE (DOTAGA-k(Ruc-Sub)-d-βA-TXA-Nal-KuE) (PSMARuc-13) (16):**

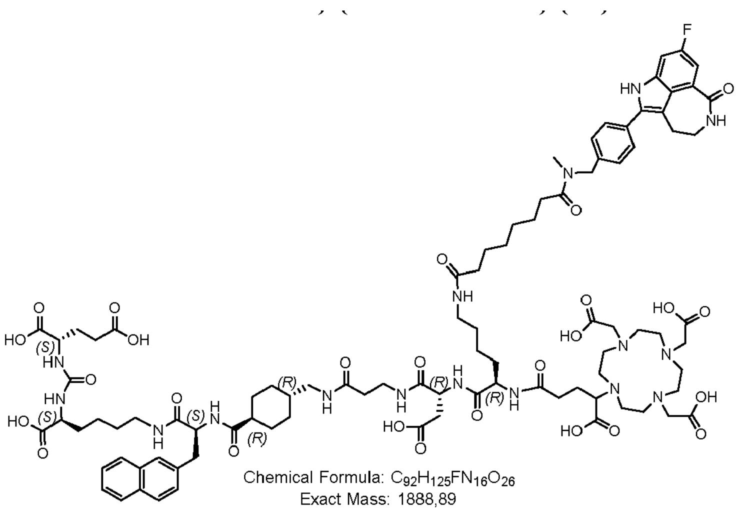

Chemical Formula: $C_{92}H_{125}FN_{16}O_{26}$
Exact Mass: 1888,89
Molecular Weight: 1890,10

In similarity to **12, 16** was synthesized through coupling of **4** (1.89 mg, 3.01 µmol, 1.5 eq.) instead of **2.** After semipreparative RP-HPLC purification (20-60%, buffer B, 15 min, buffer A and B without TFA addition), **16** (3.00 mg, 1.58 µmol, 78%) was acquired as a white solid. **RP-HPLC** (10-95%, 15 min): $t_R$ = 9.72 min. **MS** (ESI, positive): m/z = 1890.7 [M+2H$^+$], 946.0 [M/2].

**Ruc-SS-MMC-n-Asp-n-Asp-β-Ala-TXA-L-Nal-KuE (Ruc-SS-MMC-dd-βA-TXA-Nal-KuE) (PSMARuc-14) (17):**

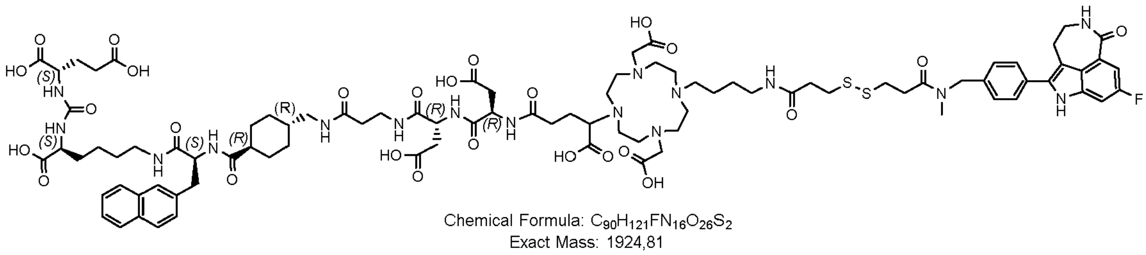

Chemical Formula: $C_{90}H_{121}FN_{16}O_{26}S_2$
Exact Mass: 1924,81
Molecular Weight: 1926,16

The synthesis of **17** was accomplished in similarity to **10,** using Fmoc-L-Nal-OH, Fmoc-TXA, Fmoc-β-Ala-OH, Fmoc-D-Asp(O$^t$Bu)-OH, Fmoc-D-Asp(O$^t$Bu)-OH, and MMC(O$^t$Bu)$_3$-NH(Boc), respectively. After semipreparative RP-HPLC (20-50%, buffer B, 15 min), conjugation of **2** (2.00 mg, 3.01 μmol, 1.5 eq.) in accordance with **GP-6,** and semi-preparative RP-HPLC purification (20-60%, buffer B, 15 min, buffer A and B without TFA addition), **17** (2.11 mg, 1.47 μmol, 1.5%) was acquired as beige powder.

**RP-HPLC** (10-95%, 15 min): $t_R$ = 9.65 min. **MS** (ESI, positive): m/z = 1925.6 [M+1H$^+$], 964.0 [M/2].

**Ruc-SS-MMC-D-Asp-D-Asp-D-Tyr-D-Phe-D-Phe-Ahx-Ahx-KuE (Ruc-SS-MMC-ddyff-Ahx-Ahx-KuE) (PSMARuc-15) (18):**

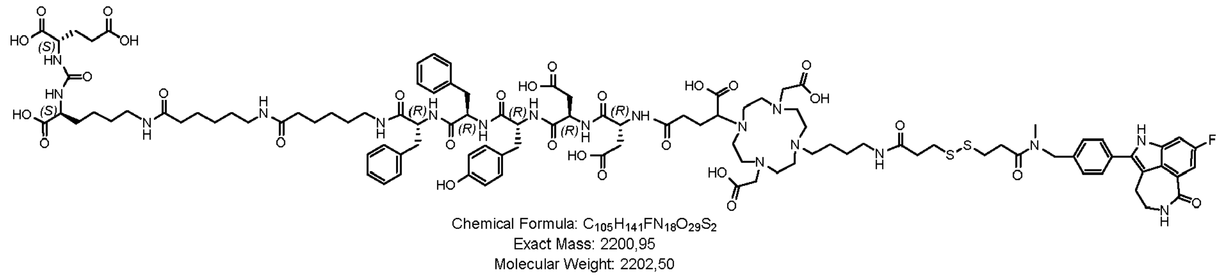

Chemical Formula: $C_{105}H_{141}FN_{18}O_{29}S_2$
Exact Mass: 2200,95
Molecular Weight: 2202,50

The synthesis was accomplished in similarity to **10,** using Fmoc-6-Ahx-OH, Fmoc-6-Ahx-OH, Fmoc-D-Phe-OH, Fmoc-D-Phe-OH, Fmoc-D-Tyr(O$^t$Bu)-OH, Fmoc-D-Asp(O$^t$Bu)-OH, Fmoc-D-Asp(O$^t$Bu)-OH, and MMC(O$^t$-Bu)$_3$-NH(Boc), respectively. The crude product was purified via semipreparative RP-HPLC (20-50%, buffer B, 15 min). After conjugation of **2** (2.00 mg, 3.01 μmol, 1.5 eq.) in accordance with **GP-6,** and semipreparative RP-HPLC purification (20-60%, buffer B, 15 min, buffer A and B without TFA addition), **18** (1.09 mg, 0.49 μmol, 24%) was acquired as beige powder.

**RP-HPLC** (10-95%, 15 min): $t_R$ = 9.69 min. **MS** (ESI, positive): m/z = 1102.2 [M/2].

**DOTAGA-D-Lys(D-Lys(Ruc-SS)$_2$)-D-Asp-D-Asp-D-Tyr-D-Phe-D-Phe-Ahx-KuE (DOTAGA- k(k(Ruc-SS)$_2$)-ddyff-Ahx-KuE) (PSMARuc-10Lys$_2$) (19):**

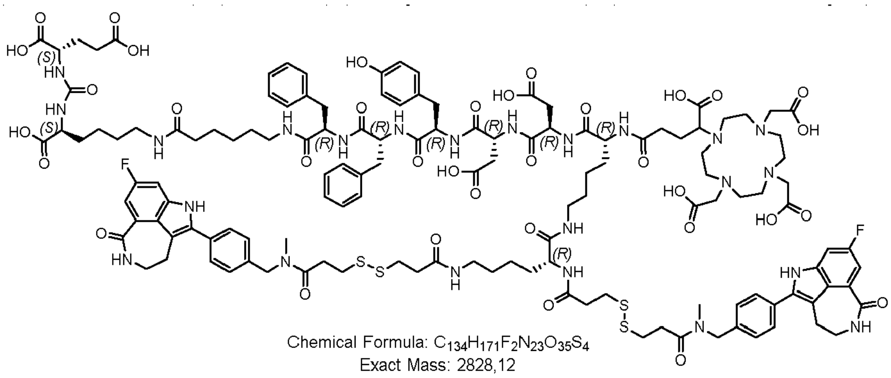

Chemical Formula: $C_{134}H_{171}F_2N_{23}O_{35}S_4$
Exact Mass: 2828,12
Molecular Weight: 2830,20

The synthesis of **19** was accomplished in similarity to **10,** using Fmoc-6-Ahx-OH, Fmoc-D-Phe-OH, Fmoc-D-Phe-OH, Fmoc-D-Tyr(O*t*Bu)-OH, Fmoc-D-Asp(O*t*Bu)-OH, Fmoc-D-Asp(O*t*Bu)-OH, Fmoc-D-Lys(Alloc)-OH, Boc-D-Lys(Boc)-OH, and DOTAGA-anhydride, respectively. The crude product was purified via semipreparative RP-HPLC (20-50%, buffer B, 15 min). Then, **2** (4.00 mg, 6.03 μmol, 3.0 eq.) was coupled according to **GP-6.** After semipreparative RP-HPLC purification (20-50%, buffer B, 15 min, buffer A and B without TFA addition), **19** (1.23 mg, 0.43 μmol, 21%) was acquired as beige solid.

**RP-HPLC** (10-95%, 15 min): $t_R$ = 10.76 min. **MS** (ESI, positive): m/z = 1416.2 [M/2].

**DOTAGA-D-Lys(D-Lys(Ruc-SS)$_2$)-D-Asp-D-Asp-D-Tyr-D-Phe-L-Nal-Ahx-KuE (DOTAGA-k(k(Ruc-SS)$_2$)-ddyf-Nal-Ahx-KuE) (PSMARuc-11Lys$_2$) (20):**

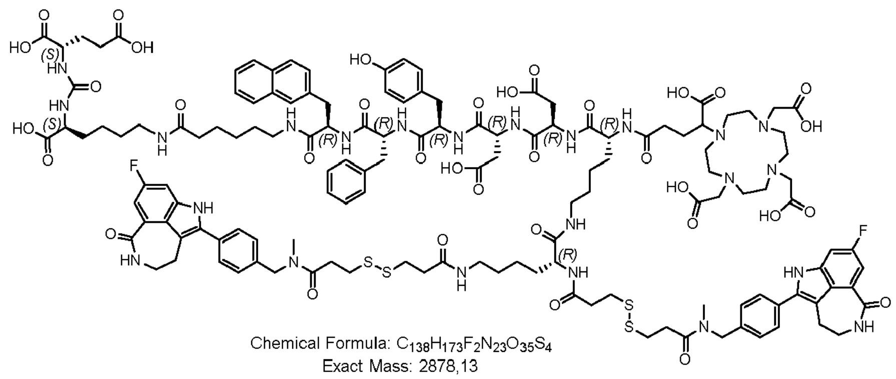

Chemical Formula: $C_{138}H_{173}F_2N_{23}O_{35}S_4$
Exact Mass: 2878,13
Molecular Weight: 2880,26

The synthesis of **20** was accomplished in similarity to **10,** using Fmoc-6-Ahx-OH, Fmoc-L-Nal-OH, Fmoc-D-Phe-OH, Fmoc-D-Tyr(O*t*Bu)-OH, Fmoc-D-Asp(O*t*Bu)-OH, Fmoc-D-Asp(O*t*Bu)-OH, Fmoc-D-Lys(Alloc)-OH, Boc-D-Lys(Boc)-OH, and DOTAGA-anhydride, respectively. The crude product was purified via semipreparative RP-HPLC (20-50%, buffer B, 15 min). After conjugation of **2** (2.00 mg, 3.01 μmol, 1.5 eq.) in accordance with **GP-6,** and semipreparative RP-HPLC purification (20-50%, buffer B, 15 min, buffer A and B without TFA addition), **20** (1.12 mg, 0.38 μmol, 19%) was acquired as beige solid.

**RP-HPLC** (10-95%, 15 min): $t_R$ = 10.82 min. **MS** (ESI, positive): m/z = 1441.0 [M/2].

**DOTAGA-D-Lys(D-Lys(Ruc-SS)$_2$)-D-Asp-D-Asp-D-Tyr-D-Phe-D-Phe-Ahx-Ahx-KuE (DOTAGA-k(k(Ruc-SS)$_2$)-ddyff-Ahx-Ahx-KuE) (PSMARuc-12Lys$_2$) (21):**

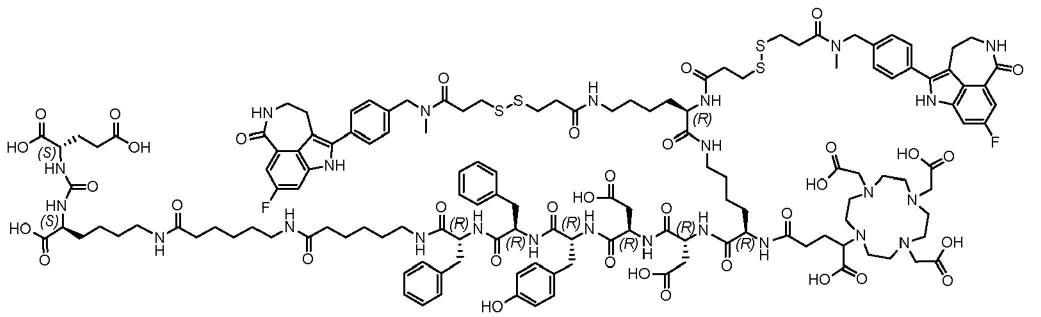

Chemical Formula: $C_{140}H_{182}F_2N_{24}O_{36}S_4$
Exact Mass: 2941,20
Molecular Weight: 2943,36

The synthesis was accomplished in accordance with **10,** using Fmoc-6-Ahx-OH, Fmoc-6-Ahx-OH, Fmoc-D-Phe-OH, Fmoc-D-Phe-OH, Fmoc-D-Tyr(O*t*Bu)-OH, Fmoc-D-Asp(O*t*Bu)-OH, Fmoc-D-Asp(O*t*Bu)-OH, Fmoc-D-Lys(Alloc)-OH, Boc-D-Lys(Boc)-OH, and DOTAGA-anhydride, respectively. After conjugation of **2** (2.00 mg, 3.01 μmol, 1.5 eq.) in accordance with **GP-6,** and semipreparative RP-HPLC purification (20-50%, buffer B, 15 min, buffer A and B

without TFA addition), **21** (1.67 mg, 0.56 μmol, 28%) was acquired as beige solid. **RP-HPLC** (10-95%, 15 min): $t_R$ = 11.01 min. **MS** (ESI, positive): m/z = 1472.5 [M/2], 982.0 [M/3].

**DOTAGA-D-Lys(D-Lys(Ruc-Sub)$_2$)-D-Asp-D-Asp-D-Tyr-D-Phe-L-Nal-Ahx-KuE (DOTAGA-k(k(Ruc-Sub)$_2$)-ddyf-Nal-Ahx-KuE) (PSMARucSub-11Lys$_2$) (22):**

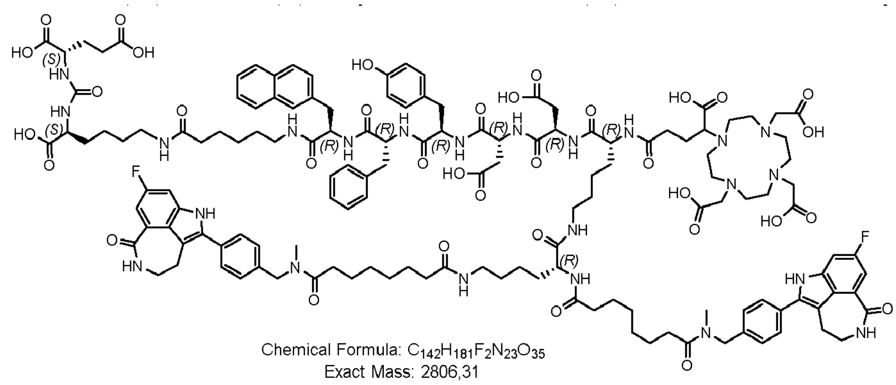

Chemical Formula: $C_{142}H_{181}F_2N_{23}O_{35}$
Exact Mass: 2806,31
Molecular Weight: 2808,13

As already described for **10,** the following amino acid sequence was coupled to 7 (100 mg resin, 96.9 μmol, 1.0 eq.): Fmoc-6-Ahx-OH, Fmoc-L-Nal-OH, Fmoc-D-Phe-OH, Fmoc-D-Tyr(O*t*Bu)-OH, Fmoc-D-Asp(O*t*Bu)-OH, Fmoc-D-Asp(O*t*Bu)-OH, Fmoc-D-Lys(Alloc)-OH, Boc-D-Lys(Boc)-OH, and DOTAGA-anhydride, respectively. After semipreparative RP-HPLC purification (20-60%, buffer B, 15 min), and coupling of **4** (3.78 mg, 6.03 μmol, 3.0 eq.) according to **GP-6, 22** (0.96 mg, 0.34 μmol, 17%) was obtained as beige powder after semipreparative RP-HPLC purification (20-60%, buffer B, 15 min, buffer A and B without TFA addition).

**RP-HPLC** (10-95%, 15 min): $t_R$ = 10.64 min. **MS** (ESI, positive): m/z = 1405.0 [M/2], 937.0 [M/3].

*Synthesis of EuE-based PSMA ligands*

**General synthesis procedure.** The peptide structures for the synthesis of the PSMA-targeting ligands were achieved according to **GP-2, GP-3, GP-4, GP-5,** and **GP-9,** respectively, via standard solid phase peptide synthesis. First, K(Dde)-(O*t*Bu)EuE(O*t*Bu)$_2$ **(9)** was Dde-deprotected according to **GP-9.** The coupling of further amino acid analogues or amino acids was achieved according to **GP-2** and **GP-3** following Fmoc-strategy. For DOTAGA coupling, the Fmoc-deprotected resin was treated with a solution of DOTAGA anhydride (4.0 eq.) and DIPEA (10 eq.) in DMSO (0.07 mM) for 48 h. Afterwards, the PSMA ligand was cleaved from the resin according to **GP-4** and globally deprotected according to **GP-5.** The crude ligands were purified via semipreparative RP-HPLC. After coupling of the Ruc-derivative according to **GP-6,** the final product was acquired after semipreparative RP-HPLC purification.

**DOTAGA-n-Lys(Ruc-SS)-n-Asp-n-Asp-β-Ala-TXA-L-Nal-n-Lys-EuE (DOTAGA-k(Ruc-Sub)-dd-βA-TXA-Nal-k-EuE) (PSMARuc-16) (23):**

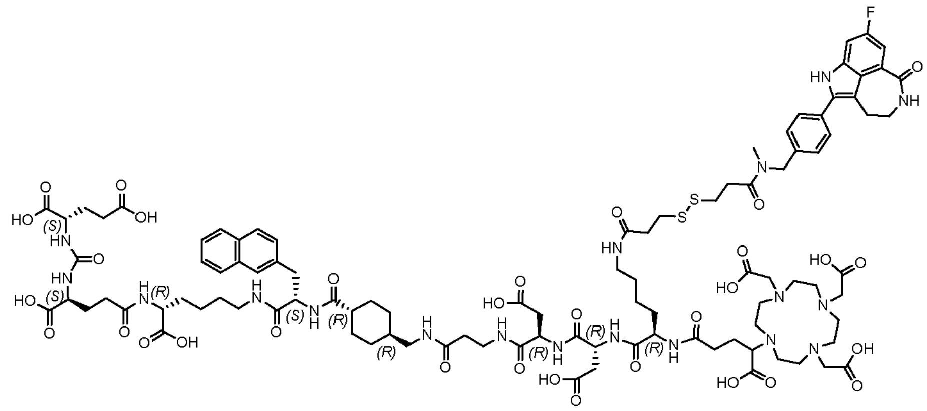

Chemical Formula: $C_{99}H_{133}FN_{18}O_{32}S_2$
Exact Mass: 2168,88
Molecular Weight: 2170,37

The peptide sequence of **23** was synthesized according to the general synthesis procedure of this chapter. The following amino acid analogues were coupled to **9** (100 mg resin, 103 μmol, 1.0 eq.): Fmoc-L-Nal-OH, Fmoc-TXA, Fmoc-β-Ala-OH, Fmoc-D-Asp(O*t*Bu)-OH, Fmoc-D-Asp(O*t*Bu)-OH, Fmoc-D-Lys(Boc)-OH, and DBCO-acid, respectively. **2** (2.00 mg, 3.01 μmol, 1.5 eq.) was then coupled according to **GP-6.** Via semipreparative RP-HPLC purification (20-60%, buffer B, 15 min, buffer A and B without TFA addition), **23** (3.74 mg, 1.72 μmol, 85%) was yielded as white solid.

**RP-HPLC** (10-95%, 15 min): $t_R$ = 9.63 min. **MS** (ESI, positive): m/z = 1086.1 [M/2].

**DOTAGA-D-Lys(Ruc-SS)-D-Asp-D-Asp-D-Tyr-D-Phe-D-Phe-Ahx-D-Lys-EuE (DOTAGA-k(Ruc-SS)-ddyff-Ahx-k-EuE) (PSMARuc-17) (24):**

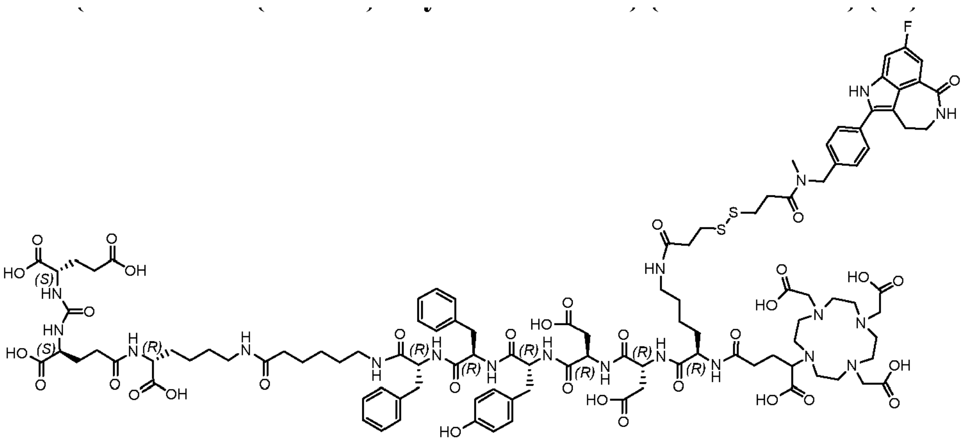

Chemical Formula: $C_{108}H_{142}FN_{19}O_{34}S_2$
Exact Mass: 2331,94
Molecular Weight: 2333,54

The synthesis of **24** was accomplished in similarity to **23,** using Fmoc-6-Ahx-OH, Fmoc-D-Phe-OH, Fmoc-D-Phe-OH, Fmoc-D-Tyr(O*t*Bu)-OH, Fmoc-D-Asp(O*t*Bu)-OH, Fmoc-D-Asp(O*t*Bu)-OH, Fmoc-D-Lys(Boc)-OH, and DOTAGA-anhydride, respectively. The crude product was purified via semipreparative RP-HPLC (5-30%, buffer B, 15 min). **2** (2.00 mg, 3.01 μmol, 1.5 eq.) was then coupled according to **GP-6.** After semipreparative RP-HPLC purification (20-60%, buffer B, 15 min, buffer A and B without TFA addition), **24** (1.48 mg, 0.63 μmol, 31%) was acquired as beige powder.

**RP-HPLC** (10-95%, 15 min): $t_R$ = 9.79 min. **MS** (ESI, positive): m/z = 1167.7 [M/2].

**Ruc-SS-MMC-n-Asp-n-Asp-β-Ala-TXA-L-Nal-n-Lys-EuE (Ruc-SS-MMC-dd-βA-TXA-Nal-k-EuE) (PSMARuc-18) (25):**

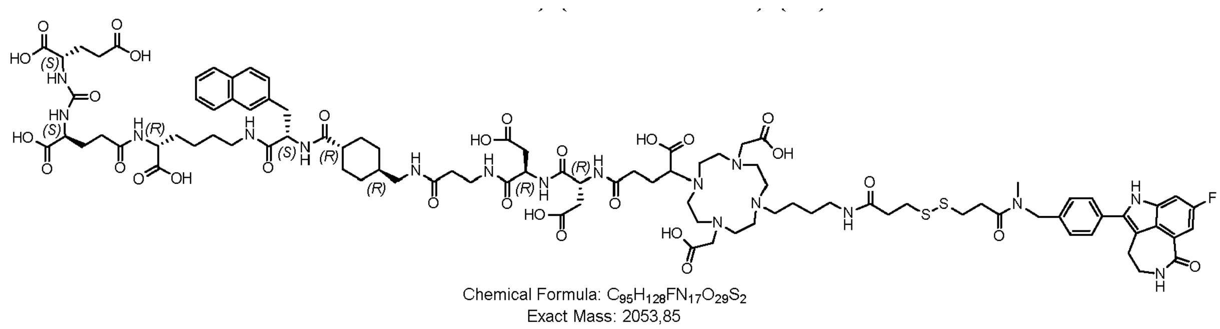

Chemical Formula: $C_{95}H_{128}FN_{17}O_{29}S_2$
Exact Mass: 2053,85
Molecular Weight: 2055,28

The synthesis was accomplished in similarity to **23,** using Fmoc-L-Nal-OH, Fmoc-TXA, Fmoc-β-Ala-OH, Fmoc-D-Asp(O*t*Bu)-OH, Fmoc-D-Asp(O*t*Bu)-OH, and MMC(O*t*Bu)$_3$-NH(Boc), respectively. The crude product was purified via semipreparative RP-HPLC (5-30%, buffer B, 15 min). Then, **2** (2.00 mg, 3.01 μmol, 1.5 eq.) was coupled according to **GP-6.** Via semipreparative RP-HPLC purification (20-50%, buffer B, 15 min, buffer A and B without TFA addition), **25** (0.91 mg, 0.39 μmol, 19%) was acquired as white powder.

**RP-HPLC** (10-95%, 15 min): $t_R$ = 9.50 min. **MS** (ESI, positive): m/z = 1028.6 [M/2].

**DOTAGA-D-Lys(D-Lys(Ruc-SS)$_2$)-D-Asp-D-Asp-P-Ala-TXA-L-Nal-D-Lys-EuE (DOTAGA- k(k(Ruc-SS)z)-dd-βA-TXA-Nal-k-EuE) (PSMARuc-16-Lys$_2$) (26):**

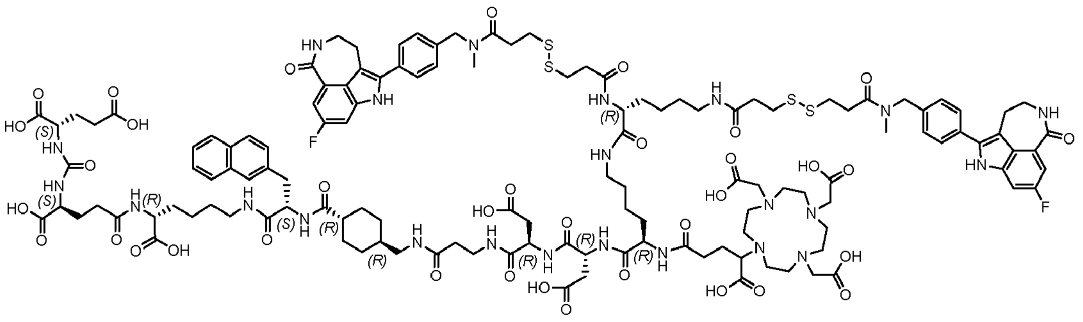

Chemical Formula: $C_{130}H_{169}F_2N_{23}O_{36}S_4$
Exact Mass: 2794,10
Molecular Weight: 2796,14

The synthesis of the title compound was accomplished in similarity to **23,** using Fmoc-L-Nal-OH, Fmoc-TXA, Fmoc-β-Ala-OH, Fmoc-D-Asp(O*t*Bu)-OH, Fmoc-D-Asp(O*t*Bu)-OH, Fmoc-D-Lys(Alloc)-OH, Boc-D-Lys(Boc)-OH, and DBCO-acid, respectively. The crude product was purified via semipreparative RP-HPLC (5-30%, buffer B, 15 min). According to **GP-6, 2** (4.00 mg, 6.03 μmol, 3.0 eq.) was coupled. After semipreparative RP-HPLC purification (20-50%, buffer B, 15 min, buffer A and B without TFA addition), **26** (1.89 mg, 0.67 μmol, 33%) was acquired as beige solid.

**RP-HPLC** (10-95%, 15 min): $t_R$ = 10.55 min. **MS** (ESI, positive): m/z = 1398.9 [M/2], 933.0 [M/3].

**DOTAGA-D-Lys(D-Lys(Ruc-SS)$_2$)-D-Asp-D-Asp-D-Tyr-D-Phe-D-Phe-Ahx-D-Lys-EuE (DOTAGA-k(k(Ruc-SS)$_2$)-ddyff-Ahx-k-EuE) (PSMARuc-17-Lys$_2$) (27):**

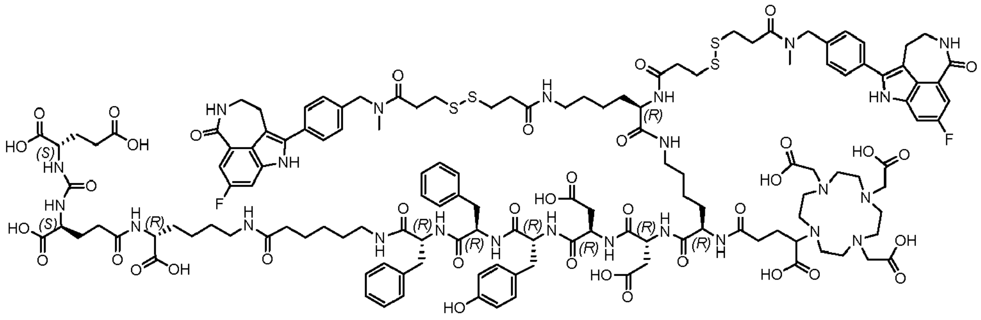

Chemical Formula: $C_{139}H_{178}F_2N_{24}O_{38}S_4$
Exact Mass: 2957,16
Molecular Weight: 2959,32

The synthesis of **24** was accomplished in similarity to **23,** using Fmoc-6-Ahx-OH, Fmoc-D-Phe-OH, Fmoc-D-Phe-OH, Fmoc-D-Tyr(O*t*Bu)-OH, Fmoc-D-Asp(O*t*Bu)-OH, Fmoc-D-Asp(O*t*Bu)-OH, Fmoc-D-Lys(Alloc)-OH, Boc-D-Lys(Boc)-OH, and DBCO-acid, respectively. The crude product was purified via semipreparative RP-HPLC (5-30%, buffer B, 15 min). According to **GP-6, 2** (4.00 mg, 6.03 µmol, 3.0 eq.) was coupled. After semi-preparative RP-HPLC purifi- cation (20-60%, buffer B, 15 min, buffer A and B without TFA addition), **27** (1.29 mg, 0.43 µmol, 21%) was acquired as beige solid.

**RP-HPLC** (10-95%, 15 min): $t_R$ = 10.64 min. **MS** (ESI, positive): m/z = 1480.5 [M/2].

### $^{nat}$Ga-labeled PSMA ligands

The labeling with $Ga(NO_3)_3$ was conducted as described under **GP-10.** Hereby, the resulting 1.0 mM solution was directly used in *in vitro* experiments.

### $^{68}$Ga-labeled PSMA ligands

The PSMA-targeting compounds were radiolabeled according to the literature (Weineisen et al., 2014; Notni et al., 2011) using a fully automated synthesis module (GallElut$^+$ from Scintomics GmbH, Germany). The following reaction steps were controlled by a computer program from Scintomics (Scintomics Control Center). In a first step, the $^{68}$Ge/$^{68}$Ga-generator with $SnO_2$ matrix (iTHEMBA labs, South Africa) was eluted with 0.1 M aq. HCl. Hereby, a fraction of 1.25 mL containing ca. 80% of the entire activity (ca. 300 MBq) was transferred to a mixture of the PSMA ligand (10 nmol) in aq. HEPES solution (2.7 M, pH 2; 75 µL). The radioactive mixture was heated to 95 °C, then passed through a previously with EtOH (5 mL) and $H_2O$ (10 mL) purged SPE cartridge (Waters SepPak® C8 light, sorbent amount 50 mg). Afterwards, the cartridge was purged with $H_2O$ (10 mL) and air (10 mL). Into a round bottom flask, the radiolabeled product was finally eluted with EtOH/$H_2O$ (1:1 (*v*/*v*)) and the cartridge was purged with PBS (1 mL; pH 7.4) and $H_2O$ (1 mL), respectively. After evaporation of EtOH under reduced pressure, the tracer was used without further purification in *in vitro* and *in vivo* experiments. Radiochemical purity was controlled using Radio-TLC (1.0 M sodium citrate buffer and 0.06 M NH4OAc/MeOH buffer (1/1 ; *v*/*v*)).

EXAMPLE 2 Ruc fluorescence correlates with Ruc concentration

To analyze Ruc fluorescence in cells, 500.000 cells/well were seeded into a 96 well plate and incubated with increasing amounts of Ruc for 30 min at 37°C. Untreated control and the 7-AAD single stain control were incubated with Medium (RPMI, 10%FCS, 1%PS) only. Afterwards cells were put on ice, washed twice with PBS, resuspended in FACS Buffer (5%FCS in PBS) and transferred into the flow cytometry tubes (obtained from VWR Catalog Number 734-0001). Prior to flow cytometry cells were incubated with eBioscience™ 7-AAD Viability Staining Solution (obtained from Thermo Fisher Scientific Catalog Number 00-6993-50) according to manufacturer's instructions. Flow Cytometry was performed using a FACS Canto II cytometer (BD Bioscience). Data was analyzed with the FlowJoTM software version 10.6.2 (BD Bioscience).

EXAMPLE 3 PSMA-Ruc conjugates show PSMA-specific internalization and high affinity to PSMA-expressing cells

To determine ligand internalization, LNCaP cells were seeded in a 96 V-bottom well plate at a density of 100,000 cells/well in 5% BSA (*w*/*v*)/RPMI medium (100 µL/well) and incubated for 30 min at 37 °C. Afterwards, solutions (25 µL/well) of either 5% BSA (w/v)/RPMI medium or of 2-PMPA (final concentration: 10 µM) in 5% BSA (w/v)/RPMI medium

for blockade were added. Then, a solution of the $^{68}Ga$-labeled PSMA-ligand (25 μL/well; 0.4 nM final concentration; $A_M$ = 26-30 MBq/nmol) was added to all wells, and the cells were incubated for 5, 15, 30, and 60 min, respectively, at 37 °C. Hereby, each time point was performed in triplicates and each experiment was repeated three times. The internalization process was terminated by placing the plate for 5 min on ice. The free radioligand was obtained by centrifugation of the plate at 600 g for 3 min and removal of the supernatant. Each well was then washed with ice-cold PBS and the fractions of the first two steps were combined. For removal of cell-bound PSMA-ligand, the cells were treated in a next step with ice-cold 2-PMPA-solution (10 μM in PBS; 150 μL/well; 15 min on ice), centrifuged (600 g, 3 min), and washed with ice-cold PBS (150 μL/well). The fractions were again combined. For the analysis of internalized radioligand, the fractions of a 1.0 N NaOH (150 μL/well; 10 min on ice) treatment as well as a concomitant washing step with 1.0 N NaOH (150 μL/well) were pooled. Free, surface bound, and internalized activity, respectively, was measured in a γ-counter.

For $IC_{50}$ value determination, LNCaP cells were seeded in a 96 V-bottom well plate at a density of 100,000 cells/well in 1% BSA (*w*/*v*)/HBSS (Hank's balanced salt solution, ThermoFisher Scientific; 100 μL/well) and incubated for 15 min on ice. In the meantime, dilution series of the compounds were prepared in 1% BSA (*w*/*v*)/HBSS in a separate 96 U-bottom deep well plate ranging from 12 μM to 153.6 pM in 1:5 dilution steps. Next, solutions (25 μL/well) of either 1% BSA (*w*/*v*)/HBSS (control) or of the respective dilution series were transferred to the LNCaP cells containing plate. Hereby, each concentration was pipetted in triplicates and each experiment was repeated three times. Subsequently, [$^{68}Ga$]-PSMA-617 (25 μL/well; 0.4 nM final concentration; $A_M$ = 26-30 MBq/nmol) in 1% BSA (*w*/*v*)/HBSS was added to all wells and the plate was incubated for 60 min on ice. Afterwards, the experiment was terminated by centrifugation at 600 g for 3 min and removal of the supernatant. The cells were then washed with ice-cold HBSS (150 μL/well), centrifuged (600 g, 3 min), and the supernatant as well as the washing fraction were combined, representing the amount of free, unbound radioligand. Bound radioligand was obtained by lysis of the cells with 1.0 $_N$ NaOH (150 μL/well; 10 min on ice). Afterwards, the plate was washed a second time with 1.0 $_N$ NaOH (150 μL/well). Hereby, the washing step was combined with the NaOH fraction. The quantification of bound and free radioligand was finally accomplished via measuring the samples in a γ-counter.

Lipophilicity of the conjugates was determine after radiolabeling with $^{68}Ga$. The PSMA ligand (0.80 MBq) was dissolved in PBS (500 μL/pH 7.4) in a reaction vial (1.5 mL; n = 10). Afterwards, n-octanol was added (500 μL) and the mixture was rigorously vortexed for 3 min. Subsequent centrifugation (Biofuge 15, Heraus Sepatech, Osterode, Germany) at 13,000 rpm for 5 min led to a phase separation. After measuring 100 μL of each phase in a γ-counter, the distribution coefficient ($\log D_{(O/PBS)}$) was calculated with Microsoft Excel.

EXAMPLE 4 PSMA-Ruc conjugates show PSMA-dependent uptake of Ruc Microscopy + flow

For microscopic analysis, black clear-bottom 96 well cell culture plates with cover-glass bottom (μ plate 96 well black; ibidi) were coated with poly-L-lysine (0.01% (*w*/*v*) in sterile water). Cells were seeded at a density of 20-40,000 cells/well and cultured for 24 h at 37 °C. Subsequently, the PSMA ligands (1 μM; 100 μL/well) were directly added with or without blocking and ligand treatment was accomplished for 5, 60 or 180 min at 37 °C, while in case of GSH preincubation, GSH-OEt (10 mM; 100 μL/well) was applied for 1 h at 37 °C prior to ligand treatment. For the latter case, the PSMA ligands (1 μM, 100 μL/well) were added after removal of the GSH containing medium and the incubation time was 60 min at 37 °C. Afterwards, cells were washed with PBS (2×) and fixed with 4% (*w*/*v*) PFA in PBS for 8 min at RT (pH 7.4, 100 μL/well). CellMask™ Green Plasma Membrane Stain was used to stain the cell membrane, whereas for cell nuclei DRAQ5™ was used. Before imaging, cells were washed with PBS (3×) and mounted in PBS (150 μL/well). Finally, cells were imaged using an EVOS M7000 microscope (Thermo Fisher Scientific).

For flow cytometry analysis of Rue-fluorescence in cells after incubation with PSMA-Ruc conjugates, 500.000 cells/well were seeded into a 96 well plate and incubated with a PSMA-Ruc conjugate for 30 min at 37°C. Untreated controls and the 7-AAD single stain control were incubated with Medium (RPMI, 10%FCS, 1%PS) only. Afterwards cells were put on ice, washed twice with PBS, resuspended in FACS Buffer (5%FCS in PBS) and transferred into the flow cytometry tubes (obtained from VWR Catalog Number 734-0001). Prior to flow cytometry cells were incubated with eBioscience™ 7-AAD Viability Staining Solution (obtained from Thermo Fisher Scientific Catalog Number 00-6993-50) according to manufacturer's instructions. Flow Cytometry was performed using a FACS Canto II cytometer (BD Bioscience). Data was analyzed with the FlowJoTM software version 10.6.2 (BD Bioscience).

EXAMPLE 5 Serum stability and GSH-dependent Ruc release of PSMA-Ruc conjugates

For serum stability analysis, 13.5 μL human serum were mixed with 1.5 μL of an aqueous solution of the test compound (200 μM final concentration) and incubated at 37 °C for 0.5, 1, 2, and 24 h, respectively. Afterwards, 25.0 μL MeCN were added, the suspension was centrifuged (14,000 rpm, 30 min, 4 °C), the supernatant was recovered, and finally analyzed by HPLC-ESI-MS using a linear elution gradient from 10 to 95% (v/v) MeCN/0.1% (v/v) TFA in water/0.1%

(v/v) TFA for 15 min.

For the analysis of GSH cleavage capability, we added GSH-OEt in PBS (10 mM, 15 μL) to the PSMA ligands (160 μM, 0.5 μL) and incubated for 5, 15, and 30 min, respectively, at 37 °C in reaction vials (1.5 mL). Afterwards, the samples were diluted with MeCN (30 μL) and analyzed by HPLC-ESI-MS using a linear elution gradient from 10 to 95% (v/v) MeCN/0.1% (v/v) TFA in water/0.1% (v/v) TFA for 15 min.

EXAMPLE 6 PSMA-Ruc conjugates induce selective cytotoxicity *in vitro*

Black, clear-bottom 96 well culture plates were coated with poly-L-lysine. Afterwards, the plates were washed with PBS (1 × 100 μL), cells were seeded (2000 cells/well for PC3, 10000 cells/well for LNCaP), and cultured for 24 h (PC3) or 48 h (LNCaP) at 37 °C. The culture medium was removed, and new culture medium containing 10 mM GSH-OEt was added and incubated for 1 h at 37 °C. After removal of the GSH-OEt medium, new culture medium containing the PSMA-Ruc conjugates was added. After 72 h of incubation at 37 °C, cells were treated with Alamar Blue for visualization of cell viability. Results are shown in Figure 6. The graph displays the mean and standard deviation of biological replicates. Each data points represents one biological replicate.

EXAMPLE 7 PSMA-Ruc in combination with $^{177}$Lu-PSMA treatment *in vitro*

Black, clear-bottom 96 well culture plates were coated with poly-L-lysine. Afterwards, the plates were washed with PBS (1 × 100 μL), cells were seeded (2000 cells/well for PC3, 10000 cells/well for LNCaP), and cultured for 24 h to 48 h at 37 °C. The culture medium was removed and fresh medium containing 10 mM GSH-OEt (for PSMA-Ruc-conjugate treatment; treatment for 1 h at 37 °C), 0.1875 MBq RLT ($^{177}$Lu-PSMA-I&T; treatment for 72 h) or rucaparib (0.1 - 1 μM) or both was added. After a total incubation time of 72 h, the cell viability was tested by use of Alamar Blue. The graphs display the mean and standard deviation of at least 3 biological replicates.
Results are shown in Figures 7 to 9.

EXAMPLE 8 PSMA-selective *in vivo* imaging using a PSMA-Ruc conjugate

**Radiolabeling.** Generator-produced $^{68}GaCl_3$ ($t_{1/2}$ = 68 min) was purchased from the MD Anderson radiopharmacy (Houston, TX) and converted to $^{68}$Ga as previously described (Zhernosekov ***et al.,*** 2007). PSMA-617, PSMA-Ruc-9, PSMA-Ruc-16 and PSMA-Ruc-18 (15 nmol each) were dissolved in 0.2 M sodium acetate buffer (pH 4.2; 100 μL), mixed with $^{68}$Ga (155-296 MBq; 100-150 μL), radiolabeled for 15 min at 95°C and purified using an activated Sep-Pak Light C18 (Waters) cartridge.

**In vivo experiment.** Athymic nude mice were subcutaneously injected with 4×10$^{6}$ PC3-PIP (PSMA+) and 3×10$^{6}$ PC3-Flu (PSMA-) cells to produce a bilateral tumor model.

Conjugate 11: Mice were injected with 7.4 MBq of$^{68}$Ga-PSMA-Ruc-11 or $^{68}$Ga-PSMA-617 (positive control) via the tail vein and PET/CT imaging was performed 1 hour post-injection. At the completion of the imaging study, mice were euthanized and organs were resected, weight, and analyzed by gamma counting to determine tracer biodistribution. Data are ported as percentage of injected activity per gram of tissue (%IA/g).

Results are shown in Figure 9.

Conjugates 9, 16 and 18: 10 days after xenografting when tumor size reached ~5- to 10-mm maximum diameter, mice (*n*=5/conjugate) were injected with 2 nmol of $^{68}$Ga-PSMA-Ruc-9 (0.5±0.1 MBq), $^{68}$Ga-PSMA-Ruc-16 (7.4±0.7 MBq), $^{68}$Ga-PSMA-Ruc-18 (15.2±5.1 MBq) or $^{68}$Ga-PSMA-617 (8.0±0.6 MBq; positive control) via the tail vein and PET/CT imaging (*n*=2/conjugate) was performed 1 hour post-injection, except for $^{68}$Ga-PSMA-Ruc-9 due to low specific activity. At the completion of the imaging study, mice were euthanized and organs were resected, weighted, and analyzed by gamma counting to determine tracer biodistribution. Biodistribution data are reported as percentage of injected dose per gram of tissue (%ID/g). Results are shown in Figure 10.

The features disclosed in the foregoing description, in the claims and/or in the accompanying drawings may, both separately and in any combination thereof, be material for realizing the invention in diverse forms thereof.

REFERENCES

Abida, W., Patnaik, A., Campbell, D., Shapiro, J., Bryce, A.H., McDermott, R., Sautois, B., Vogelzang, N.J., Bambury, R.M., Voog, E., Zhang, J., Piulats, J.M., Ryan, C.J., Merseburger, A.S., Daugaard, G., Heidenreich, A., Fizazi, K., Higano, C.S., Krieger, L.E., Sternberg, C.N., Watkins, S.P., Despain, D., Simmons, A.D., Loehr, A., Dowson, M., Golsorkhi, T., Chowdhury, S., and investigators, T. "Rucaparib in Men With Metastatic Castration-Resistant Prostate

Cancer Harboring a BRCA1 or BRCA2 Gene Alteration." J Clin Oncol 38, no. 32 (2020): 3763-72.
Antonarakis, E.S., Gomella, L.G., and Petrylak, D.P. "When and How to Use PARP Inhibitors in Prostate Cancer: A Systematic Review of the Literature with an Update on OnGoing Trials." Eur Urol Oncol 3, no. 5 (2020): 594-611.
Banerjee, S.R., Pullambhatla, M., Foss, C.A., Nimmagadda, S., Ferdani, R., Anderson, C.J., Mease, R.C., and Pomper, M.G. "64Cu-labeled inhibitors of prostate-specific membrane antigen for PET imaging of prostate cancer." J Med Chem 57, no. 6 (2014): 2657-69.
Benesova, M., Schafer, M., Bauder-Wust, U., Afshar-Oromieh, A., Kratochwil, C., Mier, W., Haberkorn, U., Kopka, K., and Eder, M. "Preclinical Evaluation of a Tailor-Made DOTA-Conjugated PSMA Inhibitor with Optimized Linker Moiety for Imaging and Endoradiotherapy of Prostate Cancer." J Nucl Med 56, no. 6 (2015): 914-20.
Bray, F., Ferlay, J., Soerjomataram, I., Siegel, R.L., Torre, L.A., and Jemal, A. "Global cancer statistics 2018: GLOBOCAN estimates of incidence and mortality worldwide for 36 cancers in 185 countries." CA Cancer J Clin 68, no. 6 (2018): 394-424.
Carpino, L. A.; Han, G. Y., 9-Fluorenylmethoxycarbonyl amino-protecting group. The Journal of Organic Chemistry 1972, 37 (22), 3404-3409.
Chen, Y., Pullambhatla, M., Foss, C.A., Byun, Y., Nimmagadda, S., Senthamizhchelvan, S., Sgouros, G., Mease, R.C., and Pomper, M.G. "2-(3-{1-Carboxy-5-[(6-[18F]fluoro-pyridine-3-carbonyl)-amino]-pentyl}-ureido)-pentanedioic acid, [18F]DCFPyL, a PSMA-based PET imaging agent for prostate cancer." Clinical cancer research : an official journal of the American Association for Cancer Research 17, no. 24 (2011): 7645-53.
Deeks, E.D. "Olaparib: first global approval." Drugs 75, no. 2 (2015): 231-40.
Eiber, M., Maurer, T., Souvatzoglou, M., Beer, A.J., Ruffani, A., Haller, B., Graner, F.P., Kubler, H., Haberkorn, U., Eisenhut, M., Wester, H.J., Gschwend, J.E., and Schwaiger, M. "Evaluation of Hybrid (6)(8)Ga-PSMA Ligand PET/CT in 248 Patients with Biochemical Recurrence After Radical Prostatectomy." J Nucl Med 56, no. 5 (2015): 668-74.
Fendler, W.P., Schmidt, D.F., Wenter, V., Thierfelder, K.M., Zach, C., Stief, C., Bartenstein, P., Kirchner, T., Gildehaus, F.J., Gratzke, C., and Faber, C. "68Ga-PSMA PET/CT Detects the Location and Extent of Primary Prostate Cancer." J Nucl Med 57, no. 11 (2016): 1720-25.
Fendler, W.P., Rahbar, K., Herrmann, K., Kratochwil, C., and Eiber, M. "(177)Lu-PSMA Radioligand Therapy for Prostate Cancer." J Nucl Med 58, no. 8 (2017): 1196-200.
Fendler, W.P., Calais, J., Eiber, M., Flavell, R.R., Mishoe, A., Feng, F.Y., Nguyen, H.G., Reiter, R.E., Rettig, M.B., Okamoto, S., Emmett, L., Zacho, H.D., Ilhan, H., Wetter, A., Rischpler, C., Schoder, H., Burger, I.A., Gartmann, J., Smith, R., Small, E.J., Slavik, R., Carroll, P.R., Herrmann, K., Czernin, J., and Hope, T.A. "Assessment of 68Ga-PSMA-11 PET Accuracy in Localizing Recurrent Prostate Cancer: A Prospective Single-Arm Clinical Trial." JAMA Oncol (2019).
Ghosh, A., and Heston, W.D. "Tumor target prostate specific membrane antigen (PSMA) and its regulation in prostate cancer." J Cell Biochem 91, no. 3 (2004): 528-39.
Gillessen, S., Omlin, A., Attard, G., de Bono, J.S., Efstathiou, E., Fizazi, K., Halabi, S., Nelson, P.S., Sartor, O., Smith, M.R., Soule, H.R., Akaza, H., Beer, T.M., Beltran, H., Chinnaiyan, A.M., Daugaard, G., Davis, I.D., De Santis, M., Drake, C.G., Eeles, R.A., Fanti, S., Gleave, M.E., Heidenreich, A., Hussain, M., James, N.D., Lecouvet, F.E., Logothetis, C.J., Mastris, K., Nilsson, S., Oh, W.K., Olmos, D., Padhani, A.R., Parker, C., Rubin, M.A., Schalken, J.A., Scher, H.I., Sella, A., Shore, N.D., Small, E.J., Sternberg, C.N., Suzuki, H., Sweeney, C.J., Tannock, I.F., and Tombal, B. "Management of patients with advanced prostate cancer: recommendations of the St Gallen Advanced Prostate Cancer Consensus Conference (APCCC) 2015." Ann Oncol 26, no. 8 (2015): 1589-604.
Harmatys, K.M., Overchuk, M., Chen, J., Ding, L., Chen, Y., Pomper, M.G., and Zheng, G. "Tuning Pharmacokinetics to Improve Tumor Accumulation of a Prostate-Specific Membrane Antigen-Targeted Phototheranostic Agent." Bioconjug Chem 29, no. 11 (2018): 3746-56.
Heinzel, A., Boghos, D., Mottaghy, F.M., Gaertner, F., Essler, M., von Mallek, D., and Ahmadzadehfar, H. "(68)Ga-PSMA PET/CT for monitoring response to (177)Lu-PSMA-617 radioligand therapy in patients with metastatic castration-resistant prostate cancer." Eur J Nucl Med Mol Imaging 46, no. 5 (2019): 1054-62.
Hensbergen, A.W., Buckle, T., van Willigen, D.M., Schottelius, M., Welling, M.M., van der Wijk, F.A., Maurer, T., van der Poel, H.G., van der Pluijm, G., van Weerden, W.M., Wester, H.-J., and van Leeuwen, F.W.B. "Hybrid Tracers Based on Cyanine Backbones Targeting Prostate-Specific Membrane Antigen: Tuning Pharmacokinetic Properties and Exploring Dye-Protein Interaction." Journal of Nuclear Medicine 61, no. 2 (2020): 234-41.
Hoffmann, M.A., Wieler, H.J., Baues, C., Kuntz, N.J., Richardsen, I., and Schreckenberger, M. "The Impact of 68Ga-PSMA PET/CT and PET/MRI on the Management of Prostate Cancer." Urology (2019).
Hoy, S.M. "Talazoparib: First Global Approval." Drugs 78, no. 18 (2018): 1939-46.
Kelly, J.M., Amor-Coarasa, A., Nikolopoulou, A., Kim, D., Williams, C., Vallabhajosula, S., and Babich, J.W. "Assessment of PSMA targeting ligands bearing novel chelates with application to theranostics: Stability and complexation kinetics of 68Ga3+, 111In3+, 177Lu3+ and 225Ac3+." Nuclear Medicine and Biology 55 (2017): 38-46.
Kossatz, S., Carney, B., Farley, C., Weber, W.A., Drain, C.M., and Reiner, T. "Direct Imaging of Drug Distribution

and Target Engagement of the PARP Inhibitor Rucaparib." J Nucl Med 59, no. 8 (2018): 1316-20.

Merrifield, R. B., Solid Phase Peptide Synthesis. I. The Synthesis of a Tetrapeptide. Journal of the American Chemical Society 1963, 85 (14), 2149-2154.

Nizialek, E., and Antonarakis, E.S. "PARP Inhibitors in Metastatic Prostate Cancer: Evidence to Date." Cancer Manag Res 12 (2020): 8105-14.

Notni, J.; Šimeček, J.; Hermann, P.; Wester, H.-J., TRAP, a Powerful and Versatile Framework for Gallium-68 Radiopharmaceuticals. Chemistry - A European Journal 2011, 17 (52), 14718-14722.

Pezaro, C. "PARP inhibitor combinations in prostate cancer." Ther Adv Med Oncol 12 (2020): 1758835919897537.

Petrov, S.A., Zyk, N.Y., Machulkin, A.E., Beloglazkina, E.K., and Majouga, A.G. "PSMA-targeted low-molecular double conjugates for diagnostics and therapy." Eur J Med Chem 225 (2021): 113752.

Rajan, A., Carter, C.A., Kelly, R.J., Gutierrez, M., Kummar, S., Szabo, E., Yancey, M.A., Ji, J., Mannargudi, B., Woo, S., Spencer, S., Figg, W.D., and Giaccone, G. "A phase I combination study of olaparib with cisplatin and gemcitabine in adults with solid tumors." Clin Cancer Res 18, no. 8 (2012): 2344-51.

Ray Banerjee, S., Chen, Z., Pullambhatla, M., Lisok, A., Chen, J., Mease, R.C., and Pomper, M.G. "Preclinical Comparative Study of 68Ga-Labeled DOTA, NOTA, and HBED-CC Chelated Radiotracers for Targeting PSMA." Bioconjug Chem 27, no. 6 (2016): 1447-55.

Robu, S., Schmidt, A., Eiber, M., Schottelius, M., Giinther, T., Hooshyar Yousefi, B., Schwaiger, M., and Wester, H.-J. "Synthesis and preclinical evaluation of novel 18F-labeled Glu-urea-Glu-based PSMA inhibitors for prostate cancer imaging: a comparison with 18F-DCFPyl and 18F-PSMA-1007." EJNMMI research 8, no. 1 (2018): 30.

Sartor, O., de Bono, J., Chi, K.N., Fizazi, K., Herrmann, K., Rahbar, K., Tagawa, S.T., Nordquist, L.T., Vaishampayan, N., El-Haddad, G., Park, C.H., Beer, T.M., Armour, A., Perez-Contreras, W.J., DeSilvio, M., Kpamegan, E., Gericke, G., Messmann, R.A., Morris, M.J., Krause, B.J., and Investigators, V. "Lutetium-177-PSMA-617 for Metastatic Castration-Resistant Prostate Cancer." N Engl J Med (2021).

Syed, Y.Y. "Rucaparib: First Global Approval." Drugs 77, no. 5 (2017): 585-92.

Takebe, N., Quinn, M., Gupta, G., and Chen, A.P. "PARP Inhibition to Enhance Response to Chemotherapy." In Targeting Cell Survival Pathways to Enhance Response to Chemotherapy, 231-57: Elsevier, 2019.

Umbricht, C.A., Benešová, M., Schmid, R.M., Türler, A., Schibli, R., van der Meulen, N.P., and Müller, C. "44Sc-PSMA-617 for radiotheragnostics in tandem with 177Lu-PSMA-617-preclinical investigations in comparison with 68Ga-PSMA-11 and 68Ga-PSMA-617." EJNMMI research 7, no. 1 (2017): 9.

Umbricht, C.A., Benešová, M., Schibli, R., and Müller, C. "Preclinical Development of Novel PSMA-Targeting Radioligands: Modulation of Albumin-Binding Properties To Improve Prostate Cancer Therapy." Molecular Pharmaceutics 15, no. 6 (2018): 2297-306.

Virtanen, V., Paunu, K., Ahlskog, J.K., Varnai, R., Sipeky, C., and Sundvall, M. "PARP Inhibitors in Prostate Cancer-The Preclinical Rationale and Current Clinical Development." Genes (Basel) 10, no. 8 (2019).

Weineisen, M.; Simecek, J.; Schottelius, M.; Schwaiger, M.; Wester, H. J., Synthesis and preclinical evaluation of DOTAGA-conjugated PSMA ligands for functional imaging and endoradiotherapy of prostate cancer. Ejnmmi Res 2014, 4 (1), 63.

Wilson, T. C., Xavier, M. A., Knight, J., Verhoog, S., Torres, J. B., Mosley, M., Hopkins, S. L., Wallington, S., Allen, P. D., Kersemans, V., Hueting, R., Smart, S., Gouverneur, V., & Cornelissen, B. (2019). PET imaging of PARP expression using 18F-olaparib. Journal of Nuclear Medicine, 60(4), 504-510.

Yadav, M.P., Ballal, S., Sahoo, R.K., Dwivedi, S.N., and Bal, C. "Radioligand Therapy With (177)Lu-PSMA for Metastatic Castration-Resistant Prostate Cancer: A Systematic Review and Meta-Analysis." AJR Am J Roentgenol (2019): 1-11.

Yao, V., and Bacich, D.J. "Prostate specific membrane antigen (PSMA) expression gives prostate cancer cells a growth advantage in a physiologically relevant folate environment in vitro." The Prostate 66, no. 8 (2006): 867-75.

Zhernosekov, Filosofov, D. V., Baum, R. P., Aschoff, P., Bihl, H., Razbash, A. A., Jahn, M., Jennewein, M., & Rosch, F. (2007). Processing of Generator-Produced 68Ga for Medical Application. The Journal of Nuclear Medicine (1978), 48(10), 1741-1748. https://doi. org/10.2967/j numed.107.040378

## Claims

**1.** A conjugate compound comprising

a **disease-targeting moiety (T),**
at least one **PARP inhibitor moiety (P),**
a **cleavable linker (X)** connecting the PARP inhibitor moiety (P) to the conjugate compound,
and optionally, a **chelating agent (C),**

or a salt, solvate or tautomer thereof.

2. The conjugate compound of claim 1, wherein the **disease-targeting moiety (T)** comprises a peptide or small molecule, which internalizes upon binding to a cell surface biomarker,
wherein said peptide or small molecule preferably targets the prostate-specific membrane antigen (PSMA), somatostatin receptor (SSTR subtypes 2, 3, and 5), gastrin-releasing peptide receptor (GRPR), C-X-C motif chemokine receptor 4 (CXCR4), αvβ6-integrin, αvβ8-integrin, α5β1-integrin, αvβ1-integrin, folate receptor (FR), neurotensin receptor 1 (NTSR1), and/or melanocortin receptor 1 (MC1R).

3. The conjugate compound of claim 1 or 2, wherein the disease-targeting moiety (T) comprises monomers or multimers, and/or comprises further components, such as PEG linker(s), albumin-binding domain(s), further amino acid(s), and/or peptidomimetics.

4. The conjugate compound of any one of claims 1 to 3, wherein the disease-targeting moiety (T) targets PSMA and preferably comprises Glu-urea-Glu (EuE), Glu-urea-Lys (EuK) and/or other urea-based peptides or peptidomimetics, such as Lys-urea-aminoadipic acid (Lys-urea-Aad).

5. The conjugate compound of any one of claims 1 to 4, wherein the **PARP inhibitor moiety (P)** comprises or consists of rucaparib, olaparib, talazoparib, niraparib or analogs thereof,
and/or comprising two or more PARP inhibitor moieties (P), such as 2 or 3.

6. The conjugate compound of any one of the preceding claims, wherein the **cleavable linker (X)** comprises a substrate for enzyme cleavage or redox reaction,
such as a substrate for prostate specific antigen (PSA), caspases or cathepsins or for the antioxidant glutathione.

7. The conjugate compound of any one of the preceding claims, wherein the **chelating agent (C)** is a macrocyclic chelator or a non-cyclic chelating agent,

wherein the chelating agent (C) is preferably selected from the group consisting of

1,4,7,10-tetraazacyclododecane (cyclen),
2-(7-(4-aminobutyl)-4,10-bis(carboxymethyl)-1,4,7,10-tetraazacyclododecan-1-yl)pentanedioic acid (MMC),
2,2',2"-(10-(2,6-dioxotetrahydro-2H-pyran-3-yl)-1,4,7,10-tetraazacyclododecane-1,4,7-triyl)triacetic acid (DOTAGA),
2,2',2",2‴-(1,4,7,10-tetraazacyclododecane-1,4,7,10-tetrayl)tetraacetic acid (DOTA),
2,2',2"-(1,4,7-triazacyclononane-1,4,7-triyl)triacetic acid (NOTA),
2-[1,4,7-triazacyclononan-1-yl-4,7-bis(tBu-ester)]-1,5-pentanedioic acid (NODAGA),
1,4,7,10-tetraazacyclo- dodecane-1,4,7,10-tetrakis[methylene(2-carboxyethyl)phosphinic acid] (DOTPI),
N,N'-Di(2-hydroxybenzyl)ethylenediamine-N,N'-diacetic acid monohydrochloride (HBED),
N,N'-bis-[2-hydroxy-5-(carboxyethyl)benzyl]ethylenediamine-N,N'-diacetic acid (HBED-CC),
2,2',2",2‴-(1,4,7,10-tetraazacyclododecane-1,4,7,10-tetrayl)tetraacetamide (DOTAM),
3,3',3"-(((1,4,7-triazonane-1,4,7-triyl)tris(methylene))tris(hydroxyphosphoryl)) tripropanoic acid (TRAP),
3-(((4,7-bis((hydroxy(hydroxymethyl)phosphoryl)methyl)-1,4,7-triazonan-1-yl)methyl)(hydroxy)phosphoryl)propanoic acid (NOPO),
3,6,9,15-Tetraazabicyclo[9.3.1]pentadeca-1(15),11,13-triene-3,6,9-triacetic acid (PCTA), and
ethylenediaminetetraacetic acid (EDTA), and
derivatives thereof,

and/or wherein the chelating agent (C) comprises or contains a **radionuclide,**
wherein the radionuclide is preferably selected from $^{44}$Sc, $^{45}$Ti, $^{47}$Sc, $^{59}$Fe, $^{60}$Cu, $^{61}$Cu, $^{62}$Cu, $^{64}$Cu, $^{67}$Cu, $^{67}$Ga, $^{68}$Ga, $^{89}$Sr, $^{90}$Y, $^{94m}$Tc, $^{99m}$Tc, $^{111}$In, $^{149}$Pm, $^{153}$Gd, $^{153}$Sm, $^{161}$Tb, $^{166}$Ho, $^{177}$Lu, $^{186}$Re, $^{188}$Re, $^{203}$Pb, $^{211}$At, $^{212}$Bi, $^{212}$Pb or $^{225}$Ac.

8. The conjugate compound of any one of the preceding claims, having the general formula (I) or (II)

$$T\text{-}(S)_m\text{-}(C)_n\text{-}X\text{-}(P)_o \qquad (I)$$

$$T-(S)_m-\underset{\displaystyle (C)_n}{\underset{|}{A}}-X-(P)_o$$

(II)

wherein

**T** is said **disease-targeting moiety,**
**S** is a **functional spacer,**
**m** is 0 or 1,
**C** is said **chelating agent,**
**n** is 0 or 1,
**A** is an **anchor unit,** such as lysine or cysteine,
**X** is said **cleavable linker,**
**P** is said **PARP inhibitor moiety**
**o** is at least 1, preferably 1, 2 or 3,
or a salt, solvate or tautomer thereof.

**9.** The conjugate compound of claim 8, wherein the **functional spacer (S)** comprises at least two amino acids or amino acid derivatives, preferably

non-natural amino acids,
such as aminohexanoic acid (Ahx), naphthylalanine (Nal), trans-4-(aminomethyl)-cyclohexanecarboxylic acid (TXA),
D-amino acids,
such as D-phenylalanine, D-tyrosine, D-aspartic acid, D-lysine,
β-amino acids,
such as β-alanine (βA),

and combinations thereof,
and/or wherein the **functional spacer (S)** comprises polyethylenglycol (PEG), preferably $(PEG)_n$ wherein n is 1 to 24.

**10.** The conjugate compound of claim 8 or 9, wherein the functional spacer (S) comprises or consists of an amino acid sequence selected from

| | | |
|---|---|---|
| i. | TXA-L-Nal, | |
| ii. | D-Asp-D-Tyr-D-Phe-D-Phe-Ahx | [SEQ ID NO. 1], |
| iii. | D-Lys-D-Asp-β-Ala-TXA-L-Nal | [SEQ ID NO. 2], |
| iv. | D-Lys-D-Asp-D-Tyr-D-Phe-D-Phe-Ahx | [SEQ ID NO. 3], |
| v. | D-Lys-D-Asp-D-Tyr-D-Phe-L-Nal-Ahx | [SEQ ID NO. 4], |
| vi. | D-Lys-D-Asp-D-Tyr-D-Phe-D-Phe-Ahx-Ahx | [SEQ ID NO. 5], |
| vii. | D-Asp-D-Asp-β-Ala-TXA-L-Nal | [SEQ ID NO. 6], |
| viii. | D-Asp-D-Asp-D-Tyr-D-Phe-D-Phe-Ahx-Ahx | [SEQ ID NO. 7], |
| ix. | D-Lys-D-Asp-D-Asp-β-Ala-TXA-L-Nal-D-Lys | [SEQ ID NO. 8], |
| x. | D-Lys-D-Asp-D-Asp-D-Tyr-D-Phe-D-Phe-Ahx-D-Lys | [SEQ ID NO. 9], |
| xi. | D-Asp-D-Asp-β-Ala-TXA-L-Nal-D-Lys | [SEQ ID NO. 10], |
| xii. | D-Lys(D-Lys)-D-Asp-D-Asp-D-Tyr-D-Phe-D-Phe-Ahx | [SEQ ID NO. 11], |
| xiii. | D-Lys(D-Lys)-D-Asp-D-Asp-D-Tyr-D-Phe-L-Nal-Ahx | [SEQ ID NO. 12], |
| xiv. | D-Lys(D-Lys)-D-Asp-D-Asp-D-Tyr-D-Phe-D-Phe-Ahx-Ahx, and | [SEQ ID NO. 13], |
| xv. | D-Lys(D-Lys)-D-Asp-D-Asp-β-Ala-TXA-L-Nal-D-Lys | [SEQ ID NO. 14]. |

**11.** The conjugate compound of any one of the preceding claims, having general formula I, wherein

**T** targets PSMA and preferably comprises Glu-urea-Glu (EuE), Glu-urea-Lys (EuK) and/or other urea-based peptides or peptidomimetics, such as Lys-urea-aminoadipic acid (Lys-urea-Aad),
**P** comprises or consists of rucaparib and **o** is 1 or 2,
**m** is 1 and **S** is as defined in claim 13,
**n** is 1 and **C** is as defined in claim 9,
**X** comprises a substrate for glutathione,

preferably having one of the following structures

Ruc-S S-MMC-D-Asp-D-Asp-(3-Ala-TXA-L-Nal-KuE,
Ruc-SS-MMC-D-Asp-D-Asp-D-Tyr-D-Phe-D-Phe-Ahx-Ahx-KuE, or
Ruc-S S-MMC-D-Asp-D-Asp-(3-Al a-TXA-L-Nal-D-Ly s-EuE,
or a salt, solvate or tautomer thereof.

**12.** The conjugate compound of any one of claims 1 to 11, having general formula II, wherein

**T** targets PSMA and preferably comprises Glu-urea-Glu (EuE), Glu-urea-Lys (EuK) and/or other urea-based peptides or peptidomimetics, such as Lys-urea-aminoadipic acid (Lys-urea-Aad),
**P** comprises or consists of rucaparib and **o** is 1 or 2,
**m** is 1 and **S** is as defined in claim 13,
**n** is 1 and **C** is as defined in claim 9,
**X** comprises a substrate for glutathione,

preferably having one of the following structures

DOTAGA-D-Ly s(Ruc-S S)-D-Asp-(3-Ala-TXA-L-Nal-KuE,
DOTAGA-D-Lys(Ruc-SS)-D-Asp-D-Tyr-D-Phe-D-Phe-Ahx-KuE,
DOTAGA-D-Lys(Ruc-SS)-D-Asp-D-Tyr-D-Phe-L-Nal-Ahx-KuE,
DOTAGA-D-Lys(Ruc-SS)-D-Asp-D-Tyr-D-Phe-D-Phe-Ahx-Ahx-KuE,
DOTAGA-D-Lys(Ruc-SS)-D-Asp-D-Asp-D-Ala-TXA-L-Nal-D-Lys-EuE,
DOTAGA-D-Lys(Ruc-SS)-D-Asp-D-Asp-D-Tyr-D-Phe-D-Phe-Ahx-D-Lys-EuE,
DOTAGA-D-Lys(D-Lys(Ruc-SS)$_2$)-D-Asp-D-Asp-D-Tyr-D-Phe-D-Phe-Ahx-KuE,
DOTAGA-D-Lys(D-Lys(Ruc-SS)$_2$)-D-Asp-D-Asp-D-Tyr-D-Phe-L-Nal-Ahx-KuE,
DOTAGA-D-Lys(D-Lys(Ruc-SS)$_2$)-D-Asp-D-Asp-D-Tyr-D-Phe-D-Phe-Ahx-Ahx-KuE,
DOTAGA-D-Lys(D-Lys(Ruc-SS)$_2$)-D-Asp-D-Asp-β-Ala-TXA-L-Nal-D-Lys-EuE, or
DOTAGA-D-Lys(D-Lys(Ruc-SS)$_2$)-D-Asp-D-Asp-D-Tyr-D-Phe-D-Phe-Ahx-D-Lys-EuE,
or a salt, solvate or tautomer thereof.

**13.** A pharmaceutical composition comprising

at least one conjugate compound of any one of claims 1 to 12 and,
optionally, pharmaceutically acceptable carrier and/or excipient(s).

**14.** The compound of any one of claims 1 to 12 as vehicle for the specific delivery of at least one PARP inhibitor moiety to cells expressing the target the disease-targeting moiety (T) recognizes or binds to,

wherein said cells are preferably tumor cells,
preferably, as vehicle for the specific delivery of at least one PARP inhibitor moiety to PSMA-positive cells and tumors,
or to NAALADaseL- or mGluR8-positive cells and tumors.

**15.** The compound of any one of claims 1 to 12 or the pharmaceutical composition of claim 13 for use in medicine.

**16.** The compound of any one of claims 1 to 12 or the pharmaceutical composition of claim 13 for use in the treatment of cancer,
wherein, preferably, the cancer is

- a PSMA-positive tumor,
such as

prostate cancer, wherein the prostate cancer can be metastatic prostate cancer and/or biochemically recurrent prostate cancer,
glioma,
lung cancer,

- tumors expressing the target, wherein the target is selected from somatostatin-receptor (SSTR subtypes 2, 3, and 5), gastrin-releasing peptide receptor (GRPR), C-X-C motif chemokine receptor 4 (CXCR4), $\alpha v\beta 6$ integrin, $\alpha v\beta 8$ integrin, $\alpha 5\beta 1$ integrin, $\alpha v\beta 1$ integrin, folate receptor (FR), neurotensin receptor 1 (NTSR1), and melanocortin receptor 1 (MC1R),

wherein the route of administration is preferably intravenous, intraperitoneal, oral, nasal, as solution or spray, more preferably intravenous or intraperitoneal.

**17.** The compound or pharmaceutical composition for use according to claim 15 or 16, in combination with another therapy,
preferably chemotherapy, hormonal therapy, immune-checkpoint blockade, DNA damage response (DDR) inhibiting agents, antiangiogenic agents, tubulin inhibitors, and/or standard of care treatment,

and/or in combination with external beam radiation therapy,
or in combination with endoradiotherapy, such as targeted radioligand therapy (RLT) or peptide receptor radiotherapy (PRRT),
preferably comprising using therapeutic isotopes in endoradiotherapy agent(s), said therapeutic isotopes being selected from $^{47}Sc$, $^{67}Cu$, $^{67}Ga$, $^{89}Sr$, $^{90}Y$, $^{149}Pm$, $^{149}Tb$, $^{153}Sm$, $^{161}Tb$, $^{166}Ho$, $^{177}Lu$, $^{186}Re$, $^{188}Re$, $^{211}At$, $^{212}Bi$, $^{212}Pb$ or $^{225}Ac$, e.g. in RLT or PRRT agents.

**18.** The compound of any one of claims 1 to 12 or the pharmaceutical composition of claim 13 for use in patient selection, preferably comprising molecular imaging, such as positron emission tomography (PET) or single-photon emission computed tomography (SPECT) with said compound or said pharmaceutical composition.

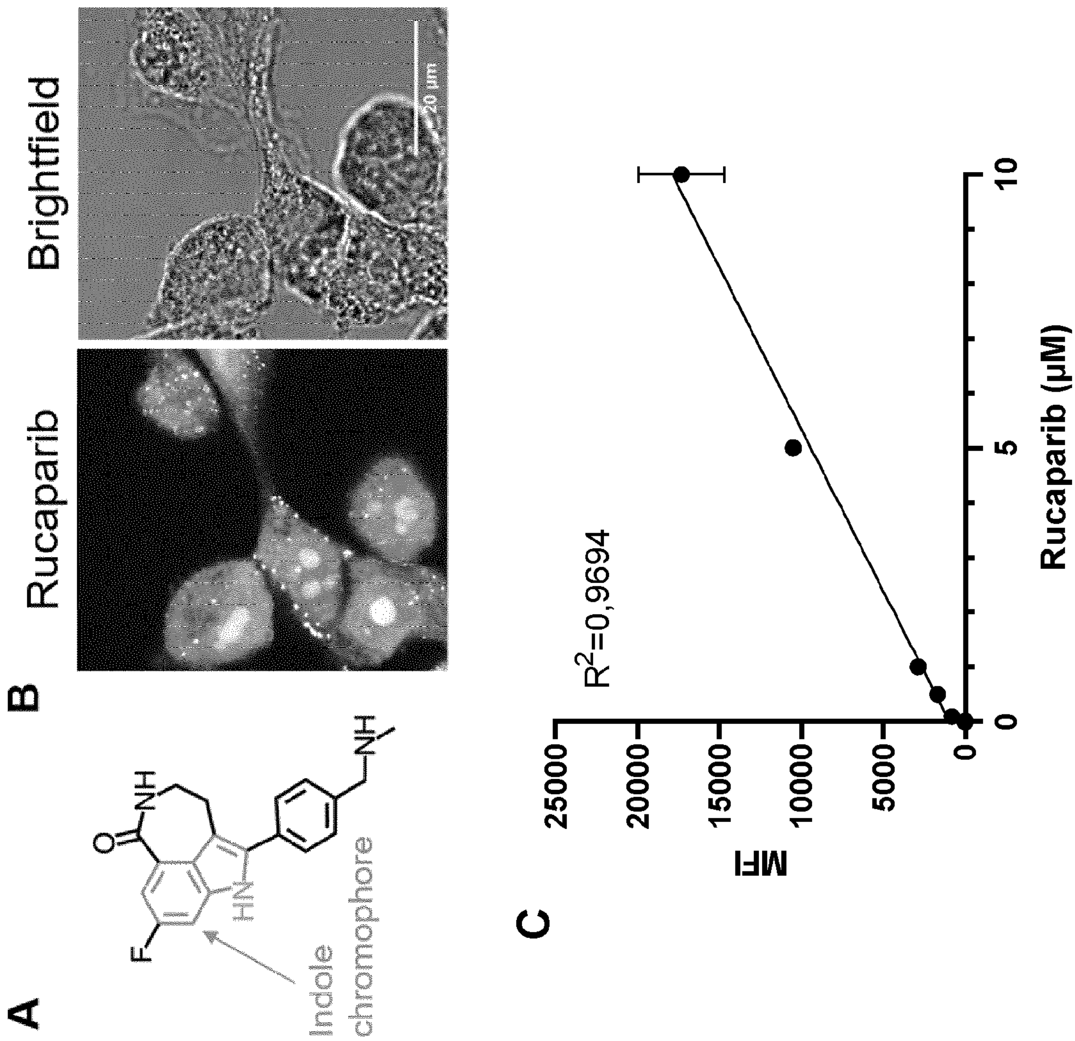
A
Indole chromophore
B
Rucaparib
Brightfield
20 µm
C
$R^2$=0,9694
MFI
25000
20000
15000
10000
5000
0
0
5
10
Rucaparib (µM)

Figure 1

Figure 2

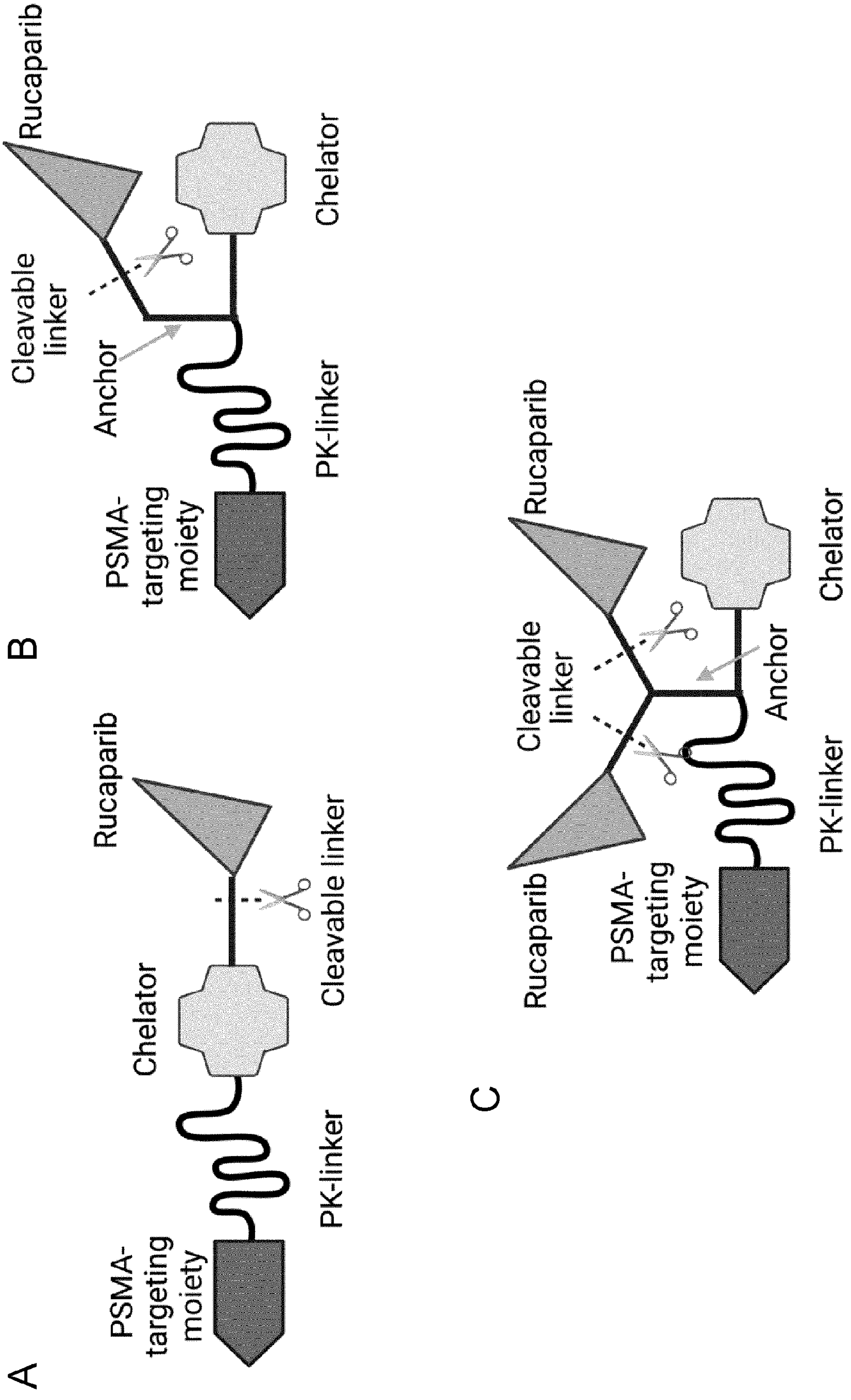
A
PSMA-targeting moiety
PK-linker
Chelator
Cleavable linker
Rucaparib
B
PSMA-targeting moiety
PK-linker
Anchor
Cleavable linker
Rucaparib
Chelator
C
Rucaparib
PSMA-targeting moiety
PK-linker
Cleavable linker
Anchor
Rucaparib
Chelator

Figure 3

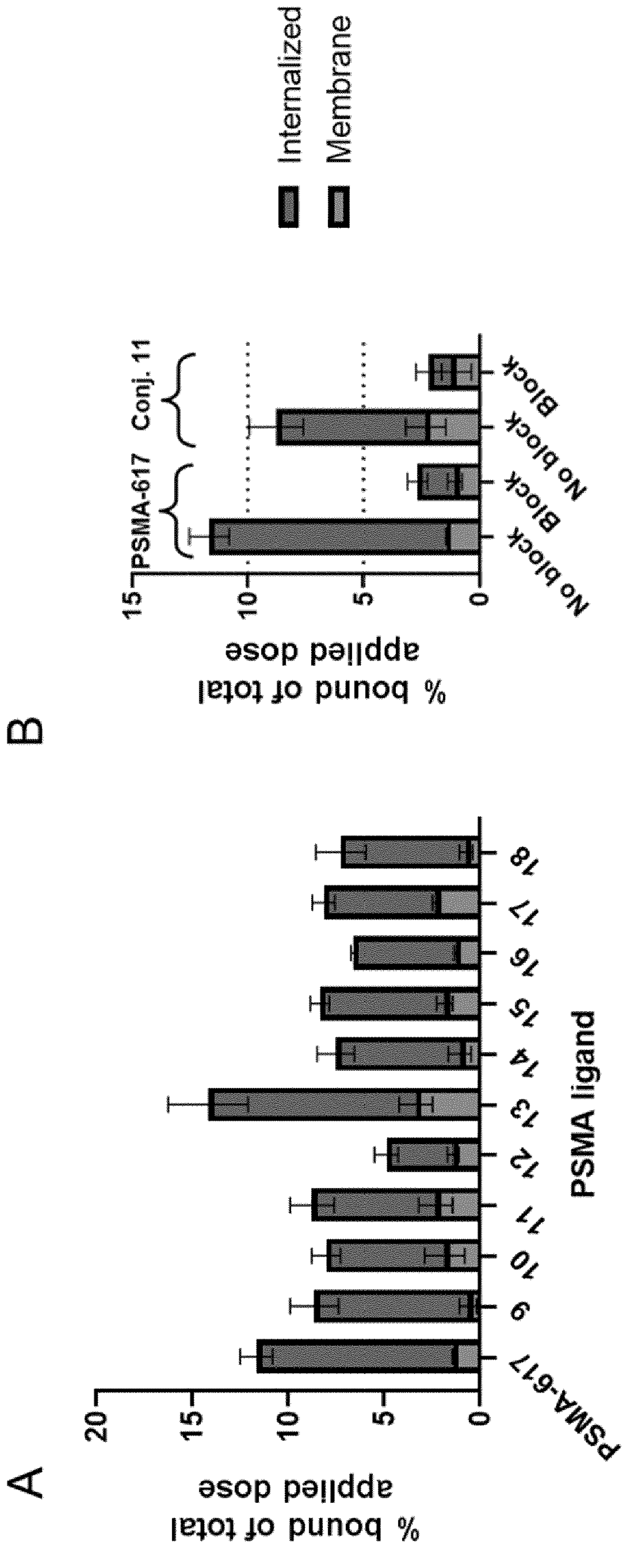
A
% bound of total applied dose
0
5
10
15
20
PSMA-617
9
10
11
12
13
14
15
16
17
18
PSMA ligand
B
% bound of total applied dose
0
5
10
15
PSMA-617
Conj. 11
No block
Block
No block
Block
Internalized
Membrane

Figure 4

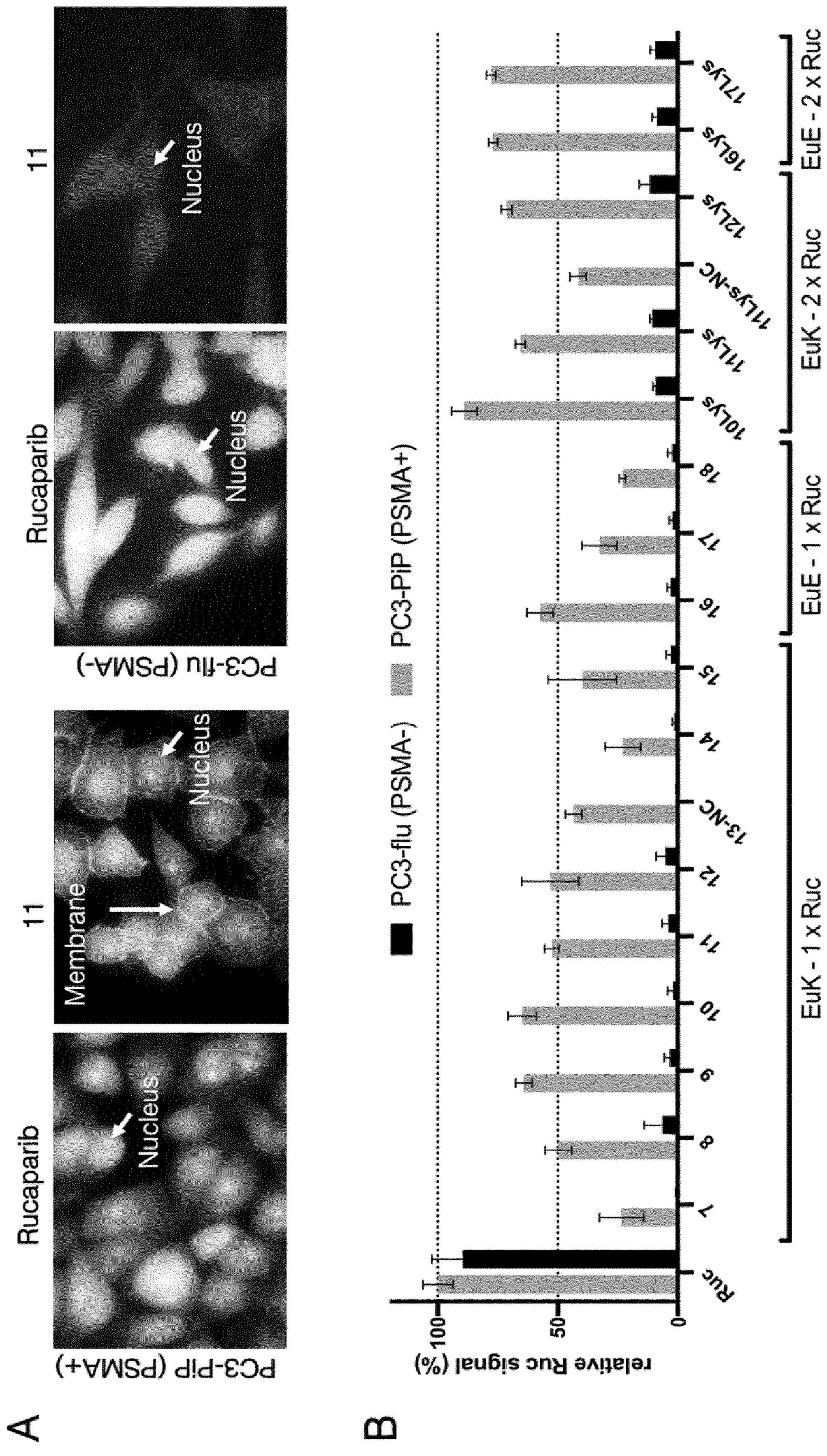
A
B
PC3-PiP (PSMA+)
PC3-flu (PSMA-)
Rucaparib
11
Nucleus
Membrane
relative Ruc signal (%)
100
50
0
PC3-flu (PSMA-)
PC3-PiP (PSMA+)
Ruc
7
8
9
10
11
12
13-NC
14
15
16
17
18
10Lys
11Lys
11Lys-NC
12Lys
16Lys
17Lys
EuK - 1 x Ruc
EuE - 1 x Ruc
EuK - 2 x Ruc
EuE - 2 x Ruc

Figure 5

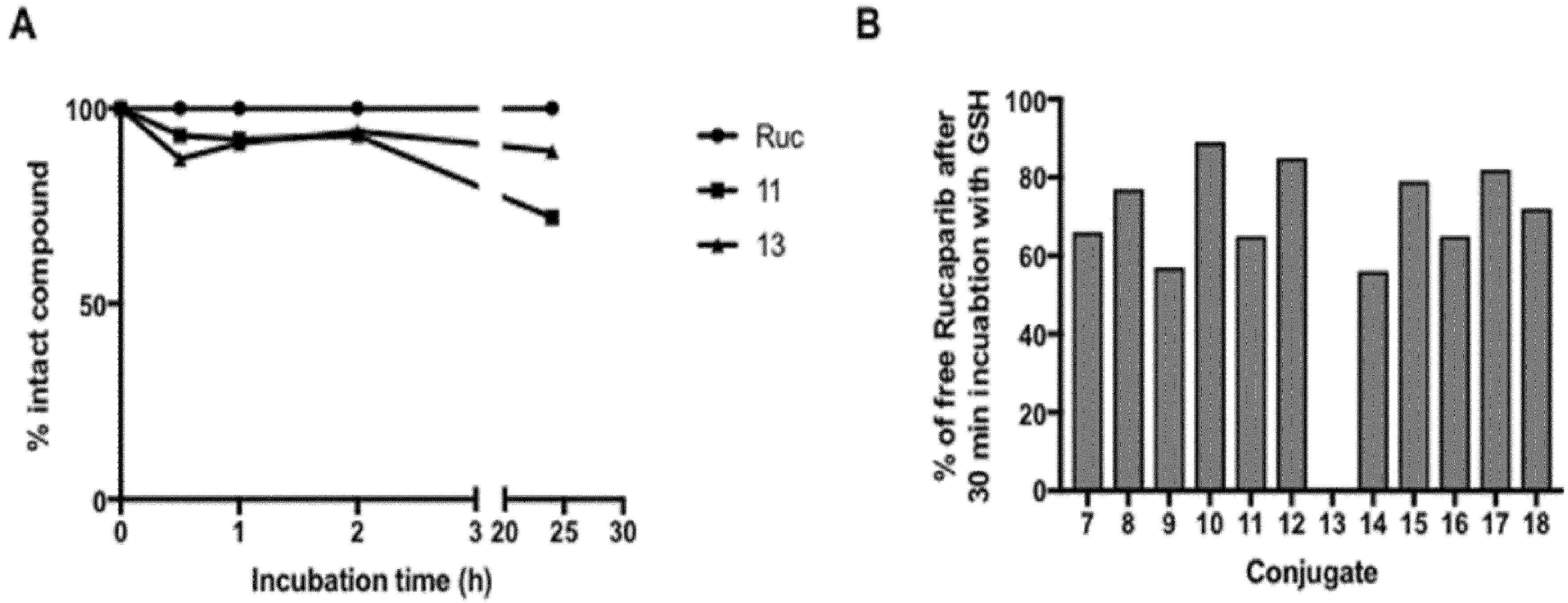
A
% intact compound
100
50
0
0
1
2
3
20
25
30
Incubation time (h)
Ruc
11
13
B
% of free Rucaparib after
30 min incuabtion with GSH
100
80
60
40
20
0
7
8
9
10
11
12
13
14
15
16
17
18
Conjugate

Figure 6

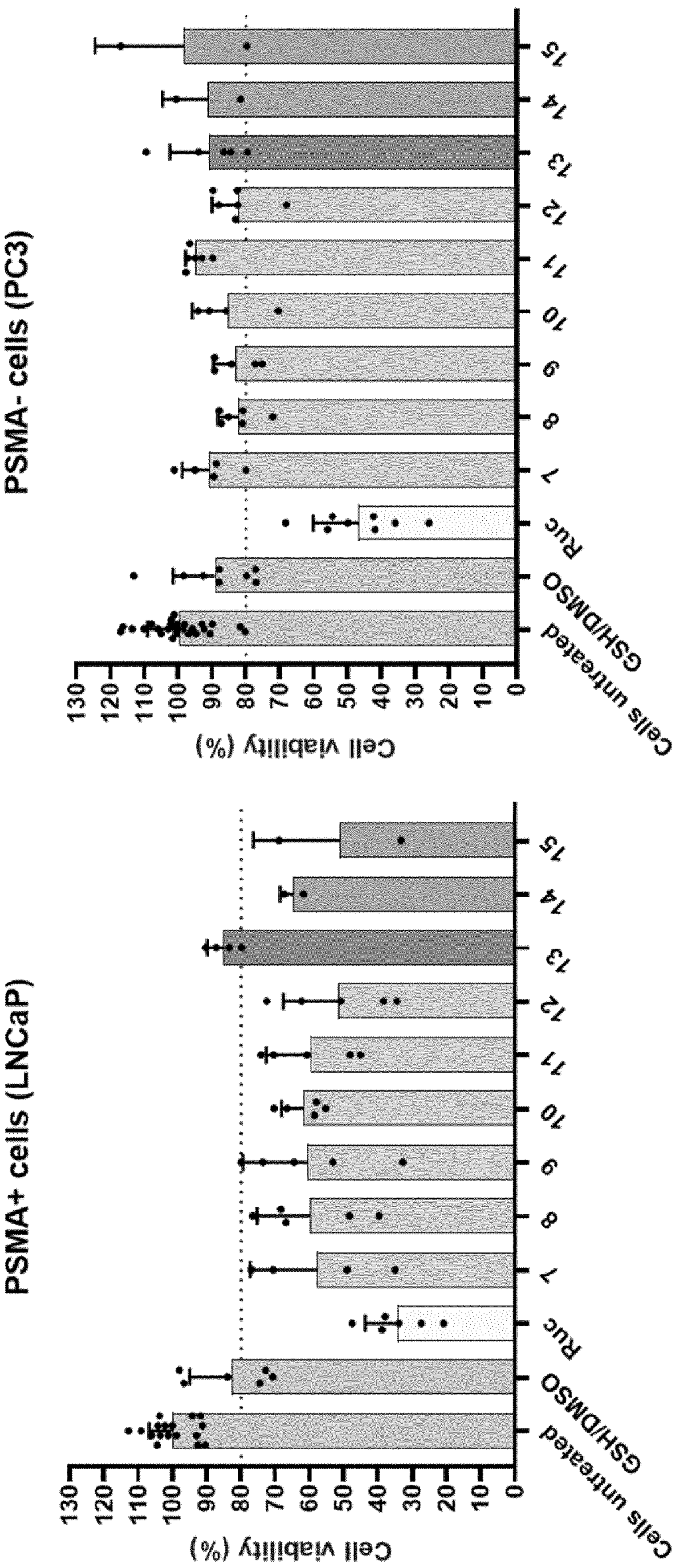
PSMA+ cells (LNCaP)
PSMA- cells (PC3)
Cell viability (%)
130
120
110
100
90
80
70
60
50
40
30
20
10
0
Cells untreated
GSH/DMSO
Ruc
7
8
9
10
11
12
13
14
15

Figure 7

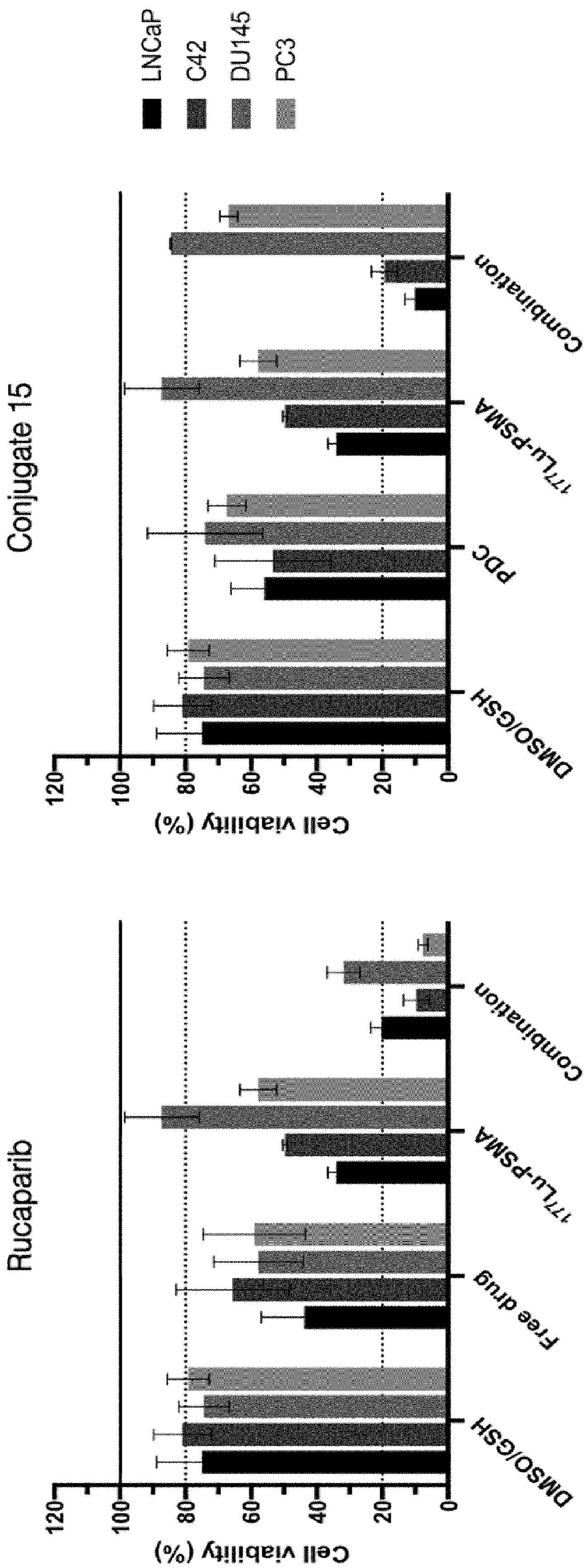
Rucaparib
Conjugate 15
Cell viability (%)
0
20
40
60
80
100
120
DMSO/GSH
Free drug
PDC
$^{177}$Lu-PSMA
Combination
LNCaP
C42
DU145
PC3

Figure 8

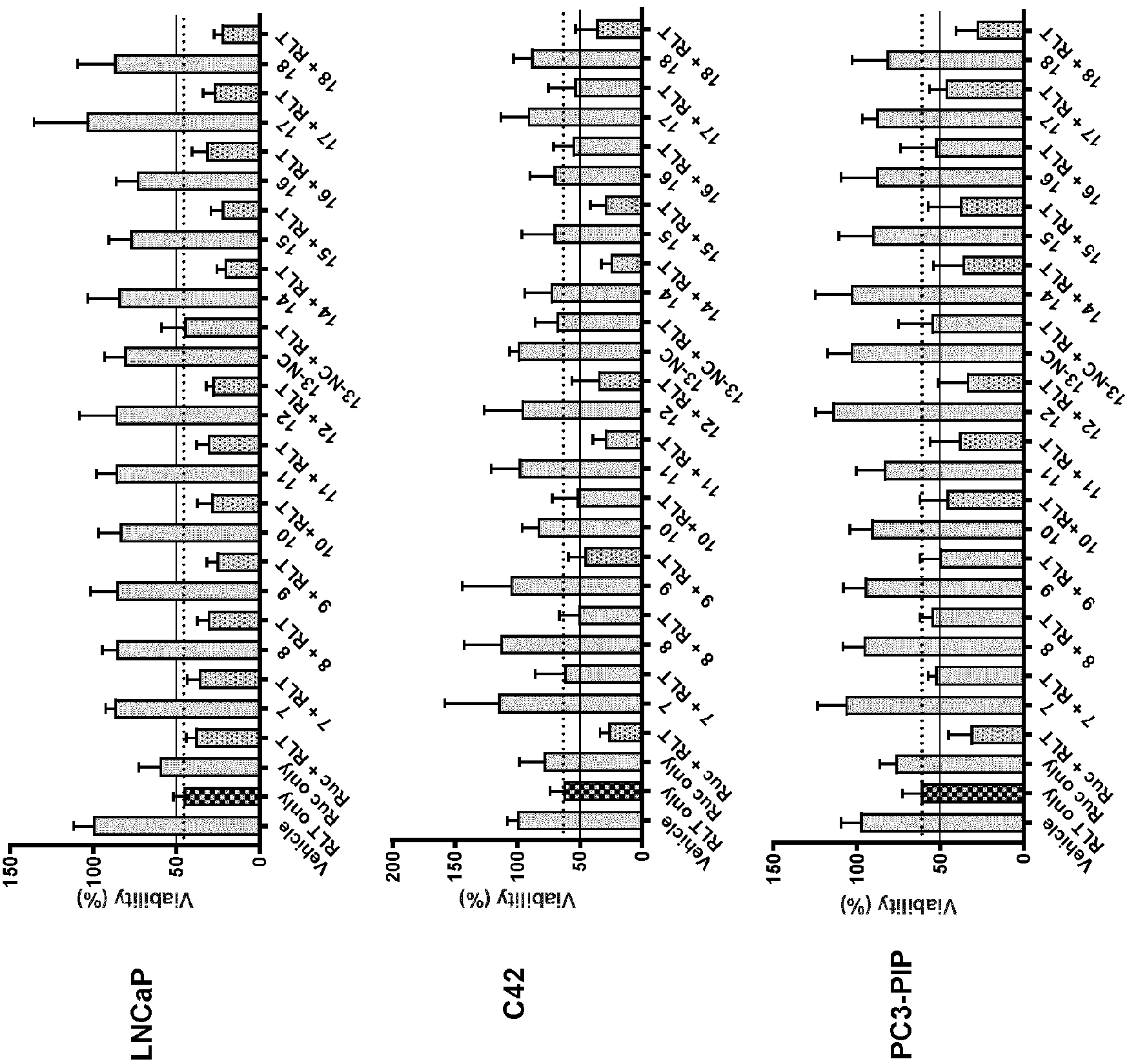
LNCaP
C42
PC3-PIP
Viability (%)
Vehicle
RLT only
Ruc only
Ruc + RLT
7
7 + RLT
8
8 + RLT
9
9 + RLT
10
10 +RLT
11
11 + RLT
12
12 + RLT
13-NC
13-NC + RLT
14
14 + RLT
15
15 + RLT
16
16 + RLT
17
17 + RLT
18
18 + RLT

# Figure 8 continued

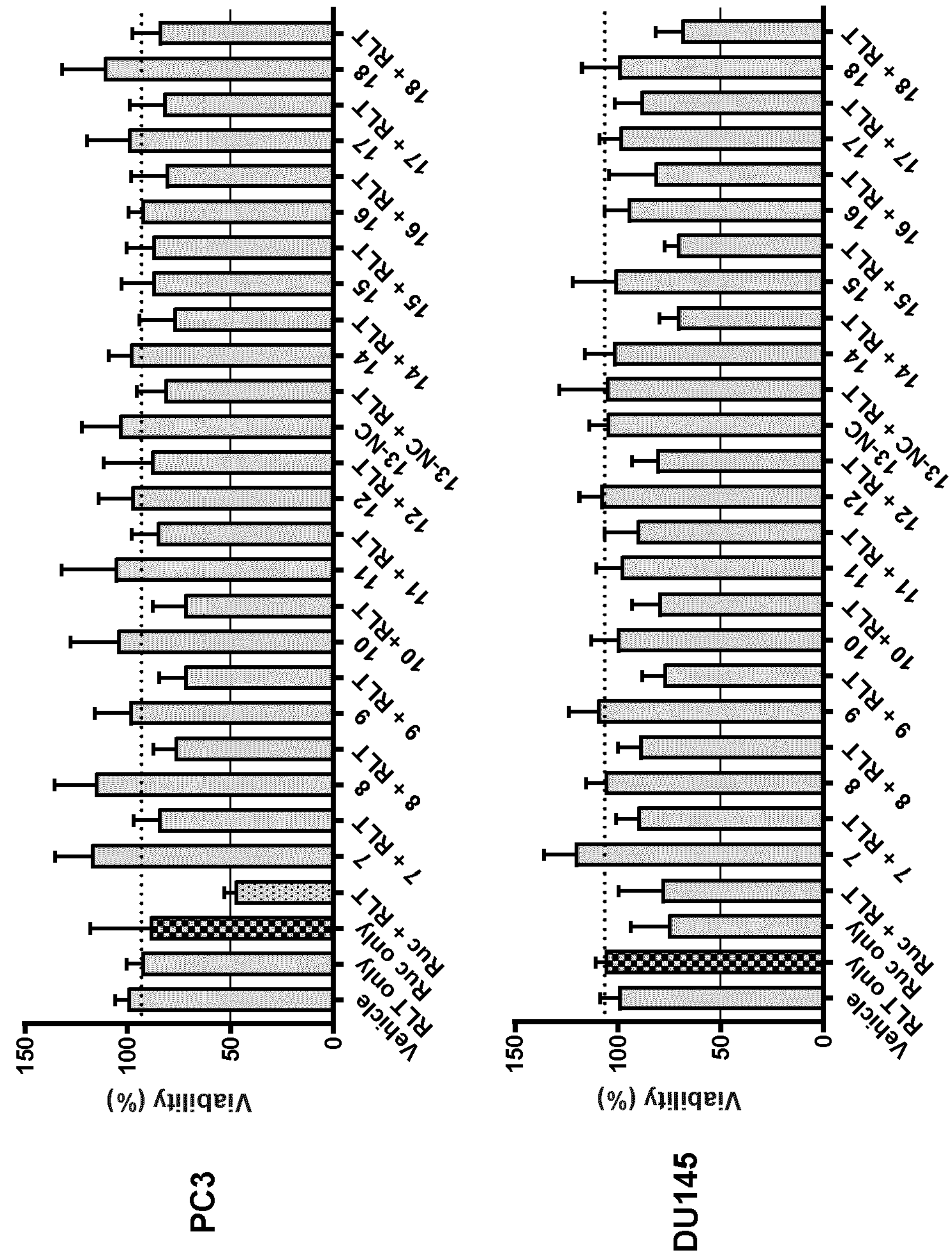

# Figure 9

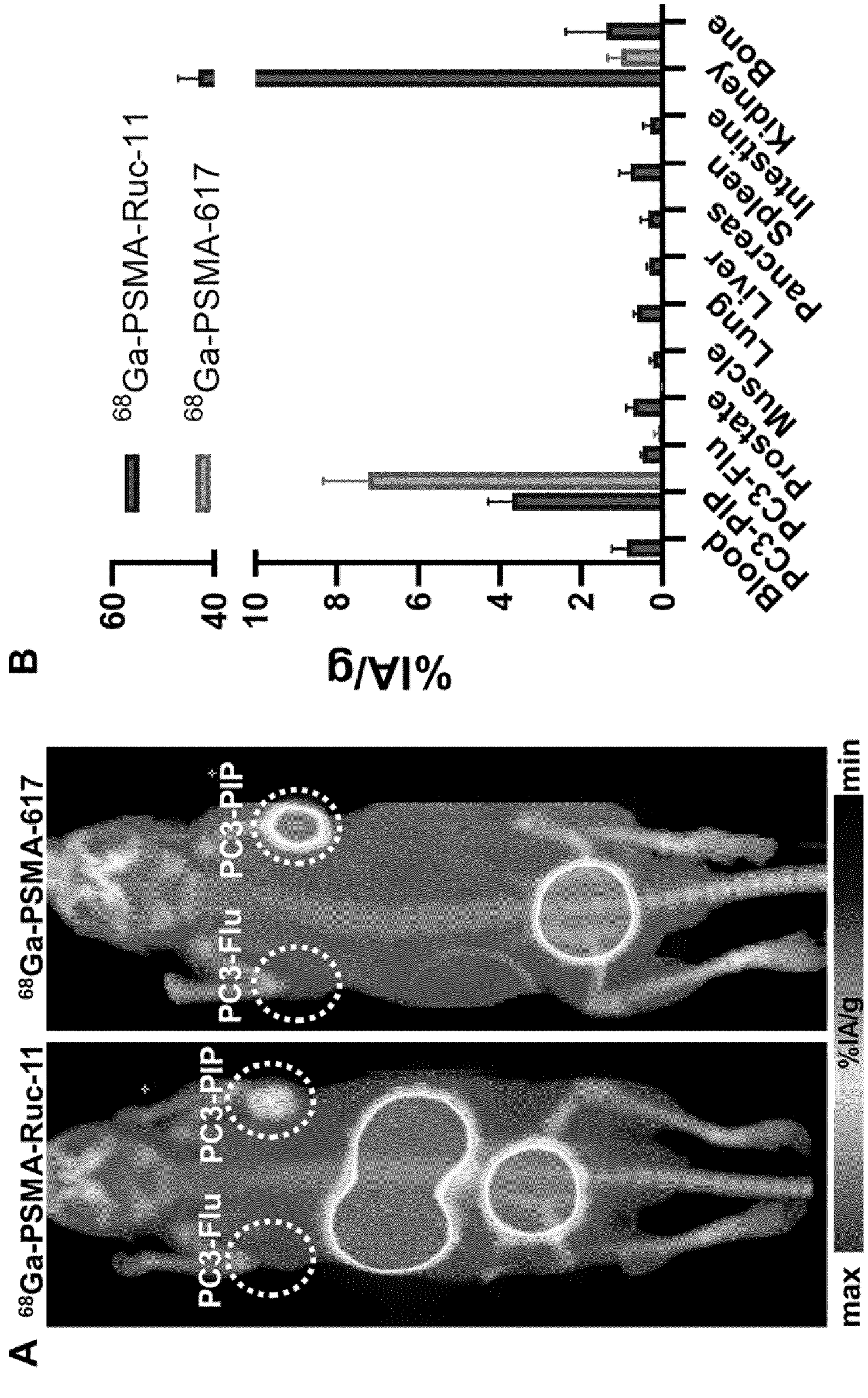
A
$^{68}$Ga-PSMA-Ruc-11
$^{68}$Ga-PSMA-617
PC3-Flu
PC3-PIP
max
%IA/g
min
B
$^{68}$Ga-PSMA-Ruc-11
$^{68}$Ga-PSMA-617
%IA/g
60
40
10
8
6
4
2
0
Blood
PC3-PIP
PC3-Flu
Prostate
Muscle
Lung
Liver
Pancreas
Spleen
Intestine
Kidney
Bone

# Figure 10

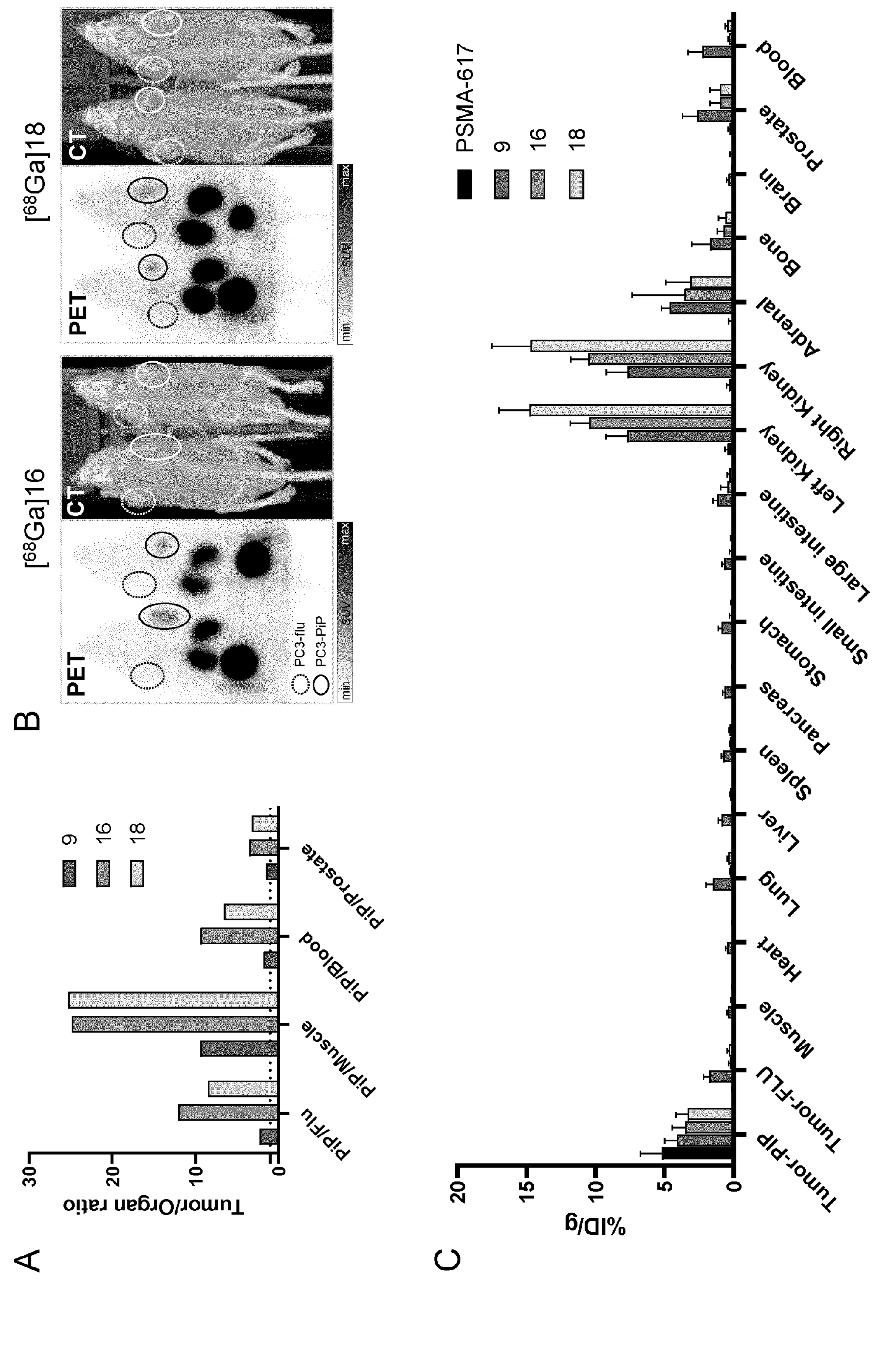
A
Tumor/Organ ratio
30
20
10
0
9
16
18
PiP/Flu
PiP/Muscle
PiP/Blood
PiP/Prostate
B
[68Ga]16
[68Ga]18
PET
CT
PC3-flu
PC3-PiP
min
SUV
max
C
%ID/g
20
15
10
5
0
PSMA-617
9
16
18
Tumor-PIP
Tumor-FLU
Muscle
Heart
Lung
Liver
Spleen
Pancreas
Stomach
Small intestine
Large intestine
Left Kidney
Right Kidney
Adrenal
Bone
Brain
Prostate
Blood

Europäisches Patentamt
European Patent Office
Office européen des brevets

# EUROPEAN SEARCH REPORT

Application Number
EP 22 21 6113

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2021/123013 A1 (UNIV MAINZ JOHANNES GUTENBERG [DE]) 24 June 2021 (2021-06-24)<br>* pages 46, 47, 50, 54, 55 *<br>* page 8, paragraph 1 *<br>* page 17, paragraph 3 - page 18, paragraph 1 *<br>----- | 1-18 | INV.<br>A61K47/54<br>A61K47/65<br>A61K51/04<br>A61P35/00 |
| E | WO 2023/278592 A1 (UNIV DUKE [US]) 5 January 2023 (2023-01-05)<br>* page 45, scheme 4; example 4; compound 19 *<br>* page 46, scheme 5; example 5; compound 23b *<br>* claims 1-4, 7,8 23, 26-35, 37 *<br>----- | 1-6,8,<br>13-18 | |

TECHNICAL FIELDS SEARCHED (IPC)

A61K
A61P

The present search report has been drawn up for all claims

2

| Place of search | Date of completion of the search | Examiner |
|---|---|---|
| The Hague | 1 June 2023 | Bliem, Barbara |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

# ANNEX TO THE EUROPEAN SEARCH REPORT ON EUROPEAN PATENT APPLICATION NO.

EP 22 21 6113

This annex lists the patent family members relating to the patent documents cited in the above-mentioned European search report. The members are as contained in the European Patent Office EDP file on
The European Patent Office is in no way liable for these particulars which are merely given for the purpose of information.

01-06-2023

| Patent document cited in search report | Publication date | Patent family member(s) | Publication date |
|---|---|---|---|
| WO 2021123013 A1 | 24-06-2021 | AU 2020406729 A1 | 30-06-2022 |
| | | BR 112022011465 A2 | 23-08-2022 |
| | | CN 114980931 A | 30-08-2022 |
| | | DE 102019135564 A1 | 24-06-2021 |
| | | EP 4076534 A1 | 26-10-2022 |
| | | JP 2023507524 A | 22-02-2023 |
| | | KR 20220137003 A | 11-10-2022 |
| | | US 2023112958 A1 | 13-04-2023 |
| | | WO 2021123013 A1 | 24-06-2021 |
| WO 2023278592 A1 | 05-01-2023 | NONE | |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- **ABIDA, W. ; PATNAIK, A. ; CAMPBELL, D. ; SHAPIRO, J. ; BRYCE, A.H. ; MCDERMOTT, R. ; SAUTOIS, B. ; VOGELZANG, N.J. ; BAMBURY, R.M. ; VOOG, E.** Rucaparib in Men With Metastatic Castration-Resistant Prostate Cancer Harboring a BRCA1 or BRCA2 Gene Alteration. *J Clin Oncol,* 2020, vol. 38 (32), 3763-72
- **ANTONARAKIS, E.S. ; GOMELLA, L.G. ; PETRYLAK, D.P.** When and How to Use PARP Inhibitors in Prostate Cancer: A Systematic Review of the Literature with an Update on OnGoing Trials. *Eur Urol Oncol,* 2020, vol. 3 (5), 594-611
- **BANERJEE, S.R. ; PULLAMBHATLA, M. ; FOSS, C.A. ; NIMMAGADDA, S. ; FERDANI, R. ; ANDERSON, C.J. ; MEASE, R.C. ; POMPER, M.G.** Cu-labeled inhibitors of prostate-specific membrane antigen for PET imaging of prostate cancer. *J Med Chem,* 2014, vol. 57 (6), 2657-69
- **BENESOVA, M. ; SCHAFER, M. ; BAUDER-WUST, U. ; AFSHAR-OROMIEH, A. ; KRATOCHWIL, C. ; MIER, W. ; HABERKORN, U. ; KOPKA, K. ; EDER, M.** Preclinical Evaluation of a Tailor-Made DOTA-Conjugated PSMA Inhibitor with Optimized Linker Moiety for Imaging and Endoradiotherapy of Prostate Cancer. *J Nucl Med,* 2015, vol. 56 (6), 914-20
- **BRAY, F. ; FERLAY, J. ; SOERJOMATARAM, I. ; SIEGEL, R.L. ; TORRE, L.A. ; JEMAL, A.** Global cancer statistics 2018: GLOBOCAN estimates of incidence and mortality worldwide for 36 cancers in 185 countries. *CA Cancer J Clin,* 2018, vol. 68 (6), 394-424
- **CARPINO, L. A. ; HAN, G. Y.** 9-Fluorenylmethoxycarbonyl amino-protecting group. *The Journal of Organic Chemistry,* 1972, vol. 37 (22), 3404-3409
- **CHEN, Y. ; PULLAMBHATLA, M. ; FOSS, C.A. ; BYUN, Y. ; NIMMAGADDA, S. ; SENTHAMIZHCHELVAN, S. ; SGOUROS, G. ; MEASE, R.C. ; POMPER, M.G** 2-(3-{1-Carboxy-5-[(6-[18F]fluoro-pyridine-3-carbonyl)-amino]-pentyl}-ureido)-pentanedioic acid, [18F]DCFPyL, a PSMA-based PET imaging agent for prostate cancer. *Clinical cancer research : an official journal of the American Association for Cancer Research,* 2011, vol. 17 (24), 7645-53
- **DEEKS, E.D.** Olaparib: first global approval. *Drugs,* 2015, vol. 75 (2), 231-40
- **EIBER, M. ; MAURER, T. ; SOUVATZOGLOU, M. ; BEER, A.J. ; RUFFANI, A. ; HALLER, B. ; GRANER, F.P. ; KUBLER, H. ; HABERKORN, U. ; EISENHUT, M.** Evaluation of Hybrid (6)(8)Ga-PSMA Ligand PET/CT in 248 Patients with Biochemical Recurrence After Radical Prostatectomy. *J Nucl Med,* 2015, vol. 56 (5), 668-74
- **FENDLER, W.P. ; SCHMIDT, D.F. ; WENTER, V. ; THIERFELDER, K.M. ; ZACH, C. ; STIEF, C. ; BARTENSTEIN, P. ; KIRCHNER, T. ; GILDEHAUS, F.J. ; GRATZKE, C.** 8Ga-PSMA PET/CT Detects the Location and Extent of Primary Prostate Cancer. *J Nucl Med,* 2016, vol. 57 (11), 1720-25
- **FENDLER, W.P. ; RAHBAR, K. ; HERRMANN, K. ; KRATOCHWIL, C. ; EIBER, M.** 177)Lu-PSMA Radioligand Therapy for Prostate Cancer. *J Nucl Med,* 2017, vol. 58 (8), 1196-200
- **FENDLER, W.P. ; CALAIS, J. ; EIBER, M. ; FLAVELL, R.R. ; MISHOE, A. ; FENG, F.Y. ; NGUYEN, H.G. ; REITER, R.E. ; RETTIG, M.B. ; OKAMOTO, S.** Assessment of 68Ga-PSMA-11 PET Accuracy in Localizing Recurrent Prostate Cancer: A Prospective Single-Arm Clinical Trial. *JAMA Oncol,* 2019
- **GHOSH, A. ; HESTON, W.D.** Tumor target prostate specific membrane antigen (PSMA) and its regulation in prostate cancer. *J Cell Biochem,* 2004, vol. 91 (3), 528-39
- **GILLESSEN, S. ; OMLIN, A. ; ATTARD, G. ; DE BONO, J.S. ; EFSTATHIOU, E. ; FIZAZI, K. ; HALABI, S. ; NELSON, P.S. ; SARTOR, O. ; SMITH, M.R.** Management of patients with advanced prostate cancer: recommendations of the St Gallen Advanced Prostate Cancer Consensus Conference (APCCC) 2015. *Ann Oncol,* 2015, vol. 26 (8), 1589-604
- **HARMATYS, K.M. ; OVERCHUK, M. ; CHEN, J. ; DING, L. ; CHEN, Y. ; POMPER, M.G. ; ZHENG, G.** Tuning Pharmacokinetics to Improve Tumor Accumulation of a Prostate-Specific Membrane Antigen-Targeted Phototheranostic Agent. *Bioconjug Chem,* 2018, vol. 29 (11), 3746-56
- **HEINZEL, A. ; BOGHOS, D. ; MOTTAGHY, F.M. ; GAERTNER, F. ; ESSLER, M. ; VON MALLEK, D. ; AHMADZADEHFAR, H.** 68)Ga-PSMA PET/CT for monitoring response to (177)Lu-PSMA-617 radioligand therapy in patients with metastatic castration-resistant prostate cancer. *Eur J Nucl Med Mol Imaging,* 2019, vol. 46 (5), 1054-62

- **HENSBERGEN, A.W. ; BUCKLE, T. ; VAN WILLIGEN, D.M. ; SCHOTTELIUS, M. ; WELLING, M.M. ; VAN DER WIJK, F.A. ; MAURER, T. ; VAN DER POEL, H.G. ; VAN DER PLUIJM, G. ; VAN WEERDEN, W.M.** Hybrid Tracers Based on Cyanine Backbones Targeting Prostate-Specific Membrane Antigen: Tuning Pharmacokinetic Properties and Exploring Dye-Protein Interaction. *Journal of Nuclear Medicine,* 2020, vol. 61 (2), 234-41
- **HOFFMANN, M.A. ; WIELER, H.J. ; BAUES, C. ; KUNTZ, N.J. ; RICHARDSEN, I. ; SCHRECKENBERGER, M.** The Impact of 68Ga-PSMA PET/CT and PET/MRI on the Management of Prostate Cancer. *Urology,* 2019
- **HOY, S.M.** Talazoparib: First Global Approval. *Drugs,* 2018, vol. 78 (18), 1939-46
- **KELLY, J.M. ; AMOR-COARASA, A. ; NIKOLOPOULOU, A. ; KIM, D. ; WILLIAMS, C. ; VALLABHAJOSULA, S. ; BABICH, J.W.** Assessment of PSMA targeting ligands bearing novel chelates with application to theranostics: Stability and complexation kinetics of 68Ga3+, 111In3+, 177Lu3+ and 225Ac3+. *Nuclear Medicine and Biology,* 2017, vol. 55, 38-46
- **KOSSATZ, S. ; CARNEY, B. ; FARLEY, C. ; WEBER, W.A. ; DRAIN, C.M. ; REINER, T.** Direct Imaging of Drug Distribution and Target Engagement of the PARP Inhibitor Rucaparib. *J Nucl Med,* 2018, vol. 59 (8), 1316-20
- **MERRIFIELD, R. B.** Solid Phase Peptide Synthesis. I. The Synthesis of a Tetrapeptide. *Journal of the American Chemical Society,* 1963, vol. 85 (14), 2149-2154
- **NIZIALEK, E. ; ANTONARAKIS, E.S.** PARP Inhibitors in Metastatic Prostate Cancer: Evidence to Date. *Cancer Manag Res,* 2020, vol. 12, 8105-14
- **NOTNI, J. ; ŠIMEČEK, J. ; HERMANN, P. ; WESTER, H.-J.** TRAP, a Powerful and Versatile Framework for Gallium-68 Radiopharmaceuticals. *Chemistry - A European Journal,* 2011, vol. 17 (52), 14718-14722
- **PEZARO, C.** PARP inhibitor combinations in prostate cancer. *Ther Adv Med Oncol,* 2020, vol. 12
- **PETROV, S.A. ; ZYK, N.Y. ; MACHULKIN, A.E. ; BELOGLAZKINA, E.K. ; MAJOUGA, A.G.** PSMA-targeted low-molecular double conjugates for diagnostics and therapy. *Eur J Med Chem,* 2021, vol. 225, 113752
- **RAJAN, A. ; CARTER, C.A. ; KELLY, R.J. ; GUTIERREZ, M. ; KUMMAR, S. ; SZABO, E. ; YANCEY, M.A. ; JI, J. ; MANNARGUDI, B. ; WOO, S.** A phase I combination study of olaparib with cisplatin and gemcitabine in adults with solid tumors. *Clin Cancer Res,* 2012, vol. 18 (8), 2344-51
- **RAY BANERJEE, S. ; CHEN, Z. ; PULLAMBHATLA, M. ; LISOK, A. ; CHEN, J. ; MEASE, R.C. ; POMPER, M.G.** Preclinical Comparative Study of 68Ga-Labeled DOTA, NOTA, and HBED-CC Chelated Radiotracers for Targeting PSMA. *Bioconjug Chem,* 2016, vol. 27 (6), 1447-55
- **ROBU, S. ; SCHMIDT, A. ; EIBER, M. ; SCHOTTELIUS, M. ; GIINTHER, T. ; HOOSHYAR YOUSEFI, B. ; SCHWAIGER, M. ; WESTER, H.-J.** Synthesis and preclinical evaluation of novel 18F-labeled Glu-urea-Glu-based PSMA inhibitors for prostate cancer imaging: a comparison with 18F-DCFPyl and 18F-PSMA-1007. *EJNMMI research,* 2018, vol. 8 (1), 30
- **SARTOR, O. ; DE BONO, J. ; CHI, K.N. ; FIZAZI, K. ; HERRMANN, K. ; RAHBAR, K. ; TAGAWA, S.T. ; NORDQUIST, L.T. ; VAISHAMPAYAN, N. ; EL-HADDAD, G.** Lutetium-177-PSMA-617 for Metastatic Castration-Resistant Prostate Cancer. *N Engl J Med,* 2021
- **SYED, Y.Y.** Rucaparib: First Global Approval. *Drugs,* 2017, vol. 77 (5), 585-92
- PARP Inhibition to Enhance Response to Chemotherapy. **TAKEBE, N. ; QUINN, M. ; GUPTA, G. ; CHEN, A.P.** Targeting Cell Survival Pathways to Enhance Response to Chemotherapy. Elsevier, 2019, 231-57
- **UMBRICHT, C.A. ; BENEŠOVÁ, M. ; SCHMID, R.M. ; TÜRLER, A. ; SCHIBLI, R. ; VAN DER MEULEN, N.P. ; MÜLLER, C.** 44Sc-PSMA-617 for radiotheragnostics in tandem with 177Lu-PSMA-617-preclinical investigations in comparison with 68Ga-PSMA-11 and 68Ga-PSMA-617. *EJNMMI research,* 2017, vol. 7 (1), 9
- **UMBRICHT, C.A. ; BENEŠOVÁ, M. ; SCHIBLI, R. ; MÜLLER, C.** Preclinical Development of Novel PSMA-Targeting Radioligands: Modulation of Albumin-Binding Properties To Improve Prostate Cancer Therapy. *Molecular Pharmaceutics,* 2018, vol. 15 (6), 2297-306
- **VIRTANEN, V. ; PAUNU, K. ; AHLSKOG, J.K. ; VARNAI, R. ; SIPEKY, C. ; SUNDVALL, M.** PARP Inhibitors in Prostate Cancer-The Preclinical Rationale and Current Clinical Development. *Genes (Basel),* 2019, vol. 10 (8
- **WEINEISEN, M. ; SIMECEK, J. ; SCHOTTELIUS, M. ; SCHWAIGER, M. ; WESTER, H. J.** Synthesis and preclinical evaluation of DOTAGA-conjugated PSMA ligands for functional imaging and endoradiotherapy of prostate cancer. *Ejnmmi Res,* 2014, vol. 4 (1), 63
- **WILSON, T. C. ; XAVIER, M. A. ; KNIGHT, J. ; VERHOOG, S. ; TORRES, J. B. ; MOSLEY, M. ; HOPKINS, S. L. ; WALLINGTON, S. ; ALLEN, P. D. ; KERSEMANS, V.** PET imaging of PARP expression using 18F-olaparib. *Journal of Nuclear Medicine,* 2019, vol. 60 (4), 504-510

- **YADAV, M.P. ; BALLAL, S. ; SAHOO, R.K. ; DWIVEDI, S.N. ; BAL, C.** Radioligand Therapy With (177)Lu-PSMA for Metastatic Castration-Resistant Prostate Cancer: A Systematic Review and Meta-Analysis. *AJR Am J Roentgenol,* 2019, 1-11
- **YAO, V. ; BACICH, D.J.** Prostate specific membrane antigen (PSMA) expression gives prostate cancer cells a growth advantage in a physiologically relevant folate environment in vitro. *The Prostate,* 2006, vol. 66 (8), 867-75
- **ZHERNOSEKOV, FILOSOFOV, D. V. ; BAUM, R. P. ; ASCHOFF, P. ; BIHL, H. ; RAZBASH, A. A. ; JAHN, M. ; JENNEWEIN, M. ; ROSCH, F.** Processing of Generator-Produced 68Ga for Medical Application. *The Journal of Nuclear Medicine (1978),* 2007, vol. 48 (10), 1741-1748, https://doi.org/10.2967/j numed.107.040378